# EUROPÄISCHE PATENTSCHRIFT

(11) **EP 0 645 386 B1**
(45) Veröffentlichungstag und Bekanntmachung des Hinweises auf die Patenterteilung: **29.12.1997**
(21) Anmeldenummer: 94114445.3
(22) Anmeldetag: 14.09.1994
(51) Int. Cl.: C07D 413/12, C07D 413/14, A01N 47/36, C07D 273/01

(54) **N-Azinyl-N'-(het)arylsulfonylharnstoffe als Herbizide**
N-Azinyl-N'-(het)arylsulfonylureas as herbicides
N-azinyl-N'-(het)arylsulfonylurées comme herbicides

(30) Priorität: 27.09.1993 DE 4332796; 29.10.1993 DE 4336875
(43) Veröffentlichungstag der Anmeldung: 29.03.1995
(73) Patentinhaber: BAYER AG, 51368 Leverkusen (DE)
(72) Erfinder: Philipp, Ulrich, Dr., D-51065 Köln (DE); Stetter, Jörg, Prof.Dr., D-42115 Wuppertal (DE); Santel, Hans-Joachim, Dr., D-51371 Leverkusen (DE); Dollinger, Markus, Dr., D-51381 Leverkusen (DE)

(56) Entgegenhaltungen:
- EP-A- 0 173 958
- EP-A- 0 301 784
- CHEMICAL ABSTRACTS, vol. 114, no. 9, 4. März 1991, Columbus, Ohio, US; abstract no. 77044x, H. OYAMA ET AL. 'Preparation of 5,6-dihydro-1,4,2-dioxazines as herbicides' Seite 267 ;

## Beschreibung

Die Erfindung betrifft neue N-Azinyl-N'-(het)arylsulfonyl-harnstoffe, Verfahren und neue Zwischenprodukte zu ihrer Herstellung und ihre Verwendung als Herbizide.

Es ist bereits bekannt, daß bestimmte N-Alinyl-N'-arylsulfonylharnstoffe mit einfachen offenkettigen Hydroxamsäureester-Gruppen im Arylteil, wie z.B. N-(4,6-Dimethylpyrimidin-2-yl)-N'-(2-methoxyaminocarbonyl-phenylsulfonyl)-harnstoff und der entsprechende - N'-(2-n-octyloxyaminocarbonylphenylsulfonyl)-harnstoff herbizide Eigenschaften aufweisen (vgl. DE-A-3 516 435 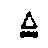 EP-A-173 958 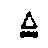 US-4 704 158). Die Herbizidwirkung dieser bekannten Verbindungen ist jedoch nicht in allen Belangen zufriedenstellend.

Weiterhin sind auch bestimmte herbizid wirksame N-Azinyl-N'-(het)arylsulfonylharnstoffe bekannt geworden, welche im (Het)arylteil durch O,O-dialkylierte, ebenfalls offenkettige Hydroxamsäure-Gruppen substituiert sind (vgl. EP-A-301 784); entsprechende cyclische Hydroxamsäurederivate sind dagegen bisher nicht beschrieben worden.

Es wurden nun neue N-Azinyl-N'-(het)arylsulfonyl-harnstoffe der allgemeinen Formel (I), 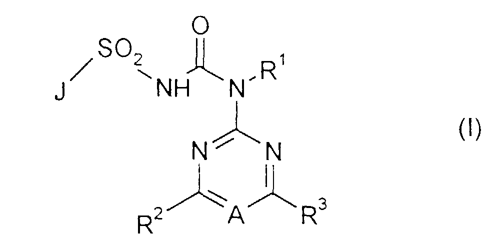 in welcher
- A: für Stickstoff oder eine CR¹¹-Gruppierung steht,
wobei
- R¹¹: für Wasserstoff, Alkyl, Halogen und Haloalkyl steht,
- R¹: für Wasserstoff oder einen gegebenenfalls substituierten Rest aus der Reihe Alkyl, Alkoxy, Alkoxyalkyl, Alkenyl, Alkinyl, Cycloalkyl, Cycloalkylalkyl, Aralkyl und Aryl steht,
- R²: für Wasserstoff, Halogen oder für jeweils gegebenenfalls durch Halogen substituiertes Alkyl, Alkoxy, Alkylthio, Alkylamino oder Dialkylamino mit jeweils 1 bis 6 Kohlenstoffatomen steht,
- R³: für Wasserstoff, Halogen oder für jeweils gegebenenfalls durch Halogen substituiertes Alkyl, Alkoxy, Alkylthio, Alkylamino oder Dialkylamino mit jeweils 1 bis 6 Kohlenstoffatomen steht,
- J: für J-1 bis J-4 steht, wobei J-1 bis J-4 folgende Bedeutungen haben: 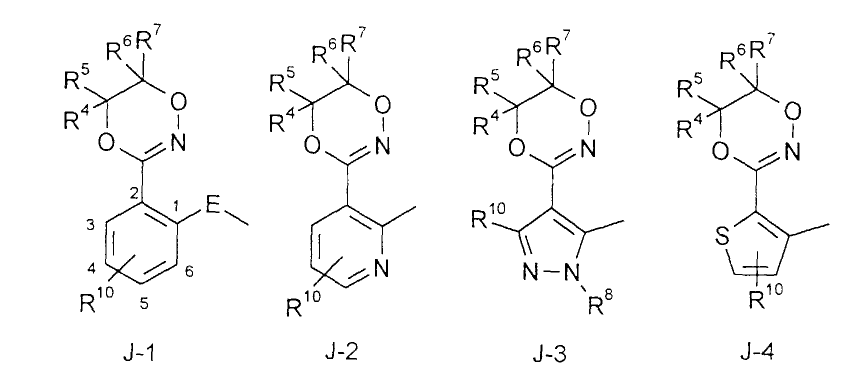 worin

- E: für eine direkte Bindung, Alkylen, Sauerstoff, Alkylamino oder Schwefel steht,
- R⁴ - R⁷: unabhängig voneinander für Wasserstoff, Halogen, Cyano, Thiocyanato oder für jeweils gegebenenfalls durch Halogen substituiertes Alkyl, Alkoxy, Alkylthio, Alkylsulfinyl, Alkylsulfonyl, Alkylamino, Alkylcarbonyl, Alkoxycarbonyl, Alkylaminocarbonyl mit jeweils 1 bis 3 Kohlenstoffatomen stehen,
- R⁸: für Wasserstoff oder einen gegebenenfalls substituierten Rest aus der Reihe Alkyl, Alkenyl, Alkinyl, Cycloalkyl, Cycloalkylalkyl, Aralkyl und Aryl oder für eine Gruppe C(=O)R⁹ steht,
wobei
- R⁹: für Wasserstoff, gegebenenfalls substituiertes Alkyl, gegebenenfalls substituiertes Aryl, Alkoxy, Alkylamino oder Dialkylamino steht,
- R¹⁰: für Wasserstoff, Halogen, Cyano, Thiocyanato oder für jeweils gegebenenfalls durch Halogen substituiertes Alkyl, Alkoxy, Alkylthio, Alkylsulfinyl, Alkylsulfonyl, Alkylamino, Alkylcarbonyl, Alkoxycarbonyl, Alkylaminocarbonyl mit jeweils 1 bis 3 Kohlenstoffatomen steht,
wobei in den vorgenannten Resten die Alkyl- und Alkylengruppen jeweils 1 bis 6 C-Atome, die Alkenyl- und Alkinylgruppen jeweils 2 bis 6 C-Atome, die Cycloalkylgruppen jeweils 3 bis 6 C-Atome und die Arylgruppen jeweils 6 bzw. 10 C-Atome enthalten können,
sowie Salze von Verbindungen der Formel (I) gefunden.

Charakteristisches Strukturmerkmal der neuen Sulfonylharnstoffe (I) ist demnach der (5,6-Dihydro-[1,4,2]-dioxazin-3-yl)-Rest als neuartiger heterocyclischer Substituent im (Het)arylteil der N-Azinyl-N'-(het)arylsulfonyl-harnstoffe. Der einfache, nicht weiter substituierte Grundkörper dieser Verbindungsklasse - das (5,6-Dihydro-[1,4,2]-dioxazin-3-yl)-benzol, welches auch als 3-Phenyl-5H-1,4,2-dioxazin bezeichnet werden kann - ist bekannt (vgl. J.E. Johnson et al., J. Org. Chem., Vol. 36 (2), (1971), S. 284-294).

Man erhält die neuen N-Azinyl-N'-(het)arylsulfonyl-harnstoffe der allgemeinen Formel (I), wenn man
(a) (Het)arylsulfonamide der allgemeinen Formel (II),

   J-SO₂-NH₂ (II)

   in welcher
   - J: die oben angegebenen Bedeutungen hat,
   mit N-Azinylcarbamaten der Formel (III), 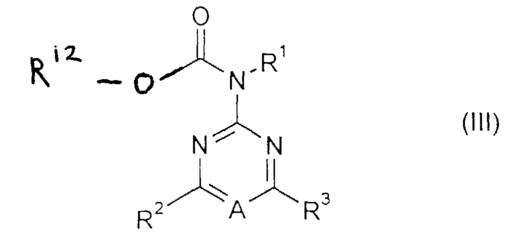 in welcher
   - A und R¹ - R³: die oben angegebenen Bedeutungen haben und
   - R¹²: für Alkyl oder Aryl steht,
   gegebenenfalls in Gegenwart eines Verdünnungsmittels und gegebenenfalls in Gegenwart eines Reaktionshilfsmittels umsetzt, oder wenn man
(b) (Het)arylsulfonylisocyanate der allgemeinen Formel (IV),

   J-SO₂-NCO (IV)

   in welcher
   - J: die oben angegebenen Bedeutungen hat,
   mit Aminoazinen der Formel (V), 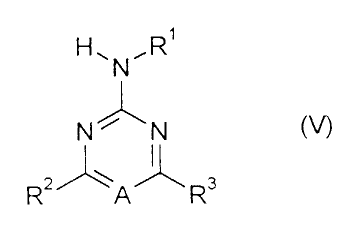 in welcher
   - A und R¹ - R³: die oben angegebenen Bedeutungen haben,
   gegebenenfalls in Gegenwart eines Verdünnungsmittels und gegebenenfalls in Gegenwart eines Reaktionshilfsmittels umsetzt, oder wenn man
(c) N-(Het)arylsulfonylcarbamate der allgemeinen Formel (VI), 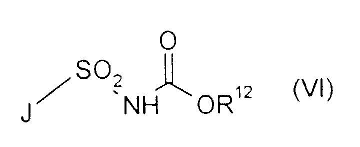 in welcher
   - J und R¹²: die oben angegebenen Bedeutungen haben,
   mit Aminoazinen der Formel (V), 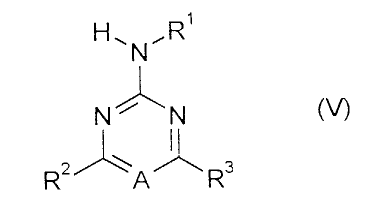 in welcher
   - A und R¹ - R³: die oben angegebenen Bedeutungen haben,
   gegebenenfalls in Gegenwart eines Verdünnungsmittels und gegebenenfalls in Gegenwart eines Reaktionshilfsmittels umsetzt,
   und gegebenenfalls die nach Verfahren (a), (b) oder (c) erhaltenen Produkte nach üblichen Methoden in Salze überführt.

Die neuen N-Azinyl-N'-(het)arylsulfonyl-harnstoffe der Formel (I) zeichnen sich durch starke herbizide Wirksamkeit aus.

Überraschenderweise zeigen die neuen Verbindungen der Formel (I) erheblich stärkere Wirkung als die nach Struktur und Wirkprofil vergleichbaren Verbindungen N-(4,6-Dimethylpyrimidin-2-yl)-N'-(2-methoxyaminocarbonyl-phenylsulfonyl)-harnstoff und der entsprechende -N'-(2-n-octyloxyaminocarbonyl-phenylsulfonyl)-harnstoff.

Gegenstand der Erfindung sind vorzugsweise Verbindungen der Formel (I),
in welcher
- A: für Stickstoff oder eine CH-Gruppierung steht,
- R¹: für Wasserstoff oder einen gegebenenfalls durch Halogen substituierten Rest aus der Reihe Alkyl, Alkoxy, Alkoxyalkyl, Alkenyl und Alkinyl mit jeweils bis zu 3 Kohlenstoffatomen steht,
- R²: für Wasserstoff, Halogen oder für jeweils gegebenenfalls durch Halogen substituiertes Alkyl, Alkoxy, Alkylthio, Alkylamino oder Dialkylamino mit jeweils 1 bis 3 Kohlenstoffatomen in den Alkylresten steht,
- R³: für Wasserstoff, Halogen oder für jeweils gegebenenfalls durch Halogen substituiertes Alkyl, Alkoxy, Alkylthio, Alkylamino oder Dialkylamino mit jeweils 1 bis 3 Kohlenstoffatomen in den Alkylresten steht,
- J: für J-1 bis J-4 steht,
- E: für eine direkte Bindung, Methylen, Sauerstoff, Alkylamino mit 1 bis 3 Kohlenstoffatomen oder Schwefel steht,
- R⁴ - R⁷: unabhängig voneinander für Wasserstoff, Halogen, Cyano, Thiocyanato oder für jeweils gegebenenfalls durch Halogen substituiertes Alkyl, Alkoxy, Alkylthio, Alkylsulfinyl, Alkylsulfonyl, Alkylamino, Alkylcarbonyl, Alkoxycarbonyl oder Alkylaminocarbonyl mit jeweils 1 bis 3 Kohlenstoffatomen in den Alkylresten steht,
- R⁸: für Wasserstoff oder einen gegebenenfalls substituierten Rest aus der Reihe C₁-C₄-Alkyl, C₃-C₆-Cycloalkyl, C₇-C₁₁-Aralkyl und C₆-C₁₀-Aryl steht,
- R¹⁰: für Wasserstoff, Halogen, Cyano, Thiocyanato oder für jeweils gegebenenfalls durch Halogen substituiertes Alkyl, Alkoxy, Alkylthio, Alkylsulfinyl, Alkylsulfonyl, Alkylamino, Alkylcarbonyl, Alkoxycarbonyl oder Alkylaminocarbonyl mit jeweils 1 bis 3 Kohlenstoffatomen in den Alkylresten steht.

Die Erfindung betrifft weiter vorzugsweise Salze, die man aus Verbindungen der Formel (I) und Basen, wie z.B. Natrium-, Kalium- oder Calcium-hydroxid, -hydrid, -amid und -carbonat, Natrium- oder Kalium-C₁-C₄-alkanolaten, Ammoniak, C₁-C₄-Alkylaminen, Di-(C₁-C₄-alkyl)-aminen oder Tri-(C₁-C₄-alkyl)-aminen, nach üblichen Verfahren erhält.

Die Erfindung betrifft insbesondere Verbindungen der Formel (I),
in welcher
- A: für Stickstoff oder eine CH-Gruppierung steht,
- R¹: für Wasserstoff, Methyl, Ethyl, Methoxy, Methoxymethyl oder Ethoxy steht,
- R²: für Wasserstoff, Chlor, Methyl, Ethyl, Trifluoromethyl, Methoxy, Ethoxy, Difluoromethoxy, Methylthio, Methylamino oder Dimethylamino steht,
- R³: für Wasserstoff, Chlor, Methyl, Ethyl, Trifluoromethyl, Methoxy, Ethoxy, Difluoromethoxy, Methylthio, Methylamino oder Dimethylamino steht,
- J: für J-1 bis J-4 steht,
- E: für eine direkte Bindung, Methylen, Sauerstoff, C₁-C₂-Alkylamino oder Schwefel steht,
- R⁴ - R⁷: unabhängig voneinander für Wasserstoff, Fluor, Chlor, Cyano, oder für jeweils gegebenenfalls durch Chlor oder Fluor substituiertes Methyl, Methylthio, Methylsulfinyl, Methylsulfonyl, Methoxycarbonyl und Ethoxycarbonyl steht,
- R⁸: für Wasserstoff, Methyl, Ethyl, Phenyl oder Benzyl steht,
- R¹⁰: für Wasserstoff, Fluor, Chlor, Brom, Cyano oder für jeweils gegebenenfalls durch Chlor oder Fluor substituiertes Methyl, Methoxy, Ethoxy, Methylthio, Ethylthio, Methylsulfinyl, Ethylsulfinyl, Methylsulfonyl, Ethylsulfonyl, Methyl- oder Dimethylamino steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- bzw. Zwischenprodukte. Diese Restedefinitionen können untereinander, also auch zwischen den angegebenen bevorzugten Bereichen beliebig kombiniert werden.

Die bei den Restedefinitionen genannten Kohlenwasserstoffreste, wie Alkyl, Alkenyl oder Alkinyl, auch in Kombinationen mit Heteroatomen, wie in Alkoxy, Alkylthio oder Alkylamino, sind auch dann, wenn dies nicht ausdrücklich angegeben ist, geradkettig oder verzweigt.

Verwendet man beispielsweise für die Verfahrensvariante (a) 2-(5,6-Dihydro-[1,4,2]-dioxazin-3-yl)benzolsulfonamid und (4,6-Dimethoxypyrimidin-2-yl)-phenylcarbamat als Ausgangsstoffe, so kann der Reaktionsverlauf durch das folgende Formelschema skizziert werden: 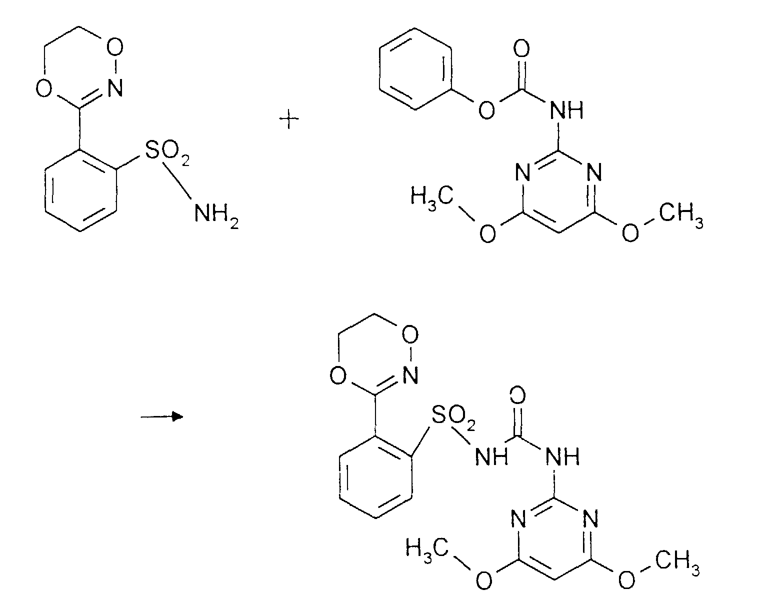 Verwendet man beispielsweise für die Verfahrensvariante (b) 2-(5,6-Dihydro-[1,4,2]-dioxazin-3-yl)benzolsulfonylisocyanat und 2-Amino-4,6-dimethoxypyrimidin als Ausgangsstoffe, so kann der Reaktionsverlauf durch das folgende Formelschema skizziert werden: 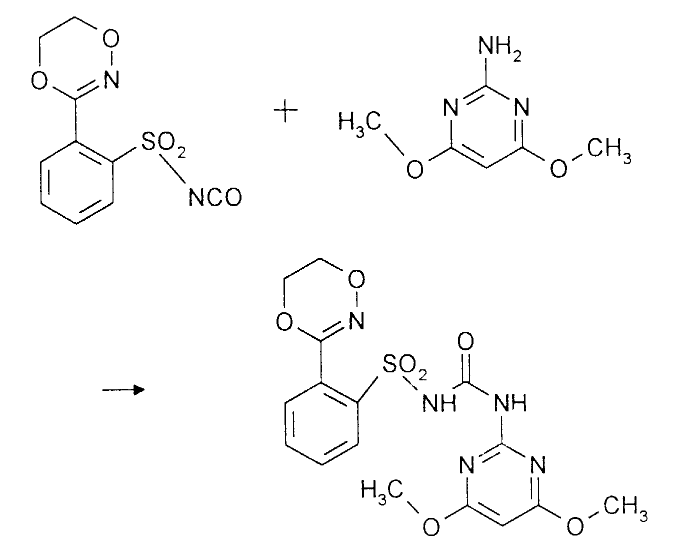 Verwendet man beispielsweise für die Verfahrensvariante (c) N-(2-(5,6-Dihydro-[1,4,2]-dioxazin-3-yl)benzolsulfonyl)-phenylcarbamat und 2-Amino-4,6-dimethoxypyrimidin als Ausgangsstoffe, so kann der Reaktionsverlauf durch das folgende Formelschema skizziert werden: 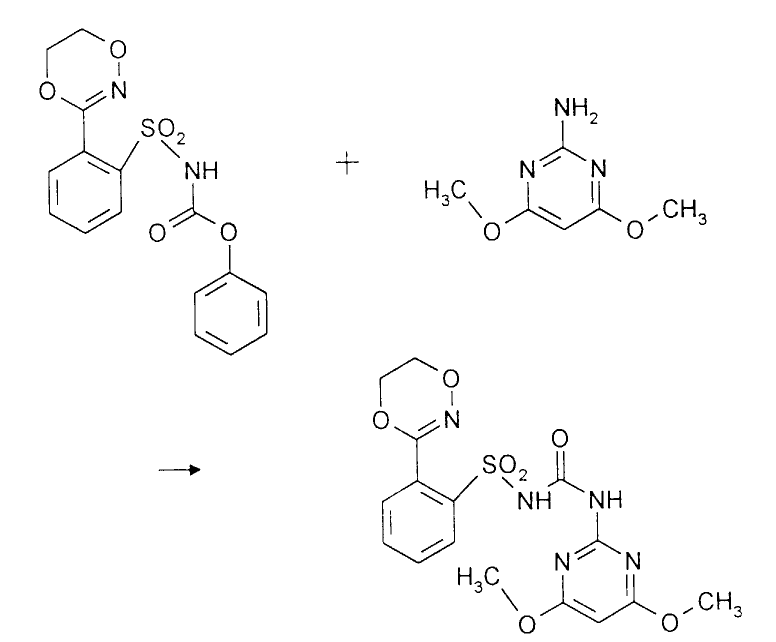 Die beim erfindungsgemäßen Verfahren (a) zur Herstellung von Verbindungen der Formel (I) als Ausgangsstoffe zu verwendenden (Het)arylsulfonamide sind durch Formel (II) allgemein definiert. In Formel (II) hat J vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für J angegeben wurden.

Als Beispiele für die Verbindungen der Formel (II) seien genannt:
2-(5,6-Dihydro[1,4,2]-dioxazin-3-yl)benzolsulfonamid, 2-(5,6-Dihydro-[1,4,2]-dioxazin-3-yl)-6-methyl-benzolsulfonamid, 2-(5,6-Dihydro-[1,4,2]-dioxazin-3-yl)-phenylmethansulfonamid, 3-(5,6-Dihydro-[1,4,2]-dioxazin-3-yl)pyridin-2-sulfonamid, 2-(5,6-Dihydro-[1,4,2]-dioxazin-3-yl)thiophen-3-sulfonamid, 1-Methyl-4-(5,6-dihydro-[1,4,2]-dioxazin-3-yl)pyrazol-5-sulfonamid, 1-Methyl-3-chlor-4-(5,6-dihydro-[1,4,2]-dioxazin-3-yl)-pyrazol-5-sulfonamid.

Die Ausgangsstoffe der Formel (II) sind noch nicht aus der Literatur bekannt und sind als neue Stoffe auch Gegenstand der vorliegenden Erfindung.

Verfahren zur Herstellung der Verbindungen der Formel (II) werden weiter unten beschrieben [vgl. Verfahren (d) und (e)].

Die bei Verfahren (a) außerdem als Ausgangsstoffe zu verwendenden N-Azinylcarbamate sind durch Formel (III) allgemein definiert. In Formel (III) haben A, R¹, R² und R³ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, R¹, R² bzw. R³ angegeben wurden. R¹² steht vorzugsweise für C₁-C₆-Alkyl oder C₆-C₁₀-Aryl, besonders bevorzugt für C₁-C₄-Alkyl oder Phenyl.

Als Beispiele für die Zwischenprodukte der Formel (III) seien genannt:
N-(4,6-Dimethoxypyrimidin-2-yl)-phenylcarbamat, N-(4,6-Diethoxypyrimidin-2-yl)-phenylcarbamat, N-Methyl-N-(4,6-dimethoxypyrimidin-2-yl)-phenylcarbamat, N-Methoxymethyl-N-(4,6-dimethoxypyrimidin-2-yl)-phenylcarbamat, N-(4,6-Dimethylpyrimidin-2-yl)-phenylcarbamat, N-(4,6-Diethylpyrimidin-2-yl)-phenylcarbamat, N-[4,6-Bis(difluormethoxy)pyrimidin-2-yl]-phenylcarbamat, N-[4,6-Bis(dimethylamino)pyrimidin-2-yl]-phenylcarbamat, N-(4-Methyl-6-ethylpyrimidin-2-yl)-phenylcarbamat, N-(4-Methoxy-6-chlorpyrimidin-2-yl)-phenylcarbamat, N-(4-Ethoxy-6-methylaminotriazin-2-yl)-phenylcarbamat, N-(4-Methoxy-6-methyltriazin-2-yl)-phenylcarbamat, N-(4-Isopropoxy-6-chlortriazin-2-yl)-phenylcarbamat, N-(4-Methoxy-6-chlortriazin-2-yl]-phenylcarbamat, N-[4-(2,2,2-Trifluoroethoxy)-6-dimethylaminotriazin-2-yl]phenylcarbamat, N-(4-Trifluoromethyl-6-methoxytriazin-2-yl)-phenylcarbamat, N-(4-Methylamino-6-chlortriazin-2-yl)-phenylcarbamat, N-(4-Methoxy-6-dimethylaminotriazin-2-yl)-phenylcarbamat, N-(4,6-Dimethoxytriazin-2-yl)-phenylcarbamat, N-(4,6-Diethoxytriazin-2-yl)-phenylcarbamat, N-(4,6-Dimethyltriazin-2-yl)-phenylcarbamat, N-(4-Methyl-6-chlorpyrimidin-2-yl)-phenylcarbamat, N-(4-Methoxy-6-methylpyrimidin-2-yl)-phenylcarbamat, N-(4-Methoxy-6-ethoxypyrimidin-2-yl)-phenylcarbamat, N-(4-Methoxy-6-dimethylaminopyrimidin-2-yl)-phenylcarbamat, N-(4-Ethoxy-6-chlorpyrimidin-2-yl)-phenylcarbamat, N-(4-Ethoxy-6-dimethylaminopyrimidin-2-yl)-phenylcarbamat, N-(4-Methyl-6-dimethylaminopyrimidin-2-yl)-phenylcarbamat, N-(4-Methyl-6-isopropoxypyrimidin-2-yl)-phenylcarbamat, N-(4-Dimethylamino-6-chlorpyrimidin-2-yl)-phenylcarbamat, N-[4-(2,2,2-Trifluoroethoxy)-6-chlorpyrimidin-2-yl]-phenylcarbamat, N-(4-Methylamino-6-chlorpyrimidin-2-yl)-phenylcarbamat, N-(4-Difluoromethoxy-6-methylpyrimidin-2-yl)-phenylcarbamat, N-[4-(2,2,2-Trifluoroethoxy-6-methylpyrimidin-2-yl]-phenylcarbamat.

Die Ausgangsstoffe der Formel (III) sind bekannt und/oder können nach an sich bekannten Verfahren hergestellt werden (vgl. EP-238 070).

Die beim erfindungsgemäßen Verfahren (b) zur Herstellung von Verbindungen der Formel (I) als Ausgangsstoffe zu verwendenden (Het)arylsulfonylisocyanate sind durch Formel (IV) allgemein definiert. In Formel (IV) hat J vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für J angegeben wurden.

Als Beispiele für die Ausgangsstoffe der Formel (IV) seien genannt:
2-(5,6-Dihydro-[1,4,2]-dioxazin-3-yl)benzolsulfonylisocyanat, 2-(5,6-Dihydro-[1,4,2]-dioxazin-3-yl)-6-methyl-benzolsulfonylisocyanat, 2-(5,6-Dihydro-[1,4,2]-dioxazin-3-yl)phenylmethansulfonylisocyanat, 3-(5,6-Dihydro-[1,4,2]-dioxazin-3-yl)-pyridin-2-sulfonylisocyanat, 2-(5,6-Dihydro-[1,4,2]-dioxazin-3-yl)thiophen-3-sulfonylisocyanat, 1-Methyl-4-(5,6-dihydro-[1,4,2]-dioxazin-3-yl)pyrazol-5-sulfonylisocyanat, 1-Methyl-3-chlor-4-(5,6-dihydro-[1,4,2]-dioxazin-3-yl)pyrazol-5-sulfonyl-isocyanat.

Die Ausgangsstoffe der Formel (IV) sind noch nicht aus der Literatur bekannt und sind als neue Stoffe auch Gegenstand der vorliegenden Erfindung.

Man erhält die neuen (Het)arylsulfonylisocyanate der Formel (IV), wenn man (Het)arylsulfonamide der obigen Formel (II) mit Phosgen in Gegenwart eines Verdünnungsmittels, wie z.B. Chlorbenzol, und von Reaktionshilfsmitteln, wie z.B. Butylisocyanat und Diazabicyclooctan (DABCO), bei Temperaturen zwischen 0°C und 200°C, vorzugsweise zwischen 20°C und 160°C, umsetzt und anschließend die flüchtigen Komponenten unter vermindertem Druck sorgfältig abdestilliert (vgl. EP-162 723).

Die beim erfindungsgemäßen Verfahren (b) zur Herstellung von Verbindungen der Formel (I) weiter als Ausgangsstoffe benötigten Aminoazine sind durch Formel (V) allgemein definiert. In Formel (V) haben A, R¹, R² und R³ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹, R² und R³ angegeben wurden.

Als Beispiele für die Ausgangsstoffe der Formel (V) seien genannt:
2-Amino-4,6-dimethoxypyrimidin, 2-Amino-4,6-diethoxypyrimidin, 2-Methylamino-4,6-dimethoxypyrimidin, 2-Methoxymethylamino-4,6-dimethoxypyrimidin, 2-Amino-4,6-dimethylpyrimidin, 2-Amino-4,6-diethylpyrimidin, 2-Amino-4,6-bis(difluormethoxy)pyrimidin, 2-Amino-4,6-bis(dimethylamino)pyrimidin, 2-Amino-4-methyl-6-ethylpyrimidin, 2-Amino-4-methoxy-6-chlorpyrimidin, 2-Amino-4-ethoxy-6-methylaminotriazin, 2-Amino-4-methoxy-6-methyltriazin, 2-Amino-4-isopropoxy-6-chlortriazin, 2-Amino-4-methoxy-6-chlortriazin, 2-Amino-4-(2,2,2-trifluorethoxy)-6-dimethylaminotriazin, 2-Amino-4-trifluormethyl-6-methoxytriazin, 2-Amino-4-methylamino-6-chlortriazin, 2-Amino-4-methoxy-6-dimethylaminotriazin, 2-Amino-4,6-dimethoxytriazin, 2-Amino-4,6-diethoxytriazin, 2-Amino-4,6-dimethyltriazin, 2-Amino-4-methyl-6-chlorpyrimidin, 2-Amino-4-methoxy-6-methylpyrimidin, 2-Amino-4-methoxy-6-ethoxypyrimidin, 2-Amino-4-methoxy-6-dimethylaminopyrimidin, 2-Amino-4-ethoxy-6-chlorpyrimidin, 2-Amino-4-ethoxy-6-dimethylaminopyrimidin, 2-Amino-4-methyl-6-dimethylaminopyrimidin, 2-Amino-4-methyl-6-isopropoxypyrimidin, 2-Amino-4-dimethylamino-6-chlorpyrimidin, 2-Amino-4-(2,2,2-trifluorethoxy)-6-chlorpyrimidin, 2-Amino-4-methylamino-6-chlorpyrimidin, 2-Amino-4-difluormethoxy-6-methylpyrimidin, 2-Amino-4-(2,2,2-trifluorethoxy)-6-methylpyrimidin.

Die Ausgangsstoffe der Formel (V) sind bekannt und/oder können nach an sich bekannten Verfahren hergestellt werden (vgl. Huaxue Shijie, 32(6), 254-7 (1991); JP-01 016 770; EP-246 984); zum Teil sind diese Stoffe im Handel erhältliche Synthesechemikalien.

Die beim erfindungsgemäßen Verfahren (c) zur Herstellung von Verbindungen der Formel (I) als Ausgangsstoffe zu verwendenden (Het)arylsulfonylcarbamate sind durch Formel (VI) allgemein definiert. In Formel (VI) haben J und R¹² vorzugsweise bzw. insbesondere diejenigen Bedeutungen, die bereits oben im Zusammenhang mit der Beschreibung der erfindungsgemäßen Verbindungen der Formeln (I) bzw. (III) vorzugsweise bzw. als insbesondere bevorzugt für J bzw. R¹² angegeben wurden.

Als Beispiele für die Ausgangsstoffe der Formel (VI) seien genannt:
N-(2-(5,6-Dihydro-[1,4,2]-dioxazin-3-yl)benzolsulfonyl)-phenylcarbamat, N-(2-(5,6-Dihydro-[1,4,2]-dioxazin-3-yl)-6-methyl-benzolsulfonyl)-phenylcarbamat, N-(2-(5,6-Dihydro-[1,4,2]-dioxazin-3-yl)phenylmethansulfonyl)-phenylcarbamat, N-(3-(5,6-Dihydro-[1,4,2]-dioxazin-3-yl)pyridin-2-sulfonyl)-phenylcarbamat, N-(2-(5,6-Dihydro-[1,4,2]-dioxazin-3-yl)thiophen-3-sulfonyl)-phenylcarbamat, N-(1-Methyl-4-(5,6-dihydro-[1,4,2]-dioxazin-3-yl)pyrazol-5-sulfonyl)-phenylcarbamat, N-(1-Methyl-3-chlor-4-(5,6-dihydro-[1,4,2]-dioxazin-3-yl)pyrazol-5-sulfonyl)-phenylcarbamat.

Die Ausgangsstoffe der Formel (VI) sind noch nicht aus der Literatur bekannt und sind als neue Stoffe auch Gegenstand der vorliegenden Erfindung.

Man erhält die neuen (Het)arylsulfonylcarbamate der Formel (VI), wenn man (Het)arylsulfonamide der obigen Formel (II) mit Chlorameisensäureester in Gegenwart eines Verdünnungsmittels, wie z.B. Dioxan oder Acetonitril, und von Reaktionshilfsmitteln, wie z.B. Pyridin, Kalium- oder Calciumcarbonat, bei Temperaturen zwischen 0°C und 120°C, vorzugsweise zwischen 20°C und 100°C, umsetzt und anschließend die flüchtigen Komponenten unter vermindertem Druck sorgfältig abdestilliert (vgl. JP-04139170).

Die beim erfindungsgemäßen Verfahren (c) weiter als Ausgangsstoffe benötigten Aminoazine der Formel (V) sind bereits oben bei Verfahren (b) näher beschrieben worden.

Die neuen, oben beschriebenen und nahe miteinander verwandten Ausgangs- bzw. Zwischenprodukte der Formeln (II), (IV) und (VI) können zusammenfassend als "(Het)arylsulfonylverbindungen" bezeichnet und durch folgende Formel (XI) dargestellt werden:

J-SO₂-G (XI),

in welcher
- J: die oben bei Formel (I) genannten Bedeutungen hat und
- G: für -NH₂, -N=C=O oder -NH-COOR¹² steht, wobei R¹² die oben angegebene Bedeutung hat.
Es wurde weiterhin gefunden, daß man die oben beschriebenen (Het)arylsulfonamide der Formel (II) erhält, wenn man
(d) (Het)arylthiobenzylether der Formel (VII), 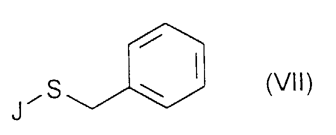 in welcher
   - J: die oben bei Formel (I) genannten Bedeutungen hat,
   mit einem Chlorierungsmittel und Wasser in Gegenwart eines inerten organischen Verdünnungsmittels und gegebenenfalls eines Reaktionshilfsmittels umsetzt und die so erhaltenen Sulfonylchloride mit Ammoniak oder einem Ammoniumsalz in Gegenwart eines inerten organischen Verdünnungsmittels und gegebenenfalls eines Reaktionshilfsmittels umsetzt (vgl. EP-232 067, EP-451 468, US-5 157 119).
   Verwendet man beispielsweise für das Verfahren (d) 1-Benzylthio-2-(5,6-dihydro-[1,4,2]-dioxazin-3-yl)benzol, Chlor, Wasser und Ammoniak als Ausgangsstoffe, so kann der Reaktionsverlauf durch das folgende Reaktionsschema skizziert werden: 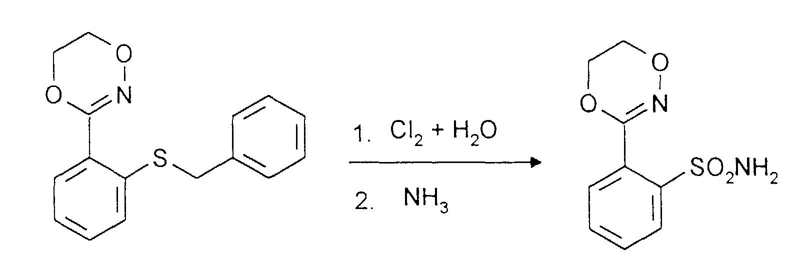
(e) Alternativ erhält man die neuen (Het)arylsulfonamide der Formel (II), wenn man (Het)arylsulfonamide der Formel (VIII)

   T-SO₂NH₂ (VIII)

   in welcher
   - T: für T-1 bis T-4 steht,
   wobei
   T-1 bis T-4 folgende Bedeutungen haben: 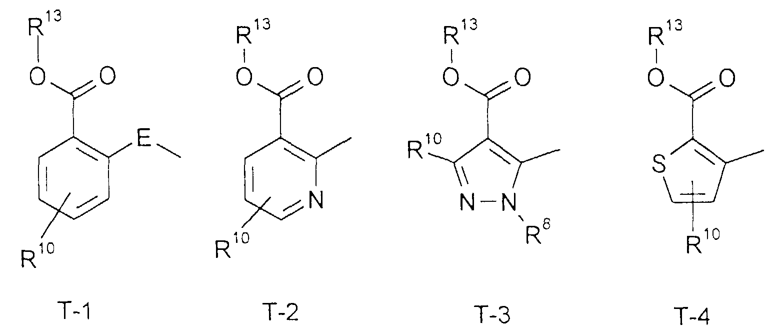
   worin
   - E, R⁸ und R¹⁰: die oben angegebenen Bedeutungen haben und
   - R¹³: für Alkyl oder Aryl, vorzugsweise für C₁-C₄-Alkyl oder Phenyl steht,
   mit Hydroxylamin-Hydrochlorid und einem substituierten Alkan der Formel (IX), 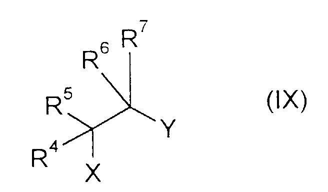 in der
   - R⁴ - R⁷: die oben angegebenen Bedeutungen haben und
   - X und Y: unabhängig voneinander für Halogen, gegebenenfalls substituiertes Alkyl- oder Arylcarbonyloxy oder -sulfonyloxy (vorzugsweise für Chlor, Brom, Iod, C₁-C₆-Alkyl oder C₆-C₁₂-Arylcarbonyloxy oder -sulfonyloxy) stehen,
   gegebenenfalls in Gegenwart eines organischen Verdünnungsmittels und gegebenenfalls in Gegenwart eines Reaktionshilfsmittels umsetzt (vgl. J. Org. Chem., Vol. 36(2), S. 284-294(1971); JP-01 221 371).
   Verwendet man beispielsweise für das Verfahren (e) 1-Methyl-4-ethoxycarbonylpyrazol-5-sulfonamid, Hydroxylamin-Hydrochlorid und 1,2-Dibromethan als Ausgangsstoffe, so kann der Reaktionsverlauf durch das folgende Reaktionsschema skizziert werden: 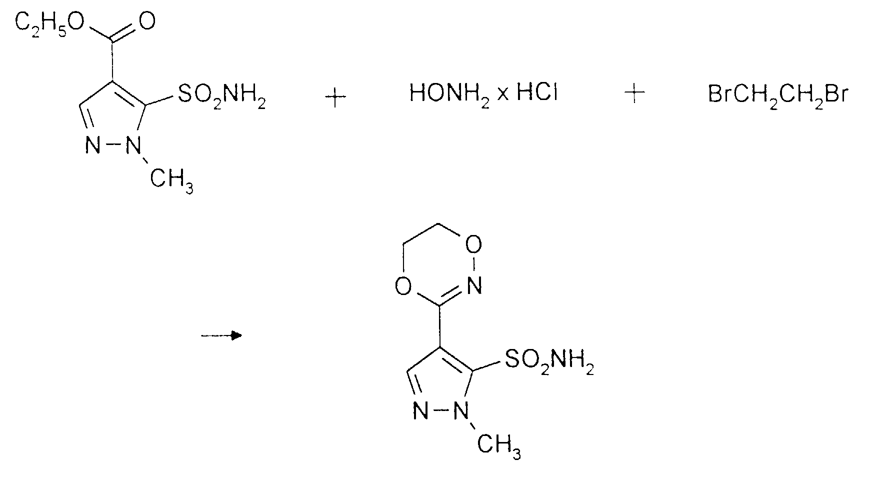 Die beim erfindungsgemäßen Verfahren (d) zur Herstellung von Verbindungen der Formel (II) als Ausgangsstoffe benötigten (Het)arylthiobenzylether sind durch Formel (VII) allgemein definiert. In Formel (VII) hat J vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für J angegeben wurden.
   Als Beispiele für die Zwischenprodukte der Formel (VII) seien genannt:
   1-Benzylthio-2-(5,6-dihydro-[1,2,4]-dioxazin-3-yl)-benzol, 1-Benzylthio-2-(5,6-dihydro-[1,4,2]-dioxazin-3-yl)-6-methyl-benzol, 1-Benzylthiomethyl-2-(5,6-dihydro-[1,4,2]-dioxazin-3-yl)benzol, 2-Benzylthio-3-(5,6-dihydro-[1,4,2]-dioxazin-3-yl)-pyridin, 1-Methyl-4-(5,6-dihydro-[1,4,2]-dioxazin-3-yl)-5-benzylthio-pyrazol, 2-(5,6-Dihydro-[1,4,2]-dioxazin-3-yl)-3-benzylthio-thiophen, 1-Methyl-3-chlor-4-(5,6-dihydro-[1,4,2]-dioxazin-3-yl)-5-benzylthio-pyrazol.
   Die Ausgangsstoffe der Formel (VII) sind noch nicht aus der Literatur bekannt und sind als neue Stoffe ebenfalls Gegenstand der vorliegenden Erfindung.
   Ein Verfahren zur Herstellung der Verbindungen der Formel (VII) wird weiter unten beschrieben (vgl. Verfahren (f)).
   Als Beispiele für die bei Verfahren (d) außerdem als Ausgangsstoffe zu verwendenden Chlorierungsmittel seien genannt:
   Chlor, Sulfurylchlorid, Alkalihypochlorit, Chlorsulfonsäure.
   Die beim erfindungsgemäßen Verfahren (e) zur Herstellung von Verbindungen der Formel (II) als Ausgangsstoffe zu verwendenden (Het)arylsulfonamide sind durch Formel (VIII) allgemein definiert. In der Formel (VIII) hat die Gruppe T diejenigen bevorzugten Bedeutungen, die sich aus den obigen Definitionen der darin enthaltenen Reste E, R⁸, R¹⁰ bzw. R¹³ ergeben.
   Als Beispiele für die Zwischenprodukte der Formel (VIII) seien genannt: 1-Methyl-4-ethoxycarbonyl-pyrazol-5-sulfonamid, 1-Methyl-3-chlor-4-ethoxycarbonyl-pyrazol-5-sulfonamid, 2-Methoxycarbonylbenzolsulfonamid, (2-Methoxycarbonylphenyl)methansulfonamid, 3-Methoxycarbonylpyridin-2-sulfonamid, 2-Methoxycarbonylthiophen-3-sulfonamid.
   Die Ausgangsstoffe der Formel (VIII) sind bekannt und/oder können nach an sich bekannten Verfahren hergestellt werden (vgl. z.B. J. Heterocycl. Chem., 28(8), 1849-52 (1991); Pestic. Sci., 32(1), 91-104 (1991); Nippon Noyaku Gakkaishi, 15(4), 531-8 (1990); JP-63 051 394, JP-61 210 003; Eur. J. Med. Chem., 23(4), 329-34 (1988); ES-547 444, DE-2 706 859, DE-2 534 689).
   Bei bei Verfahren (e) weiterhin als Ausgangsstoffe zu verwendenden substituierten Alkane sind durch die Formel (IX) allgemein definiert. In Formel (IX) haben die Substituenten R⁴, R⁵, R⁶ und R⁷ vorzugsweise bzw. insbesondere diejenigen Bedeutungen, die bereits oben im Zusammenhang mit der Beschreibung der Verbindungen der Formel (I) vorzugsweise bzw. als insbesondere bevorzugt für R⁴-R⁷ angegeben wurden; die reaktiven, vicinalen Abgangsgruppen X und Y haben die bereits bei Formel (IX) angegebenen bevorzugten Bedeutungen.
   Als Beispiele für besonders geeignete Verbindungen der Formel (IX) seien genannt:
   1,2-Dichlorethan, 1,2-Dibromethan, 1,2-Diiodethan, 1,2-Bis-(methansulfonyloxy)ethan, 1,2-Bis-(trifluormethansulfonyloxy)ethan, 1,2-Bis-(trifluoracetyloxy)-ethan, 1,2-Bis-(4-toluolsulfonyloxy)ethan.
   Die Ausgangsstoffe der Formel (IX) sind durchweg bekannte, zum Teil im Handel erhältliche Synthesechemikalien.
   Schließlich wurde gefunden, daß man die neuen, für Verfahren (d) benötigten (Het)arylthiobenzylether der obigen Formel (VII) erhält, wenn man
(f) (Het)arylthiobenzylcarbonsäureester der Formel (X) 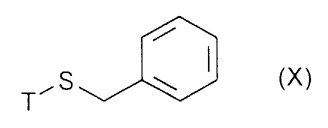 in welcher
   - T: die oben bei Verfahren (e) angegebenen Bedeutungen hat,
   mit Hydroxylamin-Hydrochlorid und einem substituierten Alkan der Formel (IX), 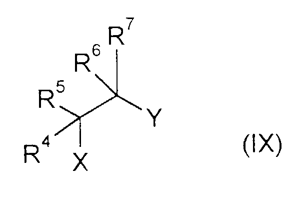 in der
   - R⁴ - R⁷: die oben angegebenen Bedeutungen haben und
   - X und Y: unabhängig voneinander für Halogen, gegebenenfalls substituiertes Alkyl- oder Arylcarbonyloxy oder -sulfonyloxy (vorzugsweise für Chlor, Brom, Iod, C₁-C₆-Alkyl- oder C₆-C₁₂-Arylcarbonyloxy oder -sulfonyloxy) stehen,
   gegebenenfalls in Gegenwart eines organischen Verdünnungsmittels und gegebenenfalls in Gegenwart eines Reaktionshilfsmittels umsetzt.

Verwendet man beispielsweise für das Verfahren (f) 2-Benzylmercaptobenzoesäuremethylester, Hvdroxylamin-Hydrochlorid und 1,2-Dibromethan als Ausgangsstoffe, so kann der Reaktionsverlauf durch das folgende Reaktionsschema skizziert werden: 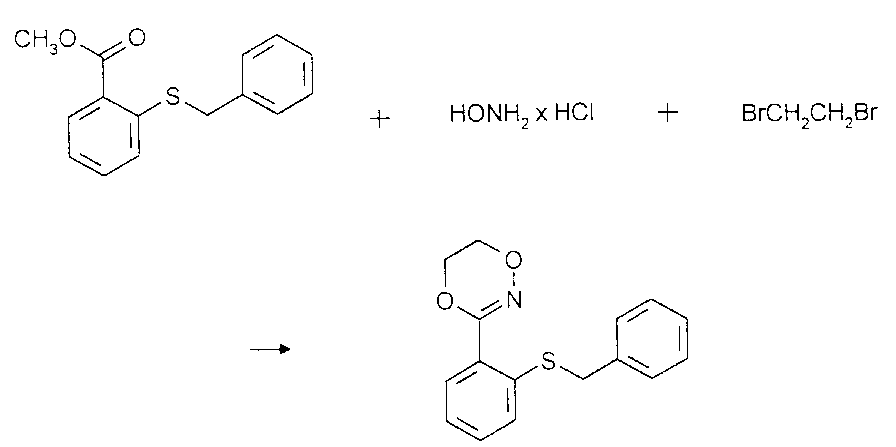 Die beim erfindungsgemäßen Verfahren (f) zur Herstellung von Verbindungen der Formel (VII) als Ausgangsstoffe benötigten (Het)arylthiobenzylcarbonsäureester sind durch Formel (X) allgemein definiert; für die Bedeutung der Gruppe T gilt das oben bei Verfahren (e) dazu Gesagte.

Als Beispiele für die Zwischenprodukte der Formel (X) seien genannt: 2-Benzylmercaptobenzoesäuremethylester, 2-Benzylmercapto-6-methyl-benzoesäuremethylester, (2-Benzylmercaptophenyl)-essigsäuremethylester, 2-Benzylmercaptopyridin-3-carbonsäuremethylester, 1-Methyl-5-benzylmercaptopyrazol-4-carbonsäuremethylester, 1-Methyl-3-chlor-5-benzylmercaptopyrazol-4-carbonsäuremethylester, 3-Benzylmercaptothiophen-2-carbonsäuremethylester.

Die Ausgangsstoffe der Formel (X) sind bekannt und/oder können nach an sich bekannten Verfahren hergestellt werden (vgl. z.B. Chem. Pharm. Bull., 34(2), 540-9 (1986), J. Heterocycl. Chem. 15(3), 513-14 (1978)).

Die bei Verfahren (f) weiter als Ausgangsstoffe zu verwendenden substituierten Alkane der Formel (IX) sind bereits oben bei Verfahren (e) näher beschrieben worden.

Die erfindungsgemäßen Verfahren (a), (b) und (c) zur Herstellung der neuen Verbindungen (I), die erfindungsgemäßen Verfahren (d) und (e) zur Herstellung der neuen Zwischenprodukte der Formel (II) sowie das erfindungsgemäße Verfahren (f) zur Herstellung der neuen Zwischenprodukte der Formel (VII) werden vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise - außer für Verfahren (d) - aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Chlorbenzol und o-Dichlorbenzol, Ether wie Diethyl- und Dibutylether, Glykoldimethylether und Diglycoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Für das Chlorierungsverfahren (d) kommen als Verdünnungsmittel hauptsächlich nur die vorerwähnten chlorierten Lösungsmittel in Betracht.

Die erfindungsgemäßen Verfahren (a), (e) und (f) werden gegebenenfalls in Gegenwart eines Reaktionshilfsmittels durchgeführt. Hierzu gehören vorzugsweise basische organische Stickstoffverbindungen, wie z.B. Trimethylamin, Triethylamin, N,N-Dimethylanilin, N,N-Dimethylbenzylamin, Pyridin, 4-Dimethylaminopyridin, Diazabicyclooctan (DABCO), Diazabicyclononen (DBN) und Diazabicycloundecen (DBU), sowie anorganische Basen, wie z.B. Natriumhydrid, Kaliumhydrid, Natriumcarbonat, Kaliumcarbonat, Calciumcarbonat, Kaliumhydroxid, Natriumhydroxid, und Calciumhydroxid.

Auch die erfindungsgemäßen Verfahren (b) und (c) werden gegebenenfalls in Gegenwart eines Reaktionshilfsmittels durchgeführt. Hierfür geeignet sind vorzugsweise basische organische Stickstoffverbindungen, wie z.B. Trimethylamin, Triethylamin, N,N-Dimethylanilin, N,N-Dimethylbenzylamin, Pyridin, 4-Dimethylaminopyridin, Diazabicyclooctan (DABCO), Diazabicyclononen (DBN) und Diazabicycloundecen (DBU).

Das Chlorierungsverfahren (d) kann ebenfalls in Gegenwart eines Reaktionshilfsmittels durchgeführt werden. Besonders geeignet hierfür sind bestimmte mineralische und organische Säuren, wie z.B. Salzsäure, verdünnte Schwefelsäure, Phosphorsäure, Essigsäure oder Propionsäure, ferner saure Salze wie z.B. NaH₂PO₄.

Die Reaktionstemperaturen können bei den Verfahren (a) bis (f) jeweils in einem größeren Bereich variiert werden. So arbeitet man
- bei Verfahren (a) im allgemeinen bei Temperaturen zwischen 0°C und 150°C, vorzugsweise bei Temperaturen zwischen 10°C und 80°C;
- bei Verfahren (b) im allgemeinen bei Temperaturen zwischen 0°C und 150°C, vorzugsweise bei Temperaturen zwischen 20°C und 100°C;
- bei Verfahren (c) im allgemeinen ebenfalls bei Temperaturen zwischen 0°C und 150°C, vorzugsweise bei Temperaturen zwischen 20°C und 100°C;
- bei Verfahren (d) im allgemeinen bei Temperaturen zwischen -20°C und +40°C, vorzugsweise bei Temperaturen zwischen -20°C und +30°C;
- bei Verfahren (e) im allgemeinen bei Temperaturen zwischen 0°C und 150°C, vorzugsweise bei Temperaturen zwischen 10°C und 80°C und
- bei Verfahren (f) im allgemeinen gleichfalls bei Temperaturen zwischen 0°C und 150°C, vorzugsweise bei Temperaturen zwischen 10°C und 80°C.

Die Verfahren (a) bis (f) werden im allgemeinen unter Normaldruck durchgeführt. Es ist jedoch jeweils auch möglich, unter erhöhtem oder vermindertem Druck zu arbeiten.

Zur Durchführung der erfindungsgemäßen Verfahren (a) bis (f) werden die jeweils benötigten Ausgangsstoffe in annähernd äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel und gegebenenfalls unter Zusatz eines Reaktionshilfsmittels durchgeführt, und das Reaktionsgemisch wird mehrere Stunden bei der jeweils erforderlichen Temperatur gerührt. Die Aufarbeitung erfolgt bei den erfindungsgemäßen Verfahren jeweils nach üblichen Methoden.

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Verdünnungsmittel, wie z.B. Methylenchlorid, Aceton, tert.-Butyl-methylether oder Toluol, und Zugabe einer geeigneten Base. Die Salze können dann - gegebenenfalls nach längerem Rühren - durch Einengen oder Absaugen isoliert werden (vgl. die Herstellbeispiele).

Die erfindungsgemäßen Wirkstoffe können als Defoliants, Desiccants, Krautabtötungsmittel und insbesondere als Unkrautvernichtungsmittel verwendet werden.Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Zitrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich zur selektiven Bekämpfung von monokotylen und dikotylen Unkräutern in verschiedenen Kulturen, teilweise z.B. in Weizen, sowohl im Vorauflauf- als auch im Nachauflauf-Verfahren.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel könne z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser.

Als feste Trägerstoffe kommen infrage: z.B. Ammoniumsalze und natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl, Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuronethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamte, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen; Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro Hektar.

Die Herstellung und die Verwendung der erfindungsgemäßen Wirkstoffe geht aus den nachfolgenden Beispielen hervor.

### Herstellungsbeispiele:

### Beispiel I-1

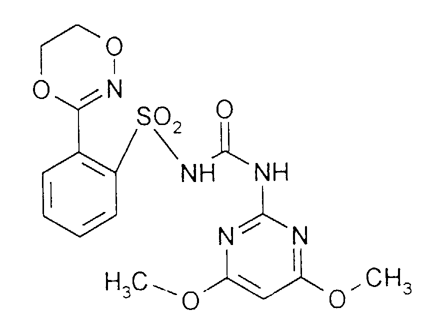

### (Verfahren (a))

Zu einer Mischung von 4.0 g (0.017 Mol) 2-(5,6-Dihydro-[1,4,2]-dioxazin-3-yl)benzolsulfonamid und 4.6 g (0.017 Mol) 2-Phenoxycarbonylamino-4,6-dimethoxypyrimidin in 20 ml absolutem Acetonitril gibt man unter Argon 0.79 g (0.033 Mol) Natriumhydrid und rührt 18 Stunden bei Raumtemperatur. Der Niederschlag wird abgesaugt und mit 20-prozentiger Natriumdihydrogenphosphatlösung verrührt. Der Niederschlag wird abgesaugt und im Hochvakuum getrocknet. Man erhält 3.9 g (56% der Theorie) N-(4,6-Dimethoxypyrimidin-2-yl)-N'-(2-(5,6-dihydro-[1,4,2]-dioxazin-3-yl)benzolsulfonyl)-harnstoff vom Schmelzpunkt 189° C.

Analog Beispiel I-1 und entsprechend der allgemeinen Beschreibung der erfindungsgemäßen Herstellungsverfahren können beispielsweise auch die weiteren, in den nachstehenden Tabellen 1, 2, 3 und 4 aufgeführten Verbindungen der Formel (I) hergestellt werden.

Abkürzungen:
Smp.: = Schmelzpunkt
Zers. oder Z. = unter Zersetzung
⁽⁺⁾ = Der angegebene Schmelzpunkt (Smp.) bezieht sich jeweils auf das entsprechende Natriumsalz.

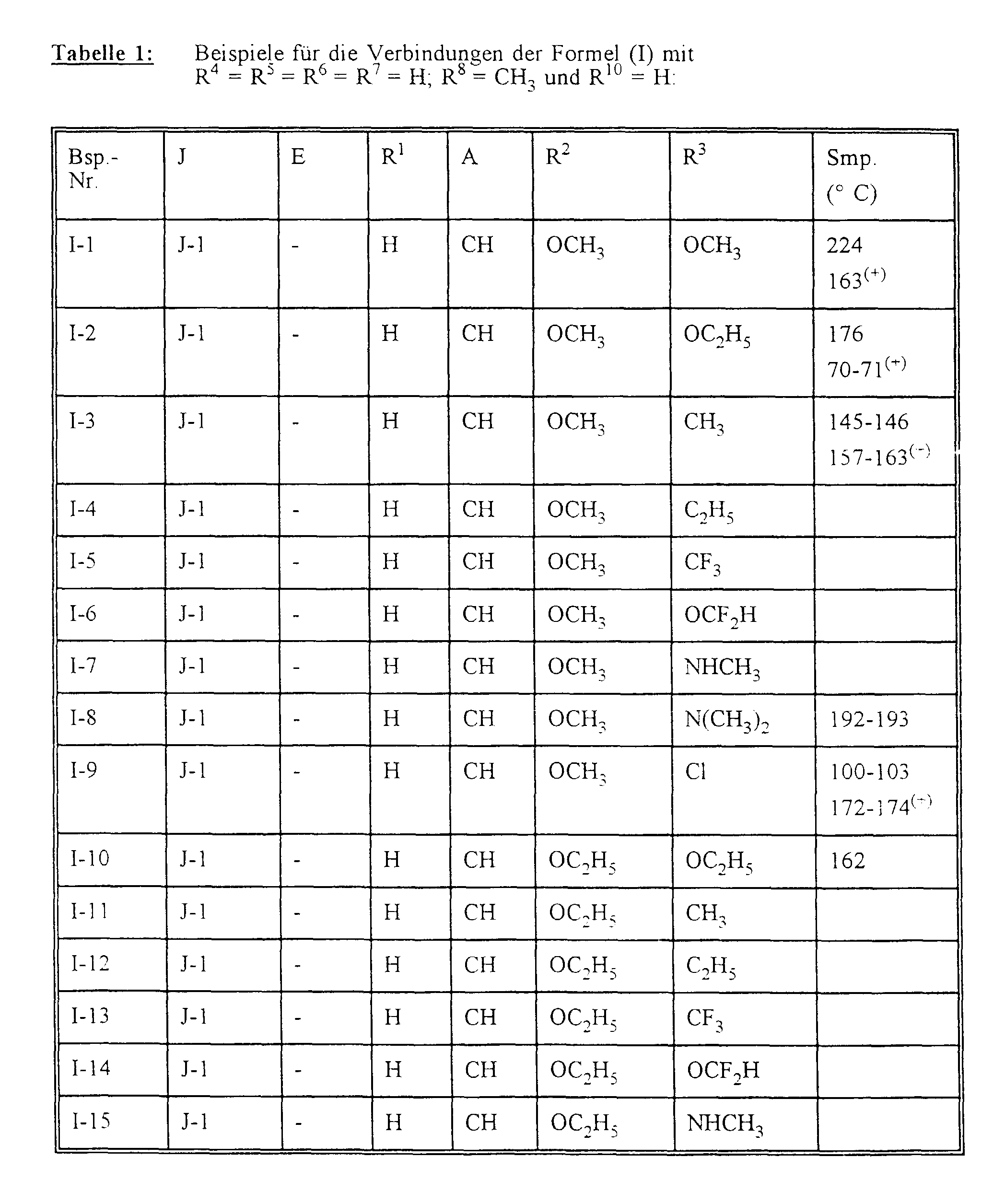

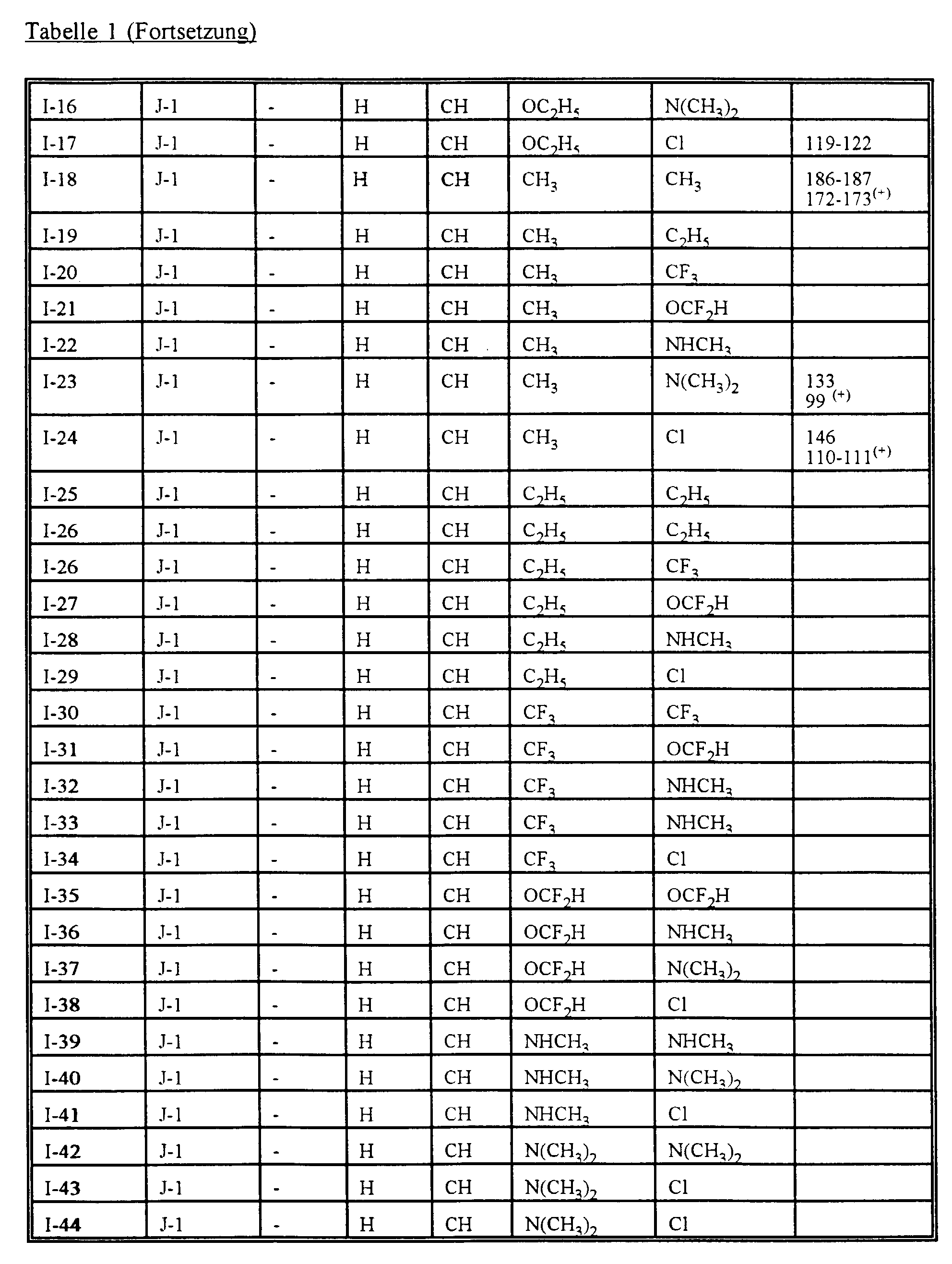

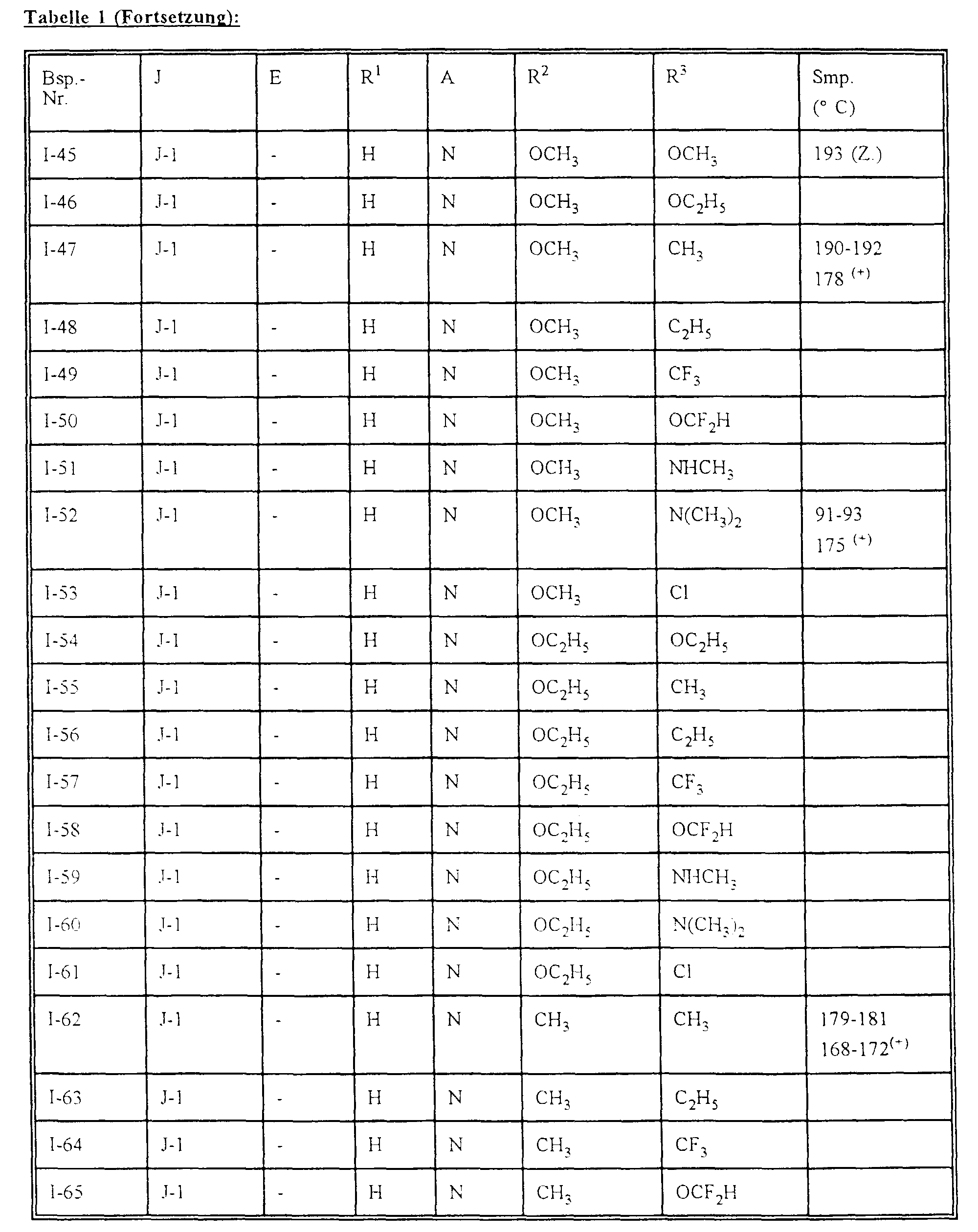

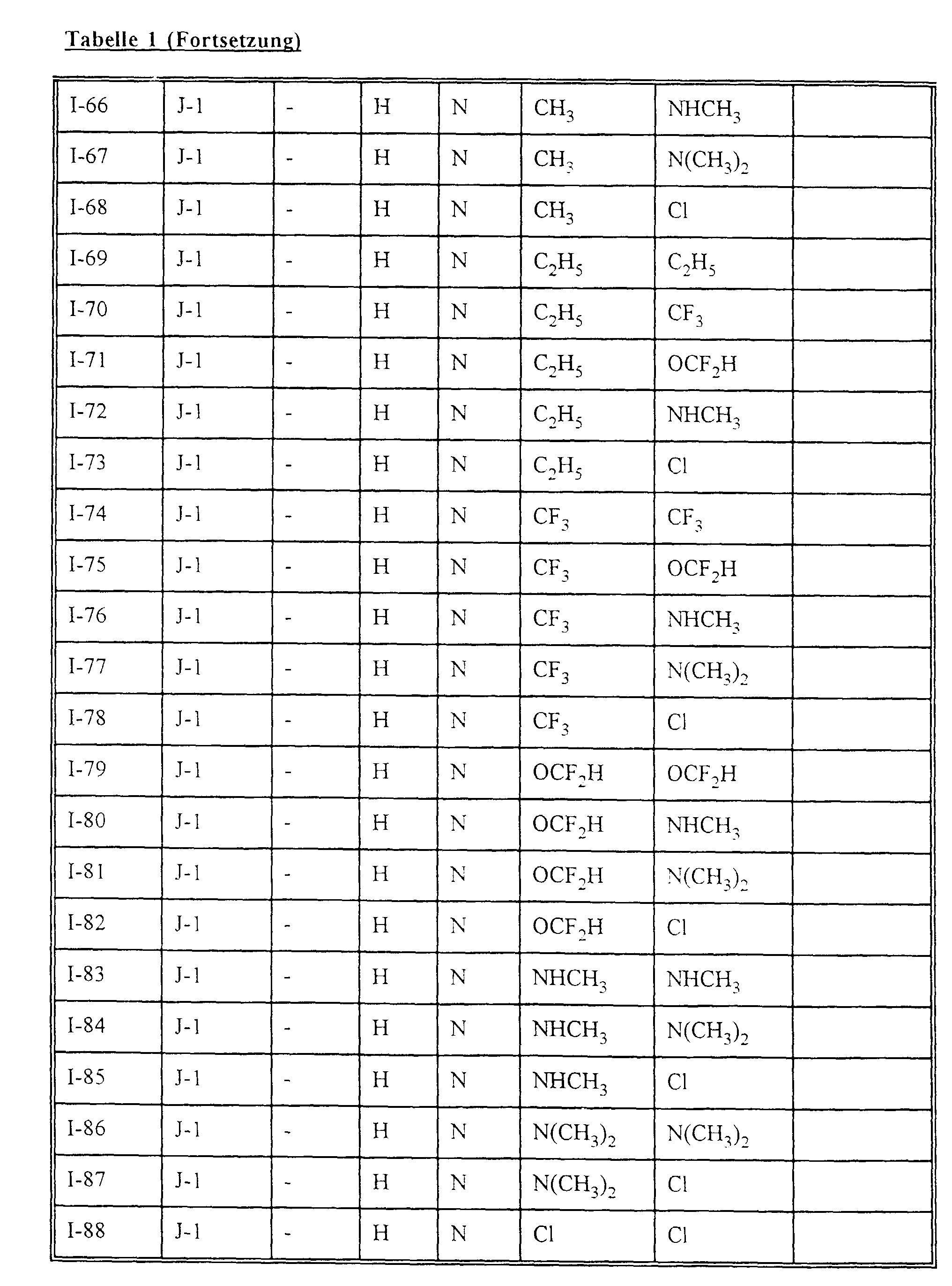

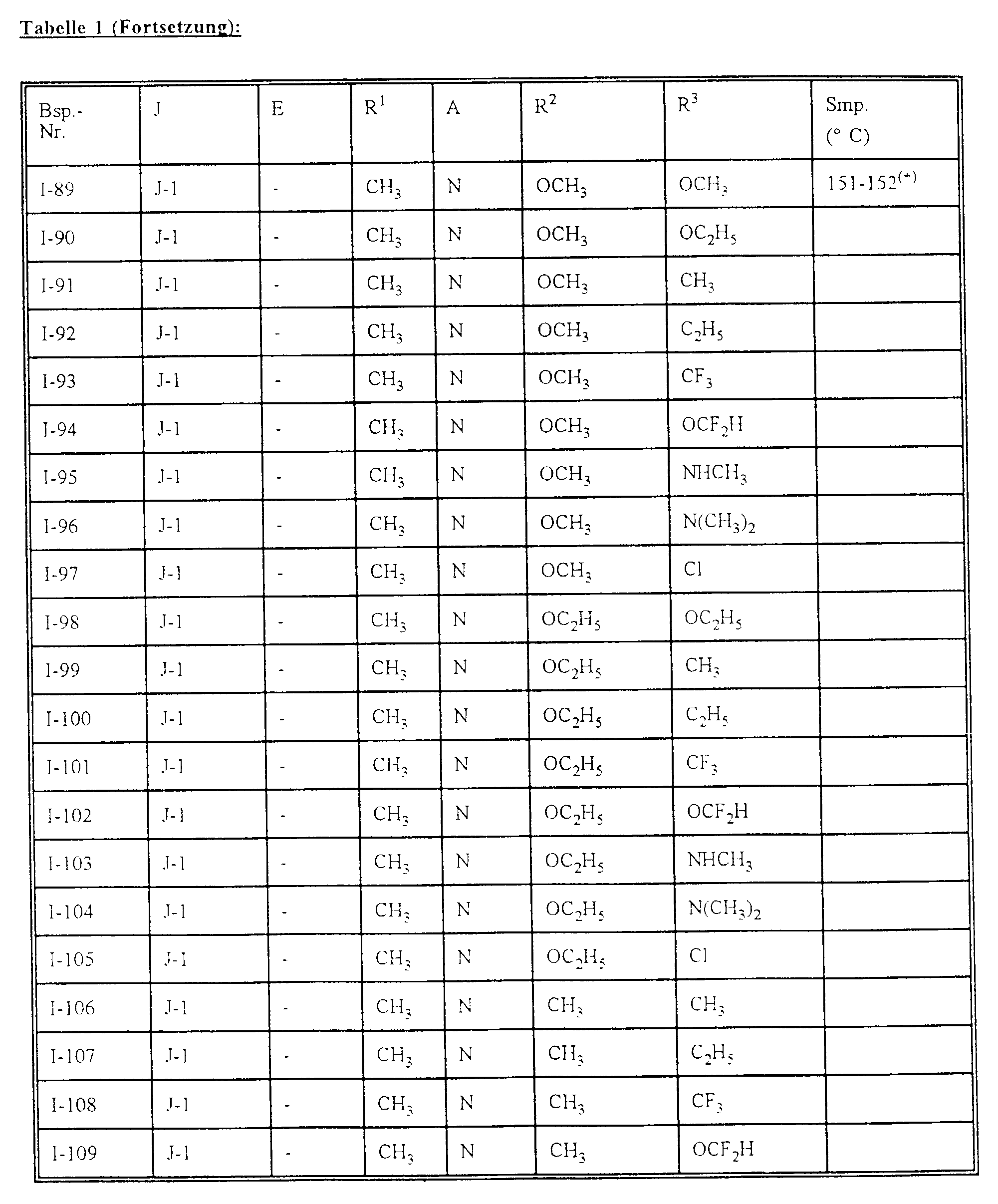

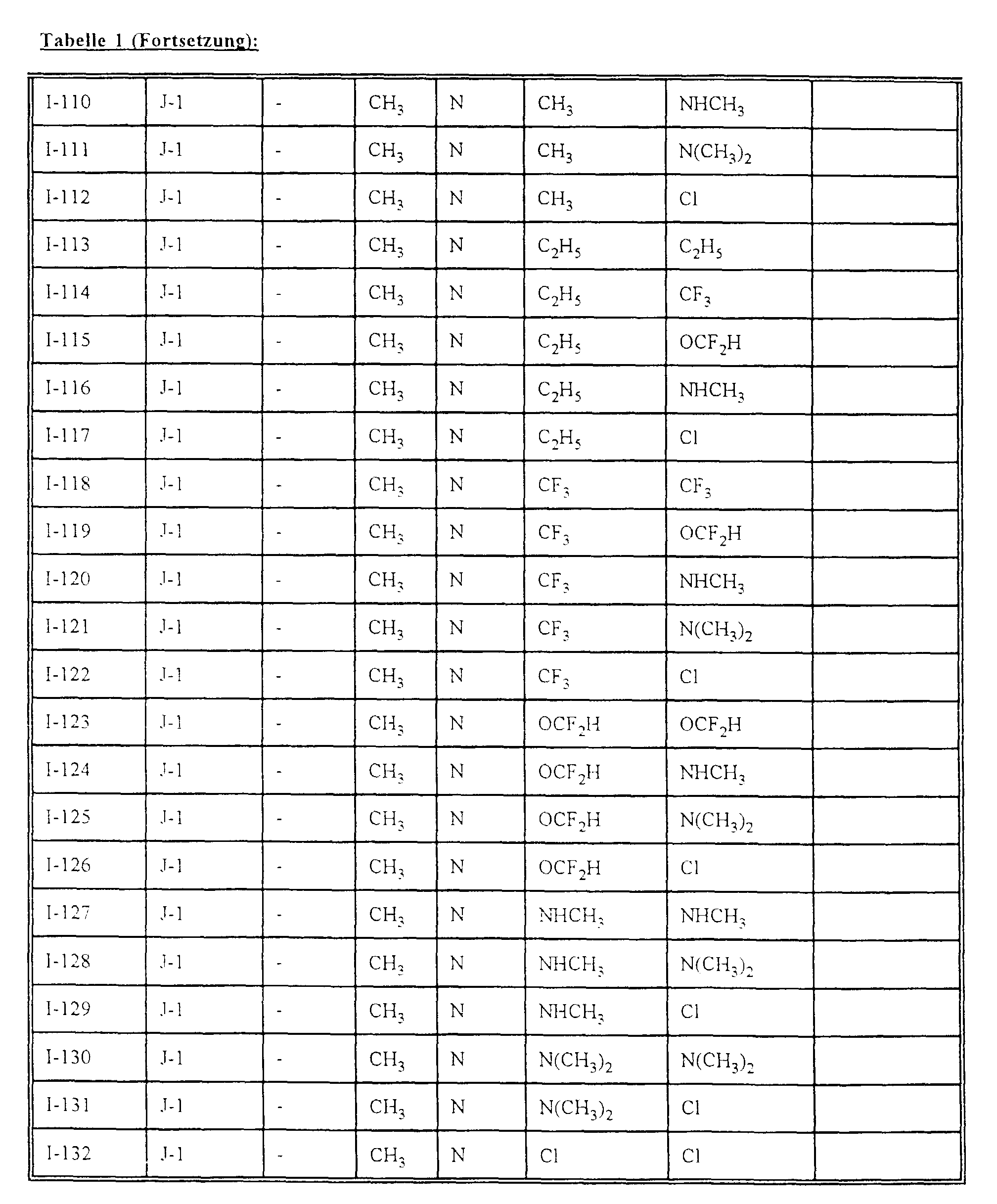

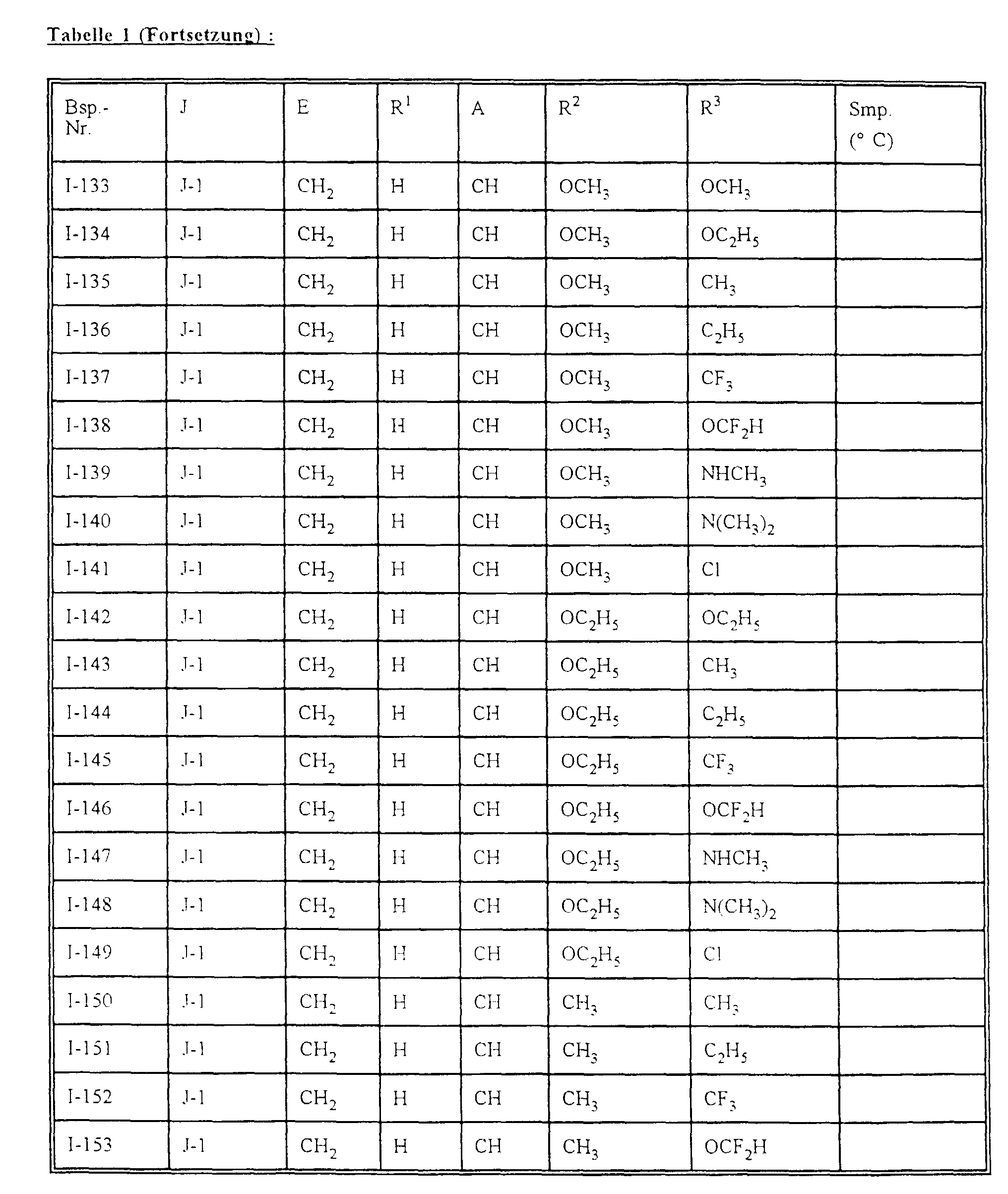

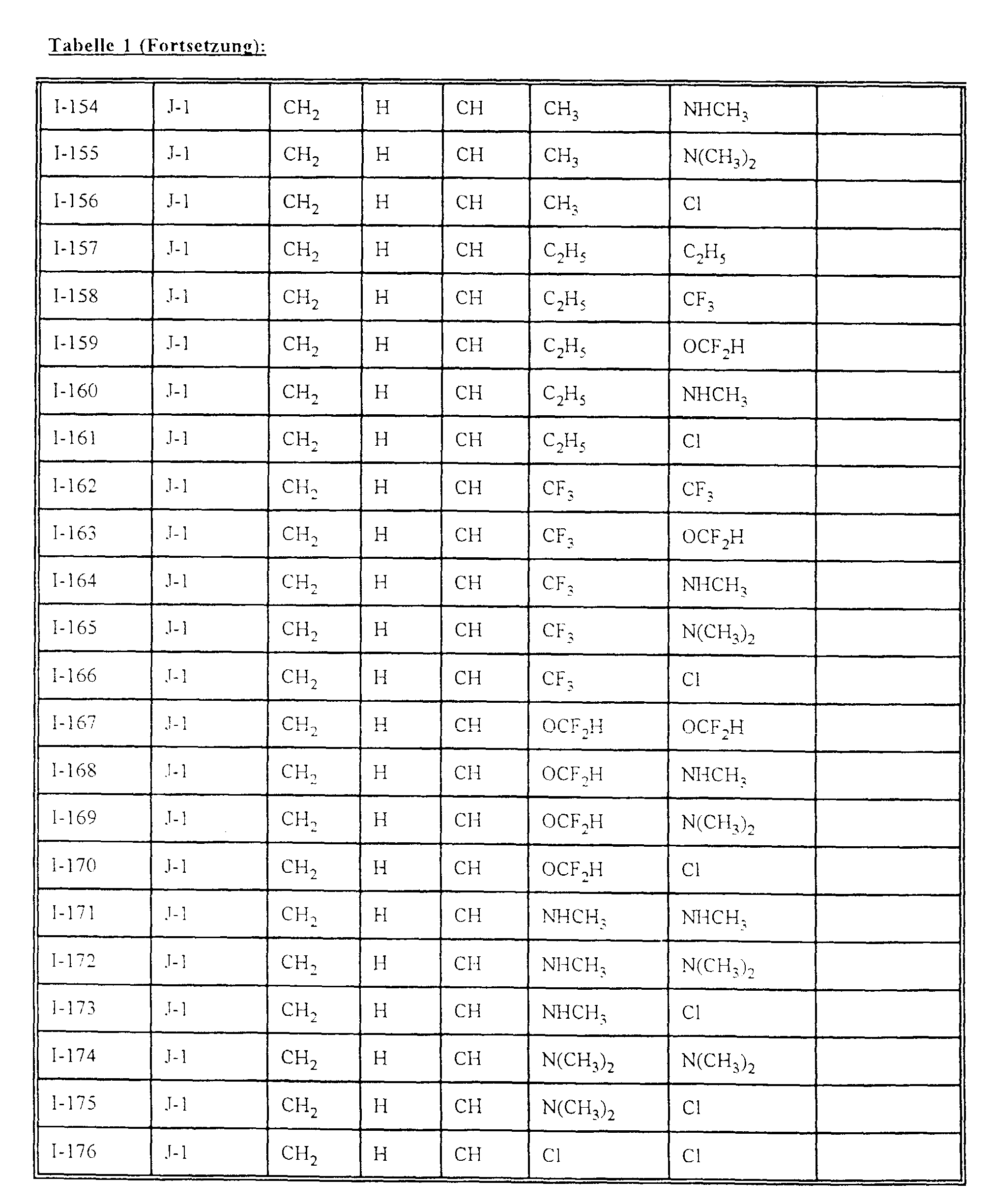

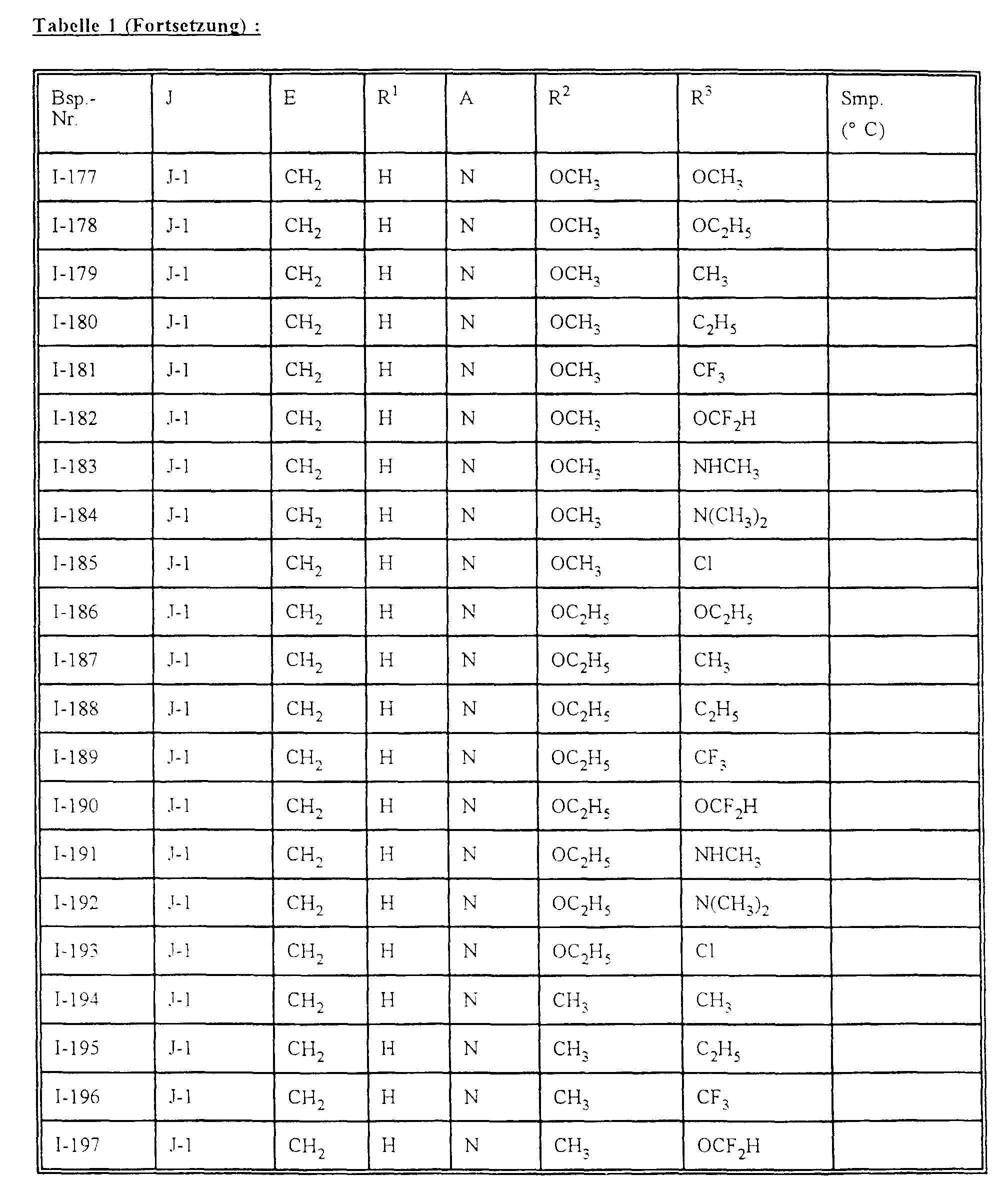

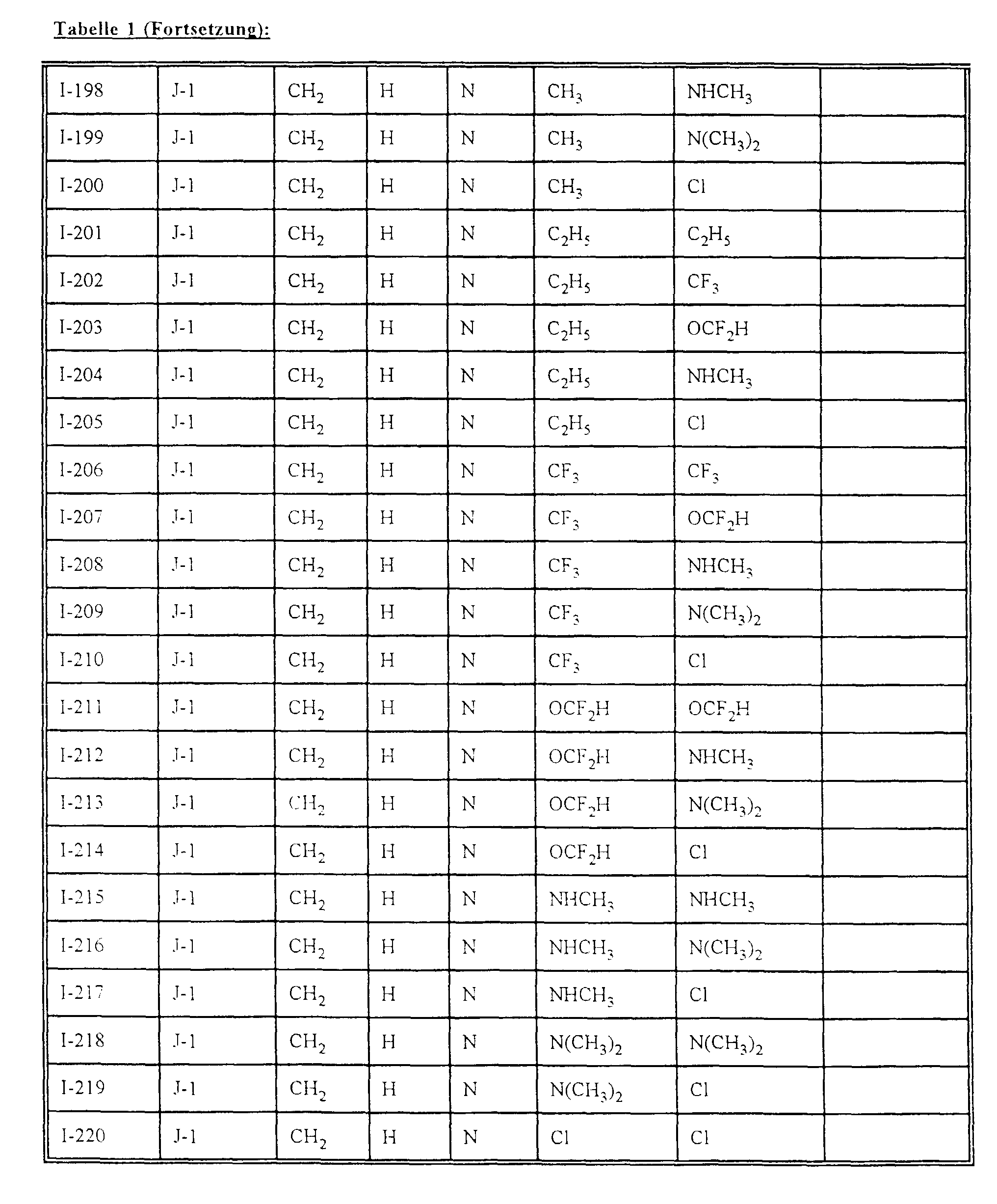

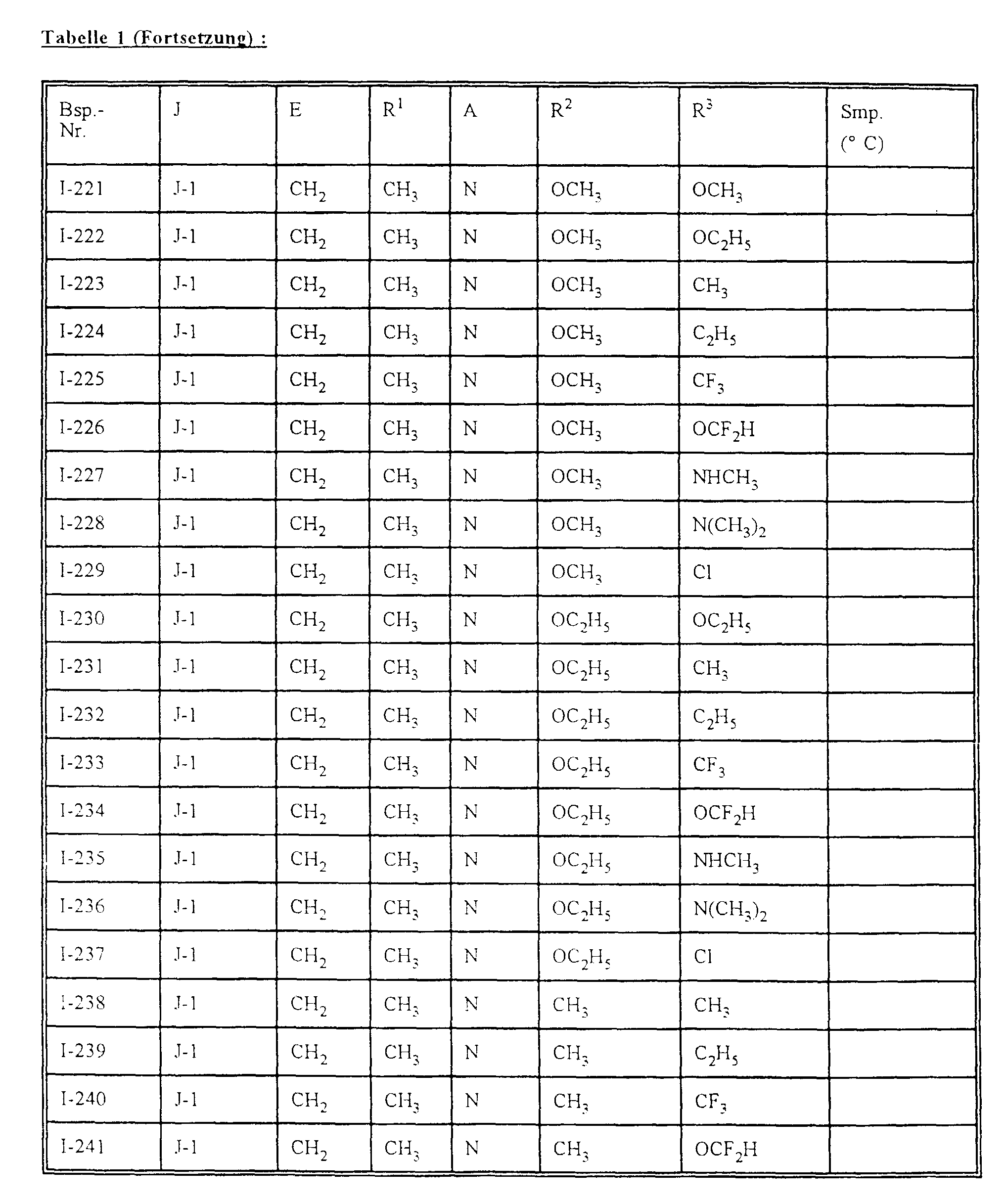

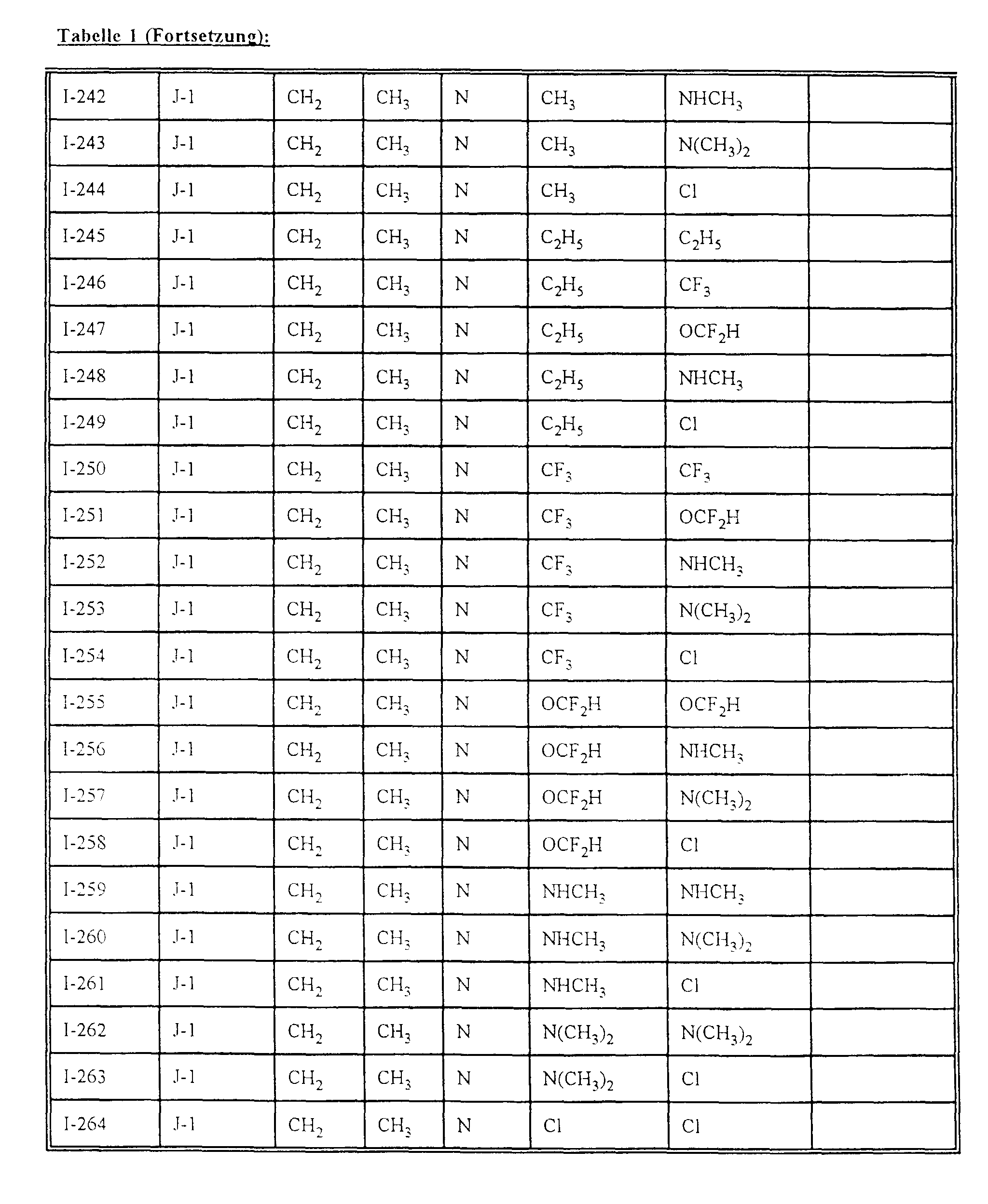

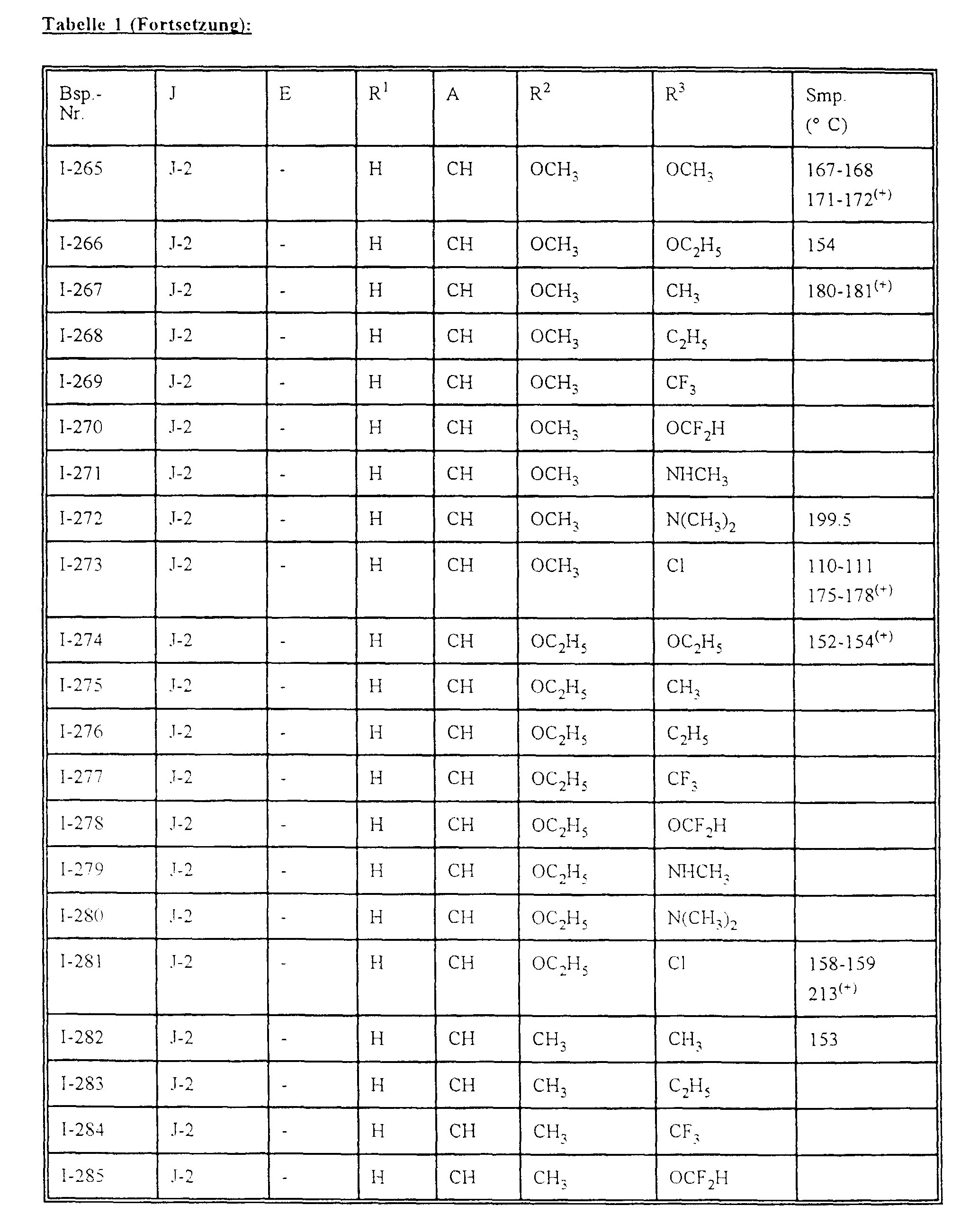

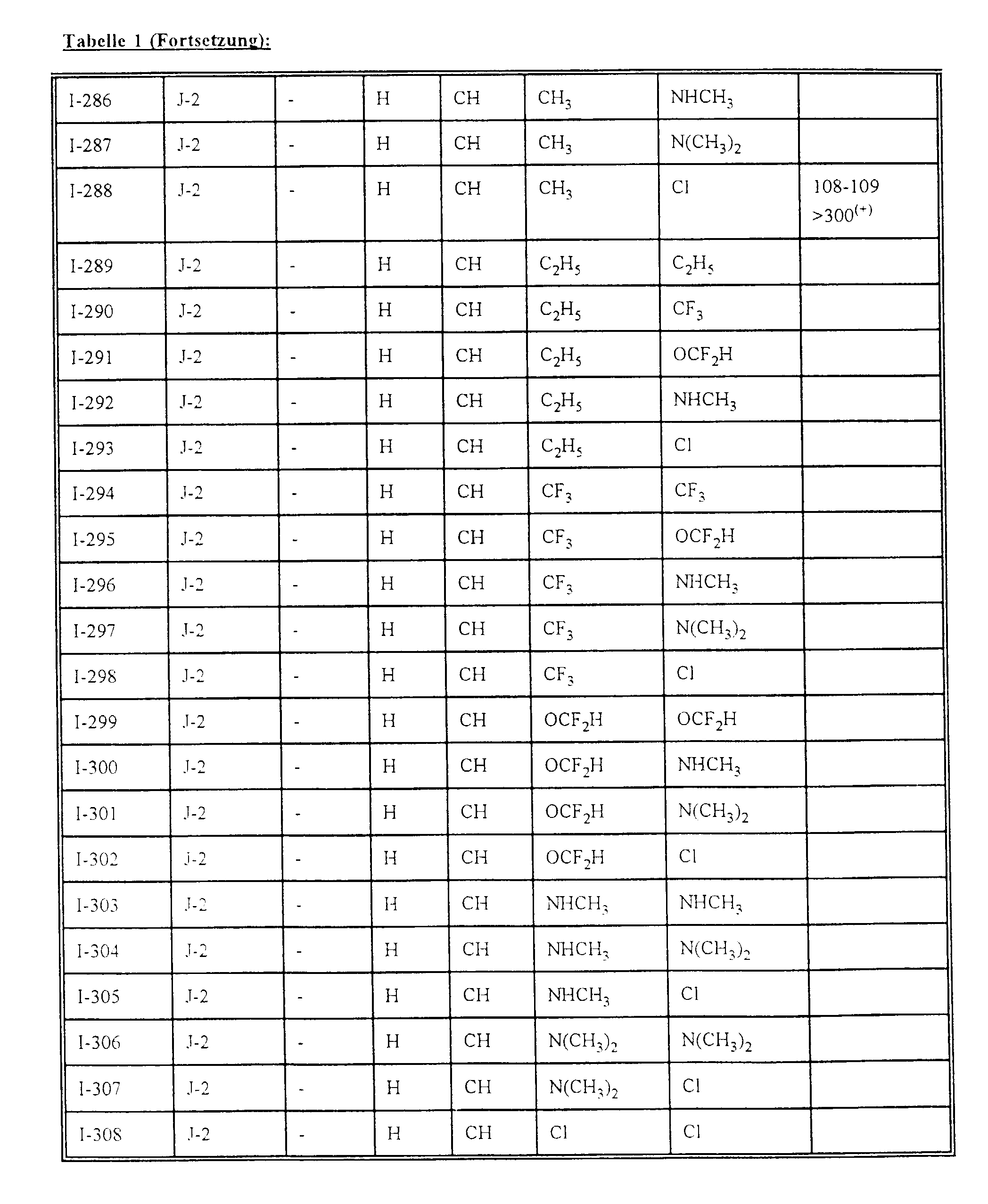

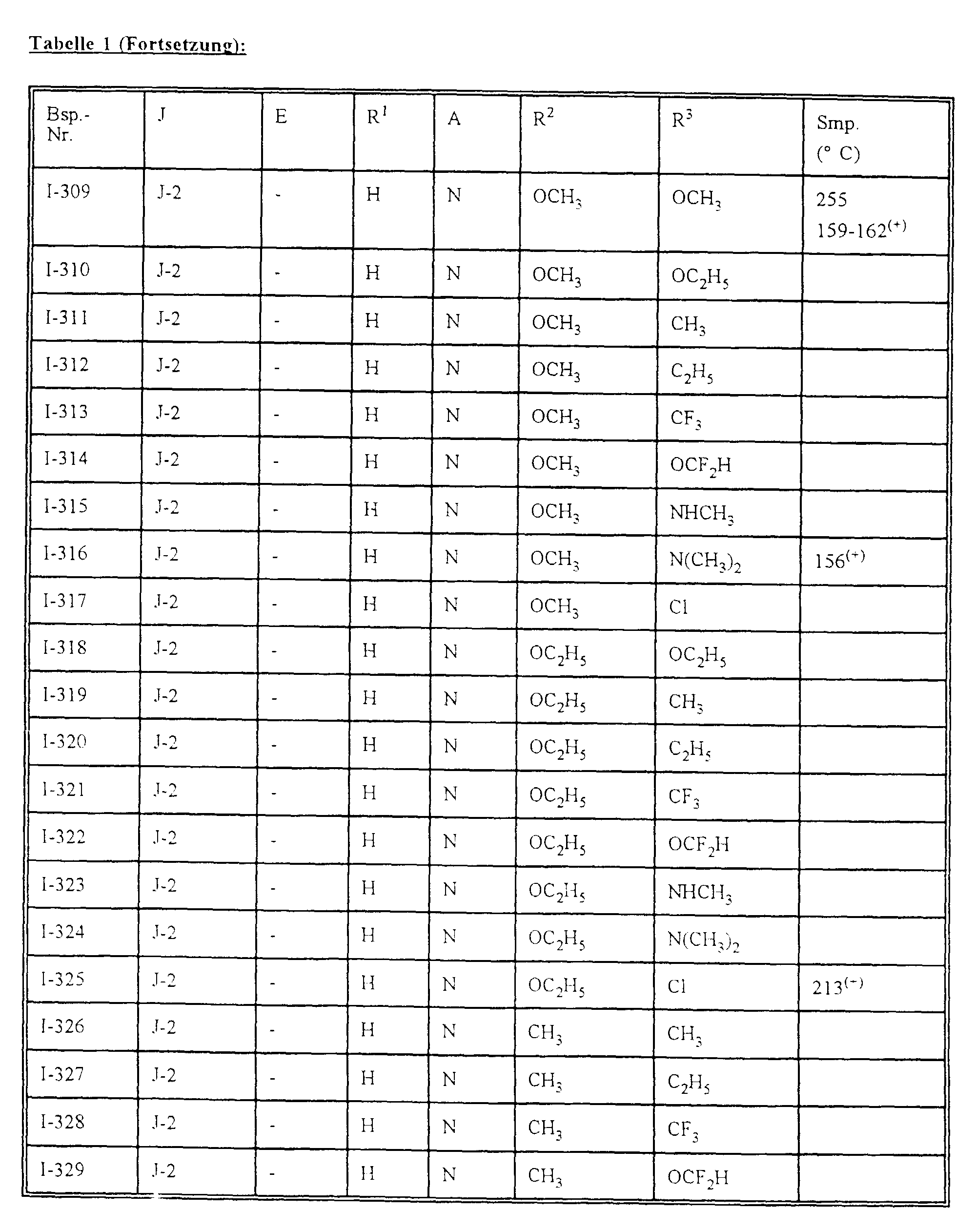

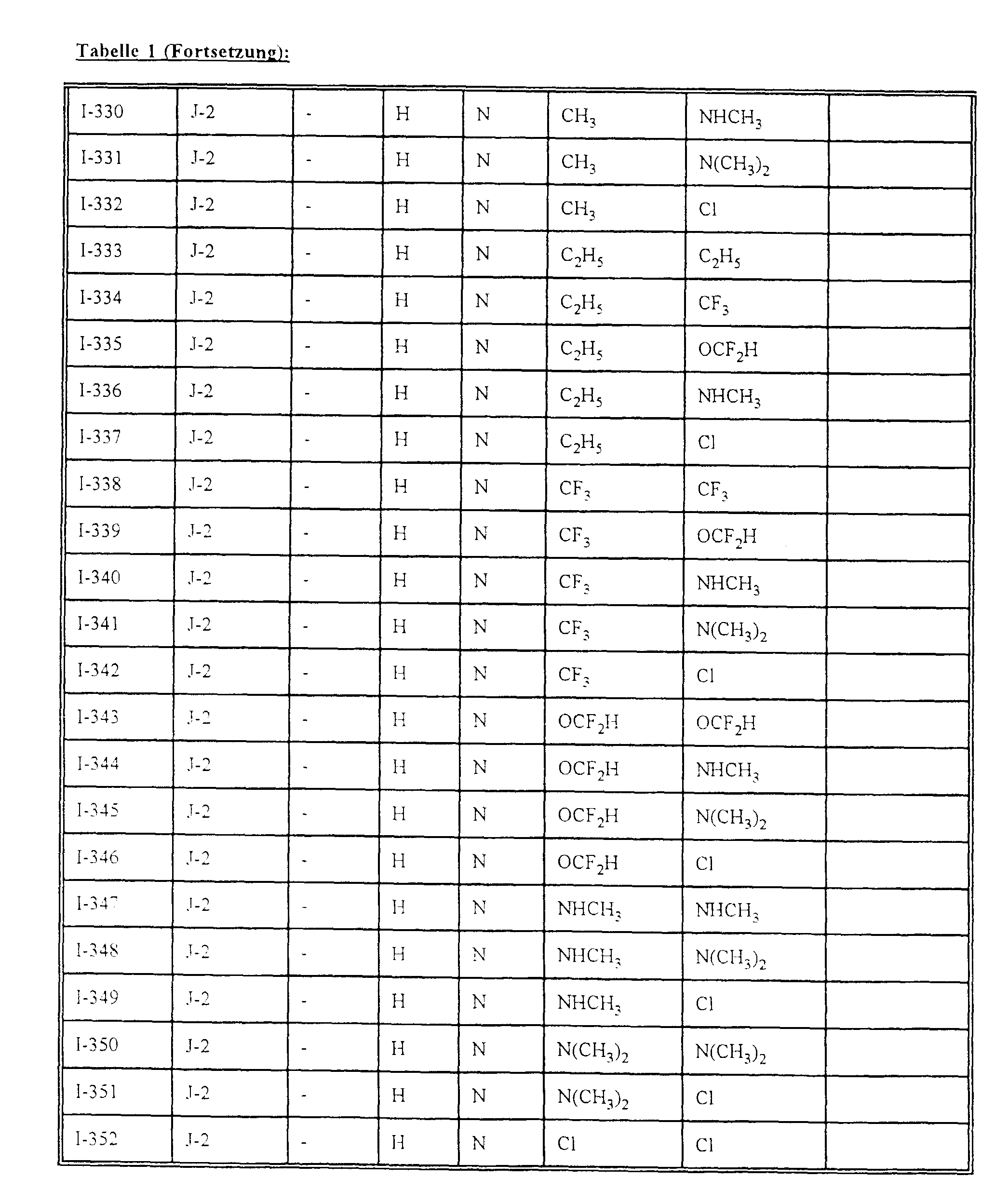

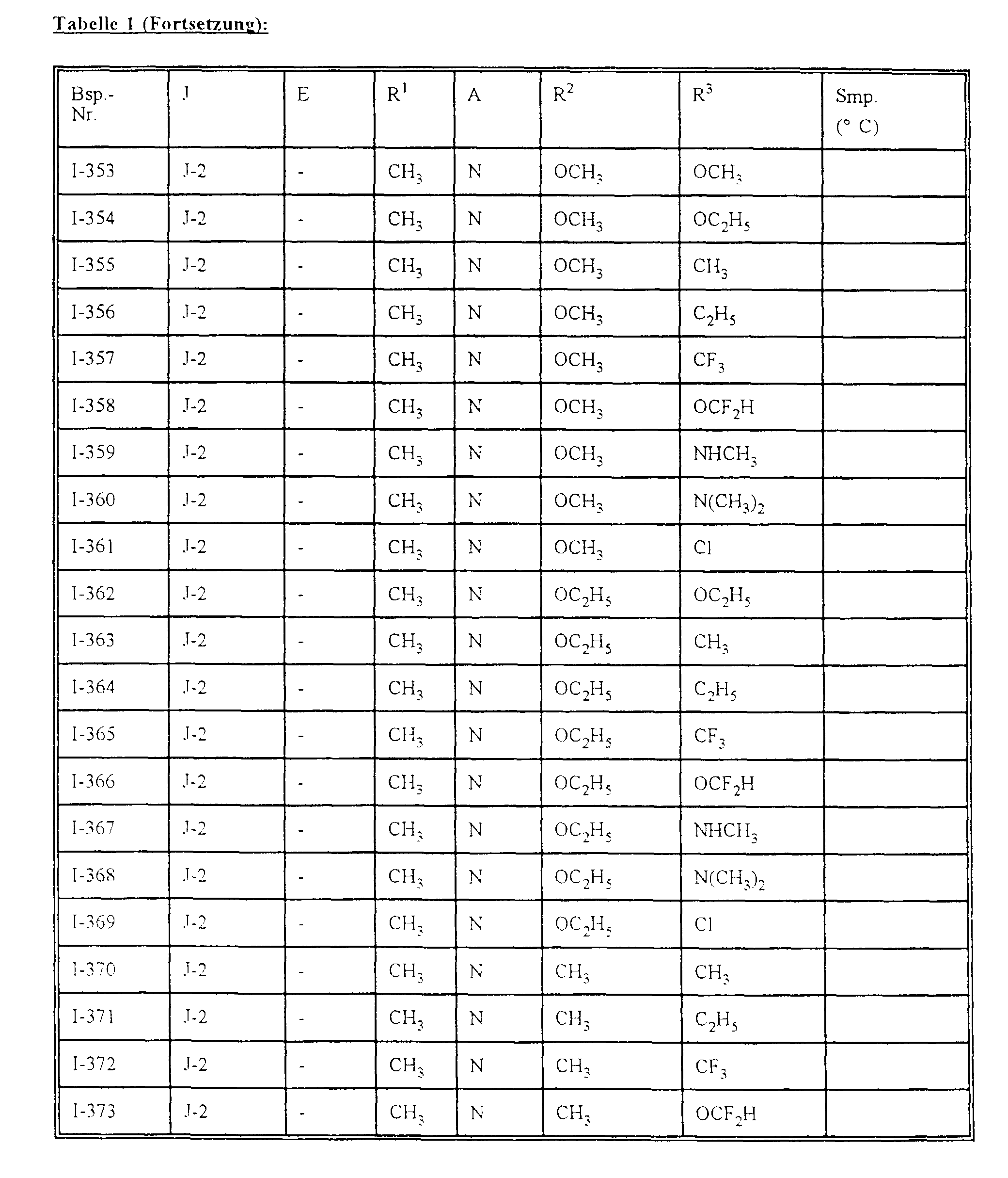

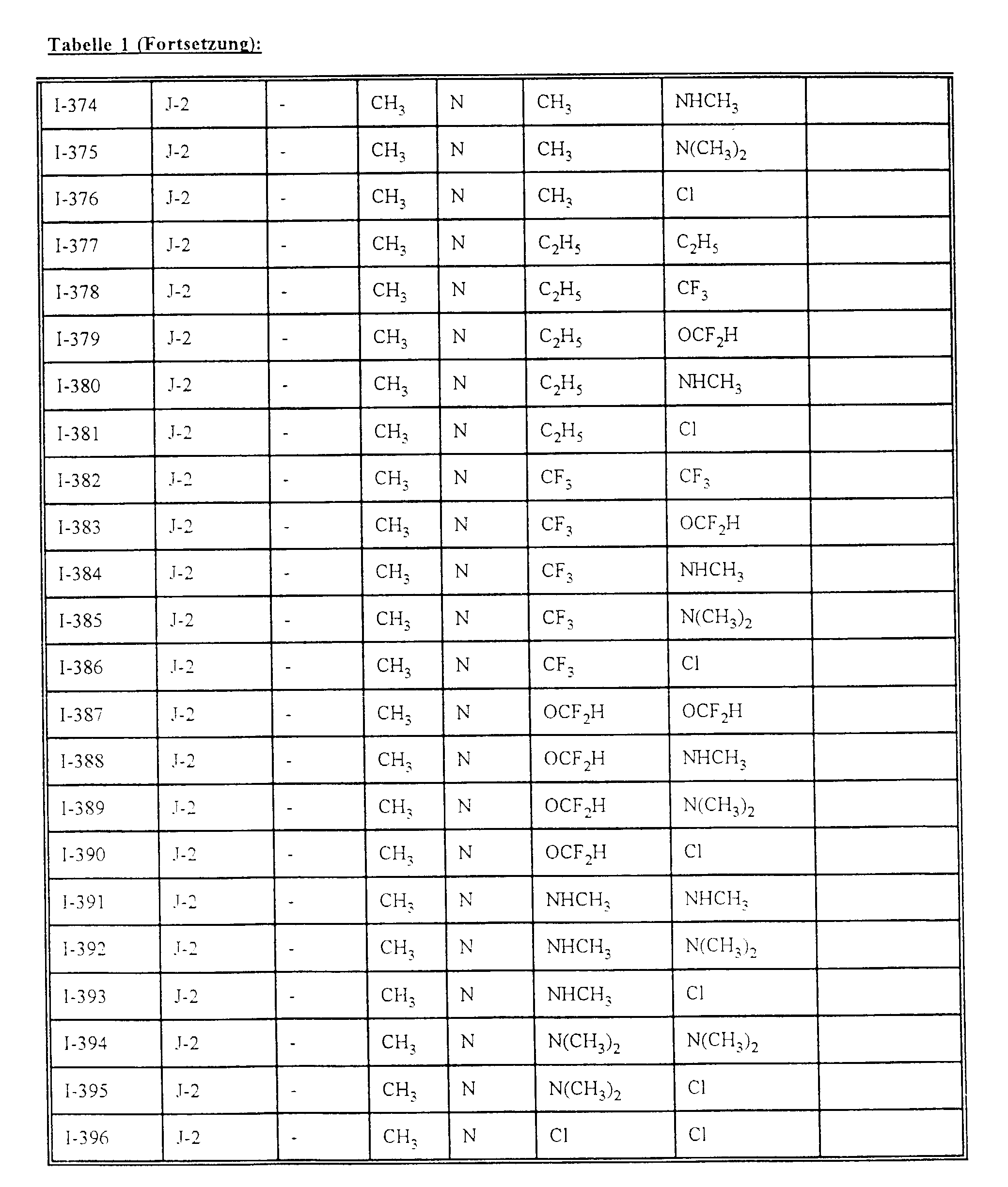

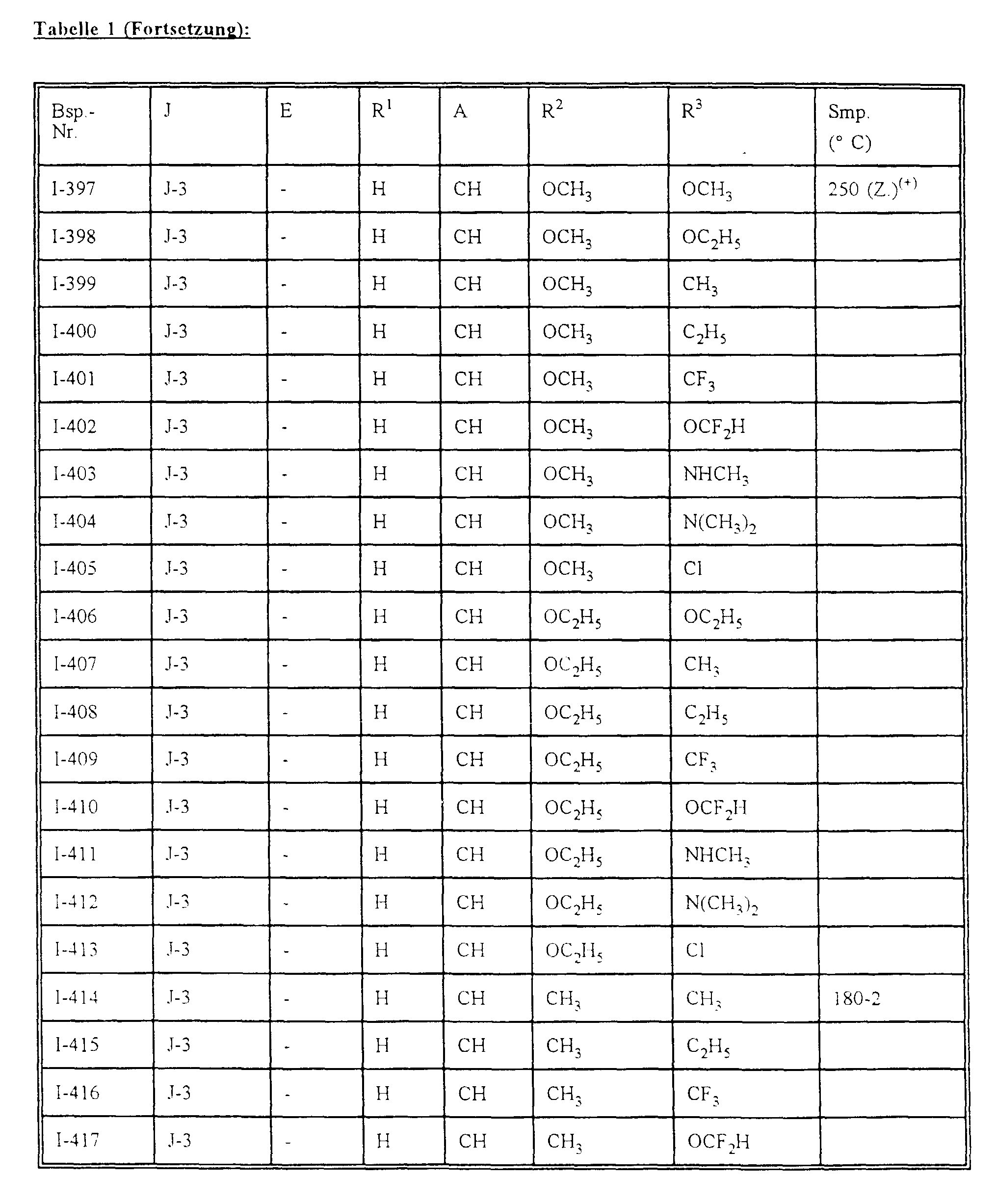

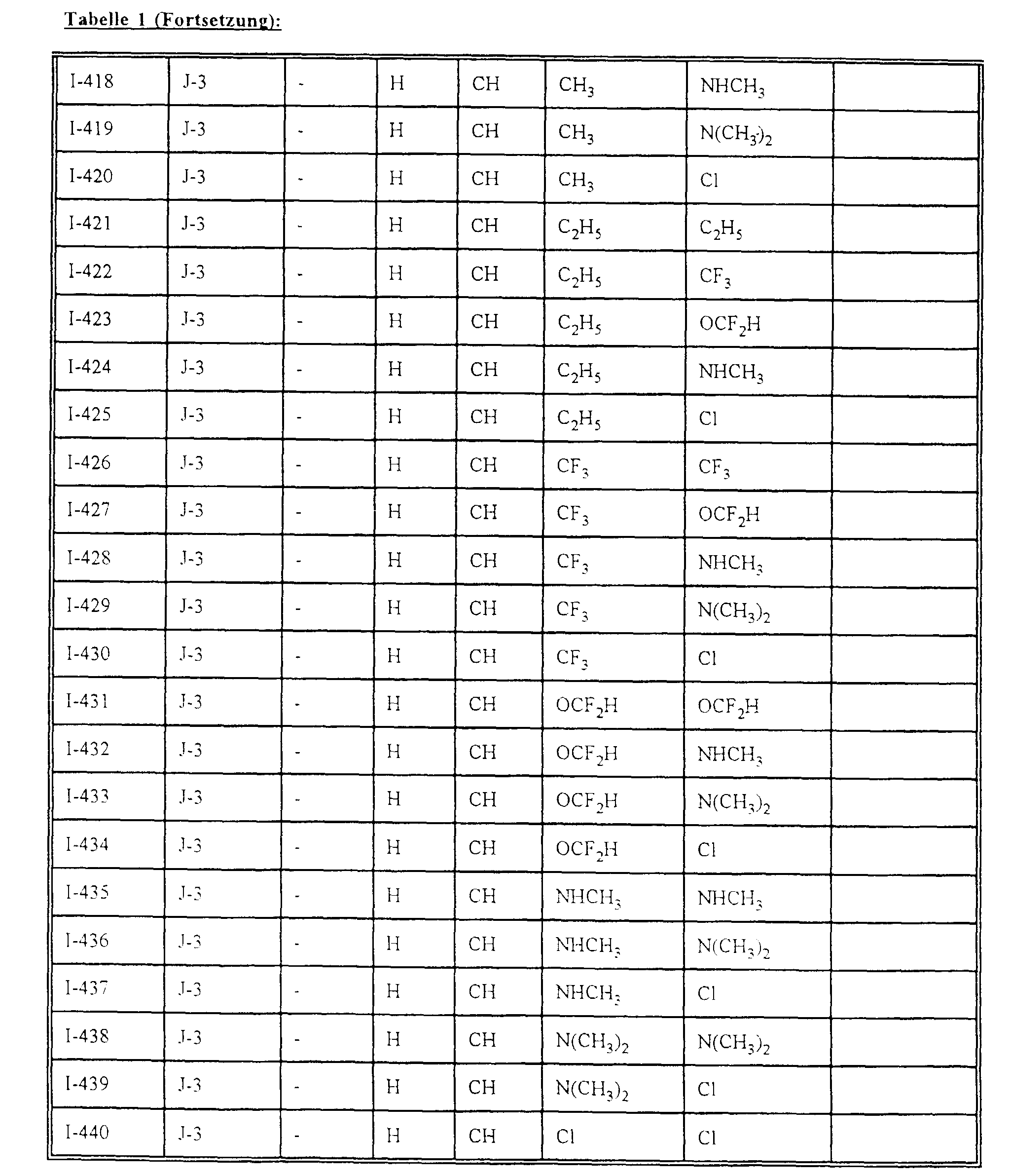

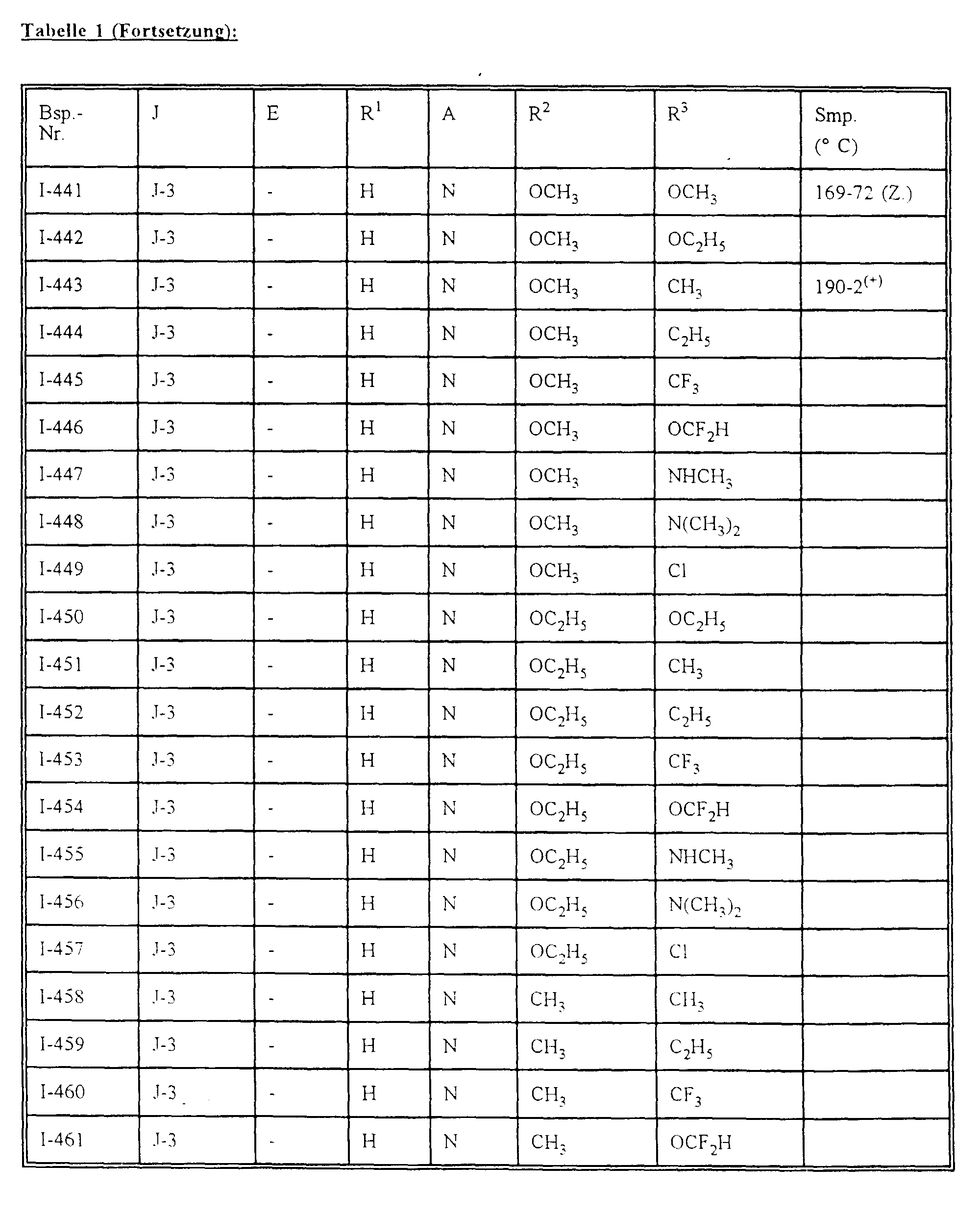

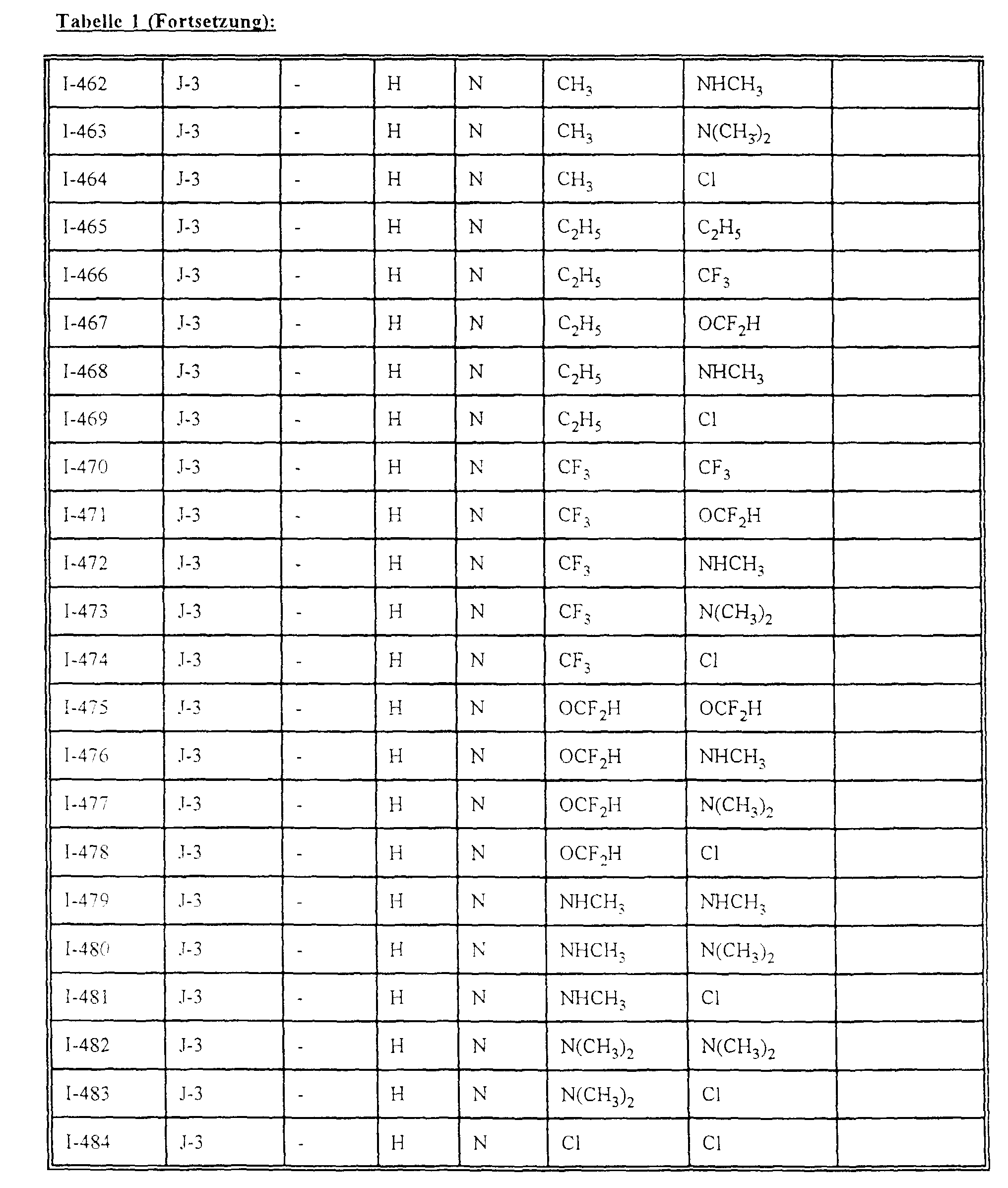

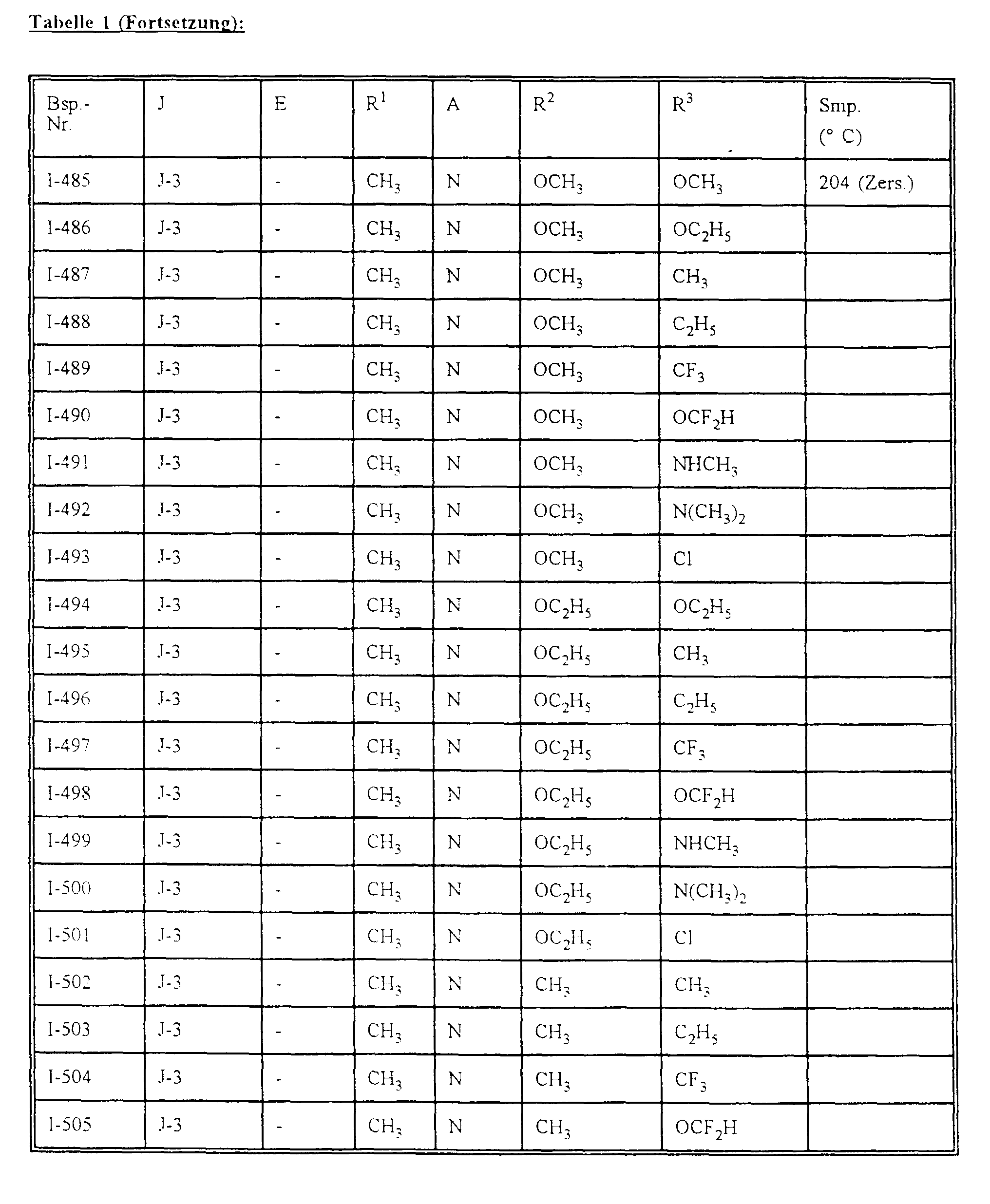

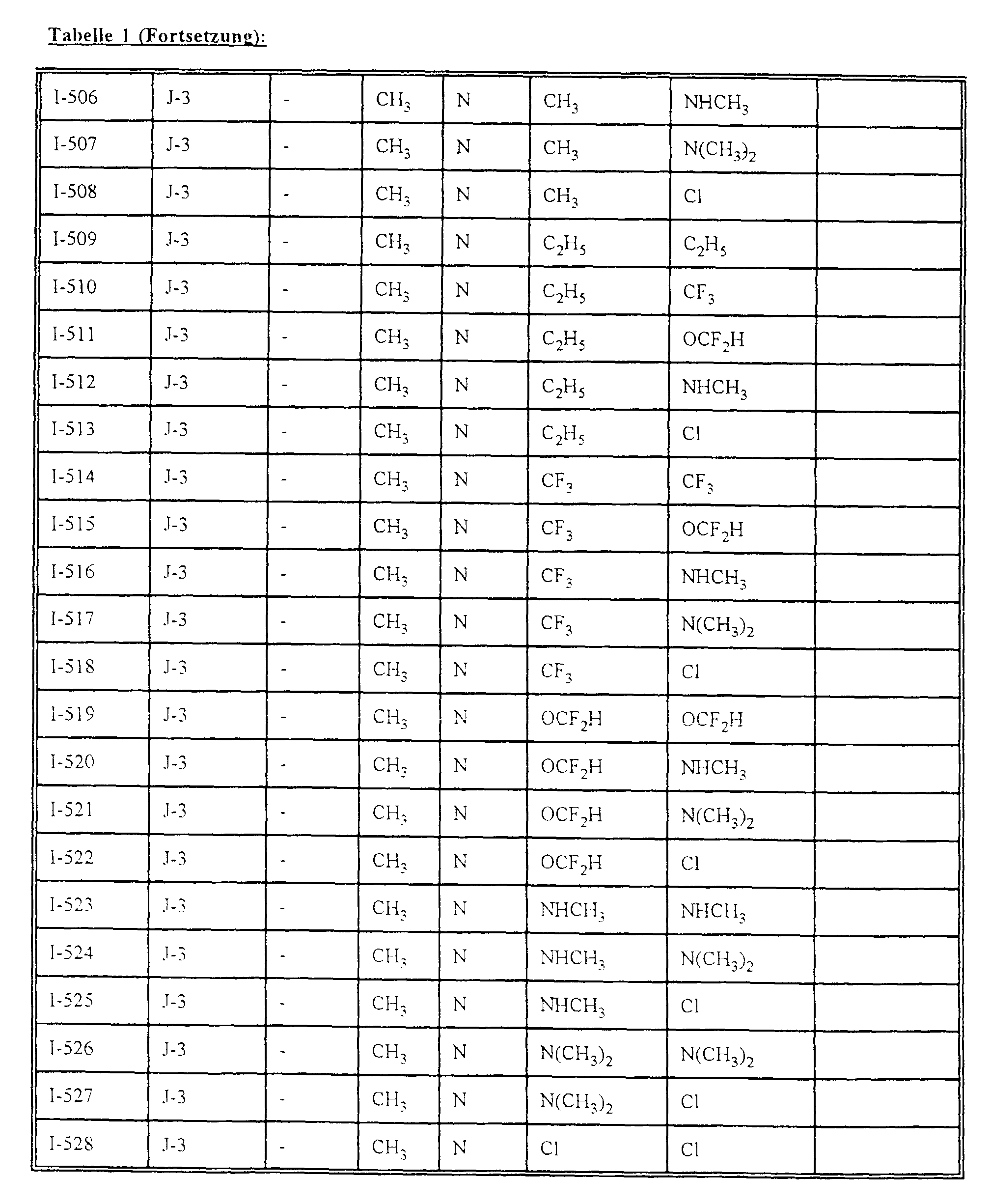

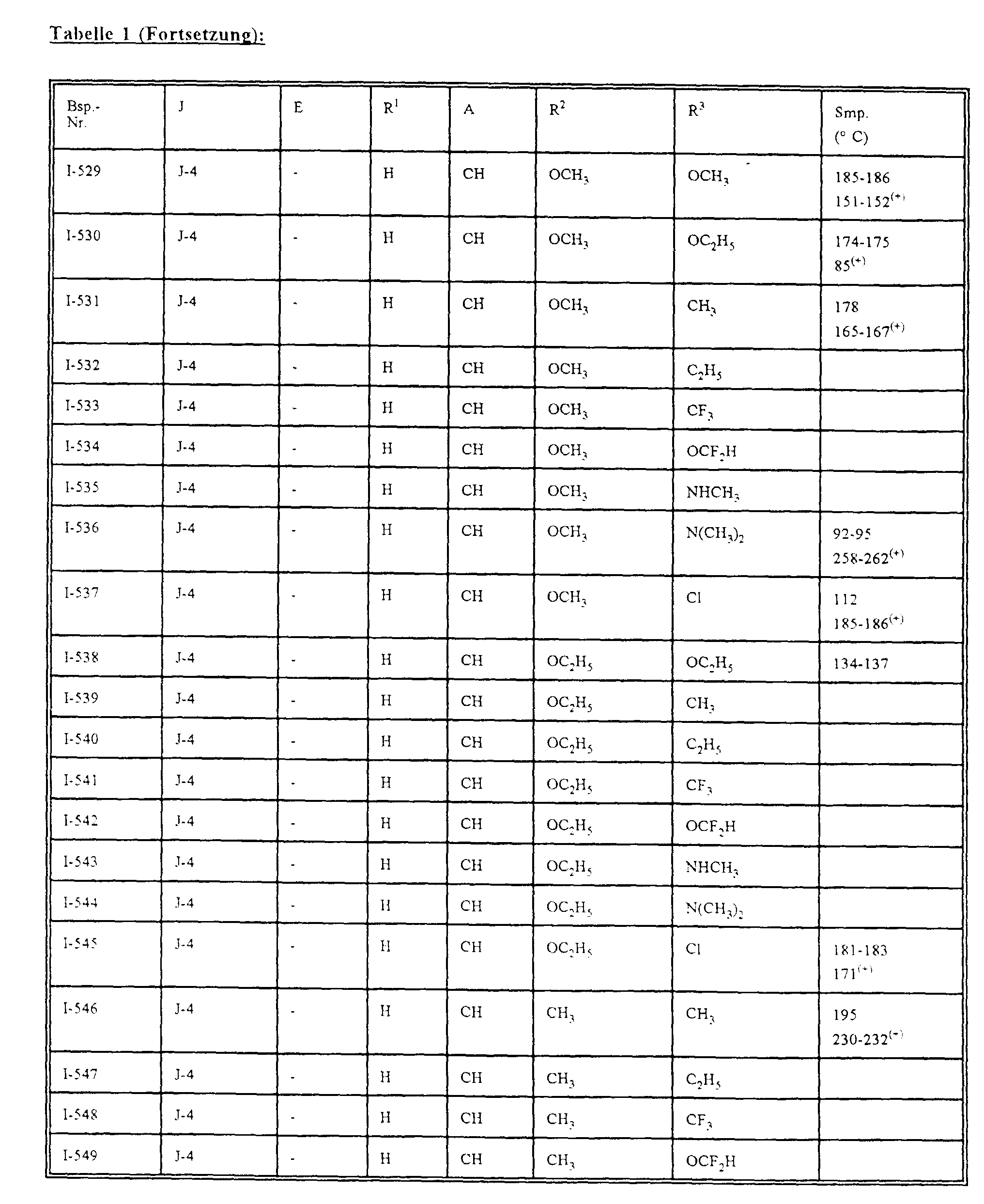

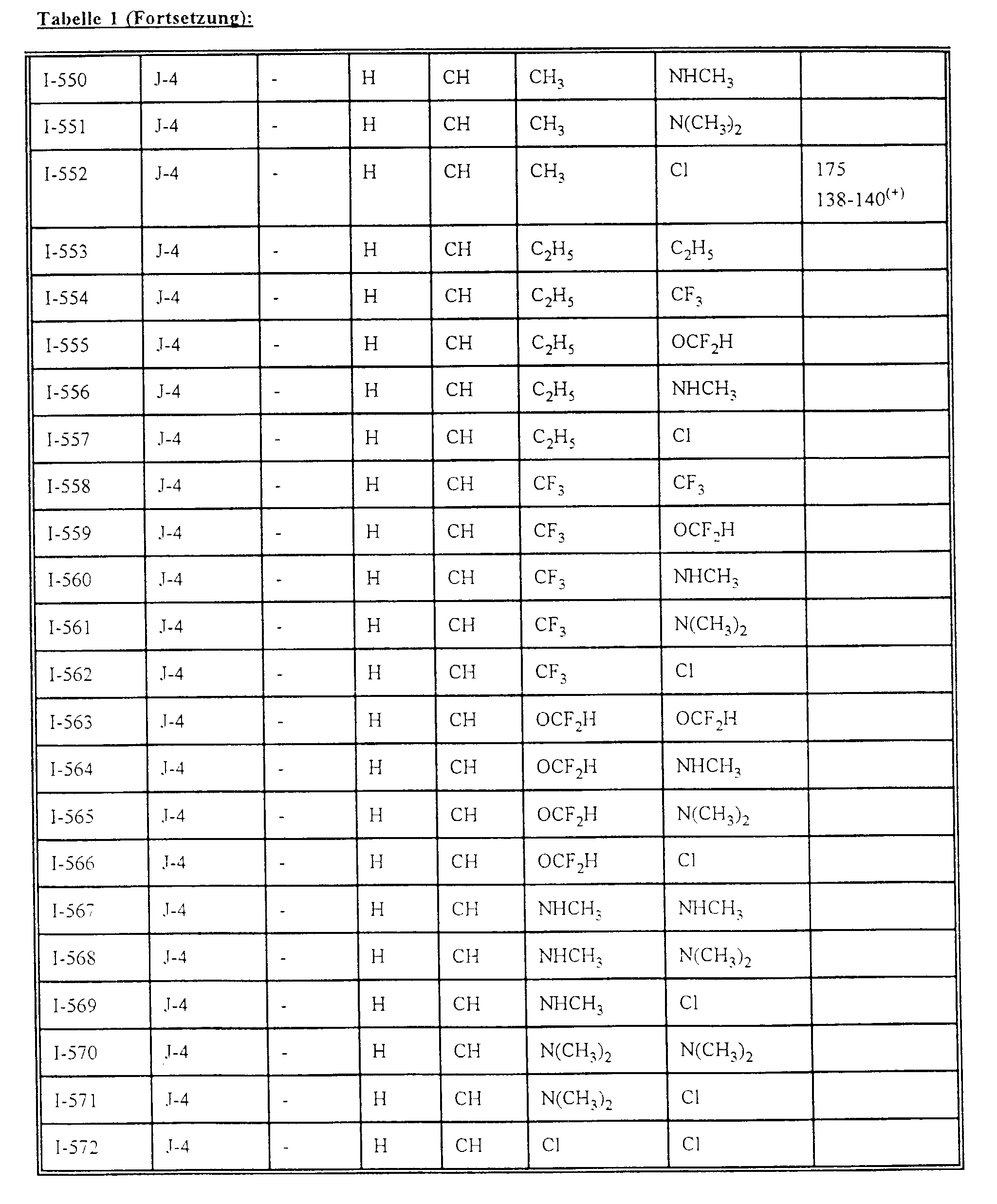

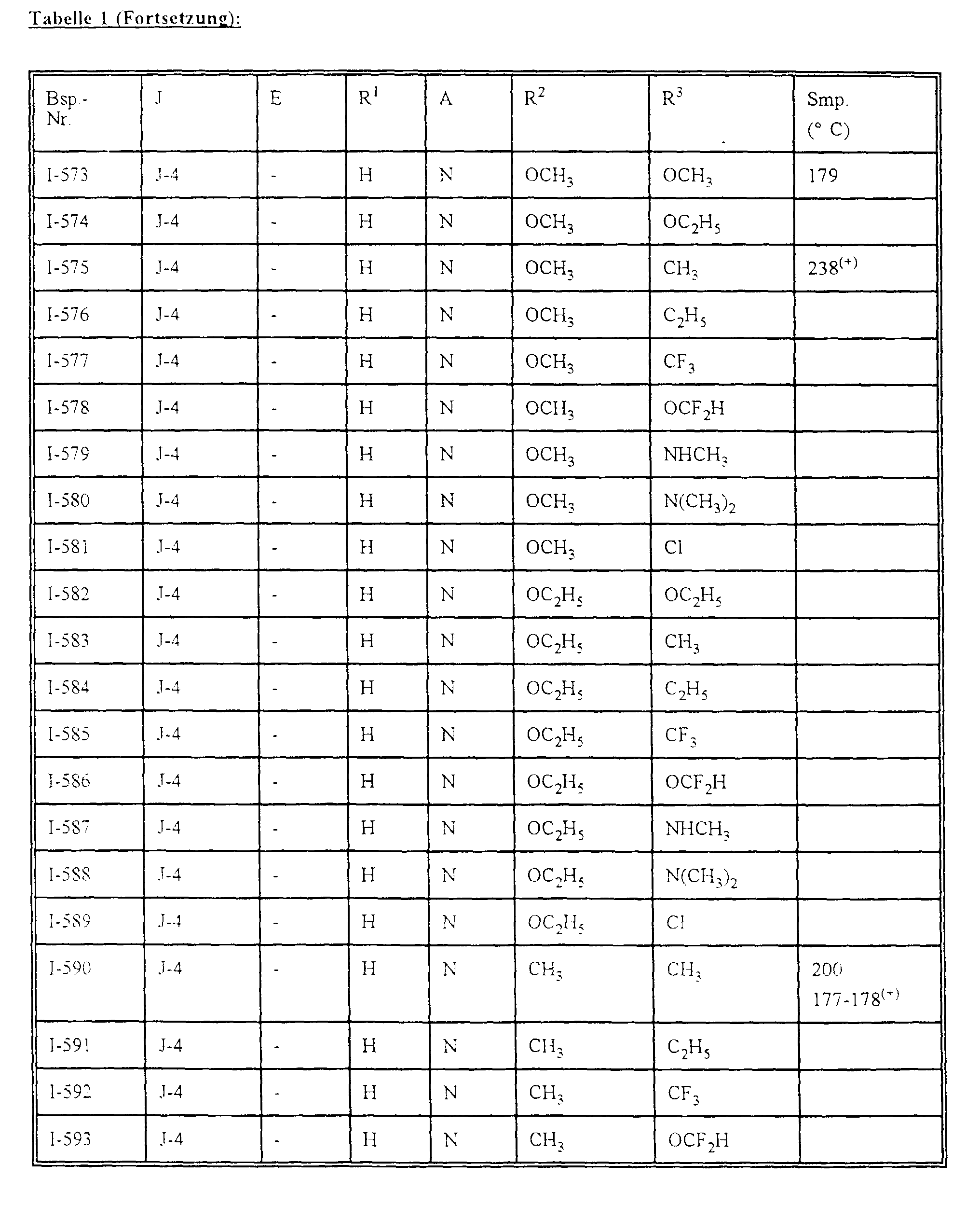

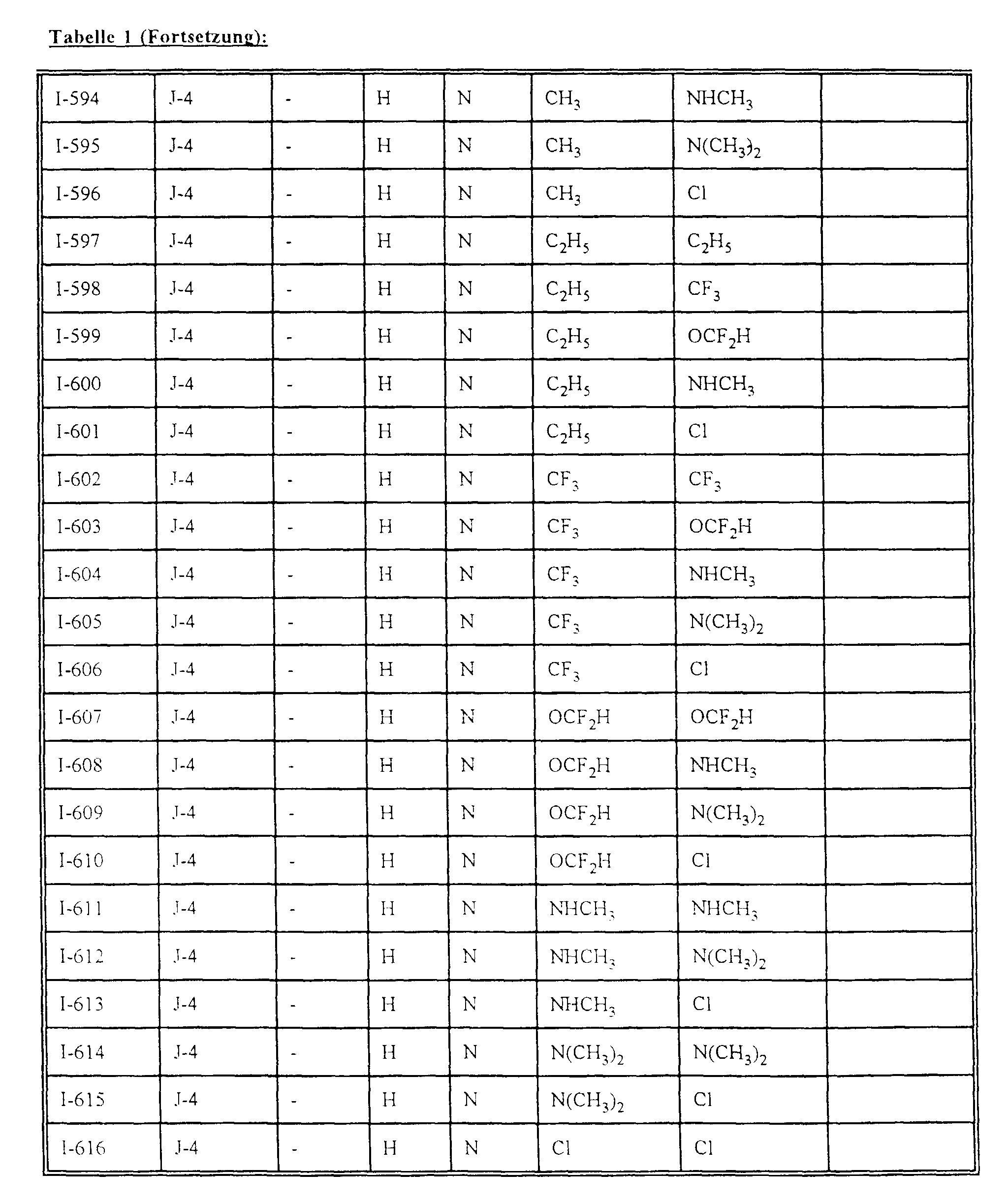

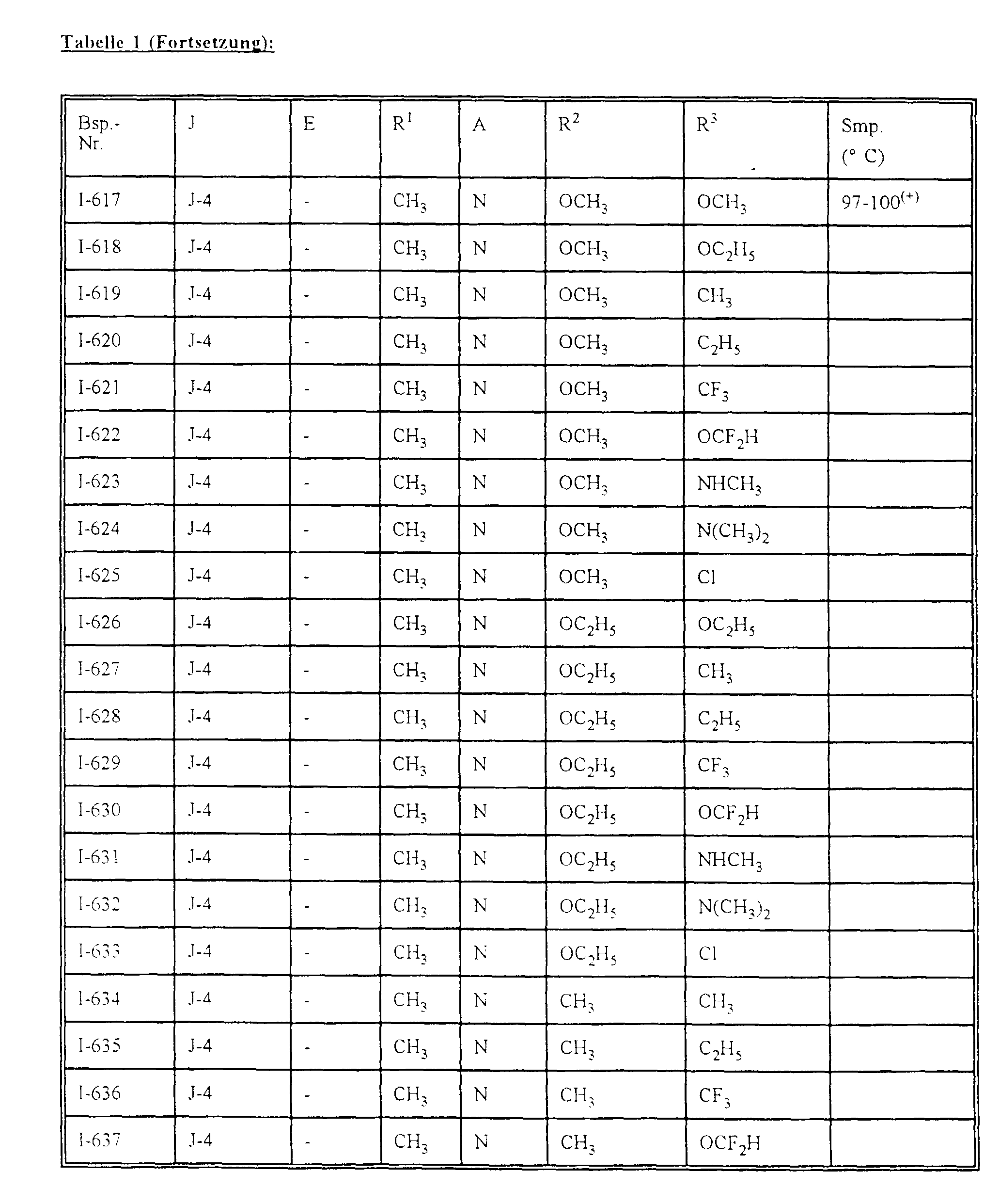

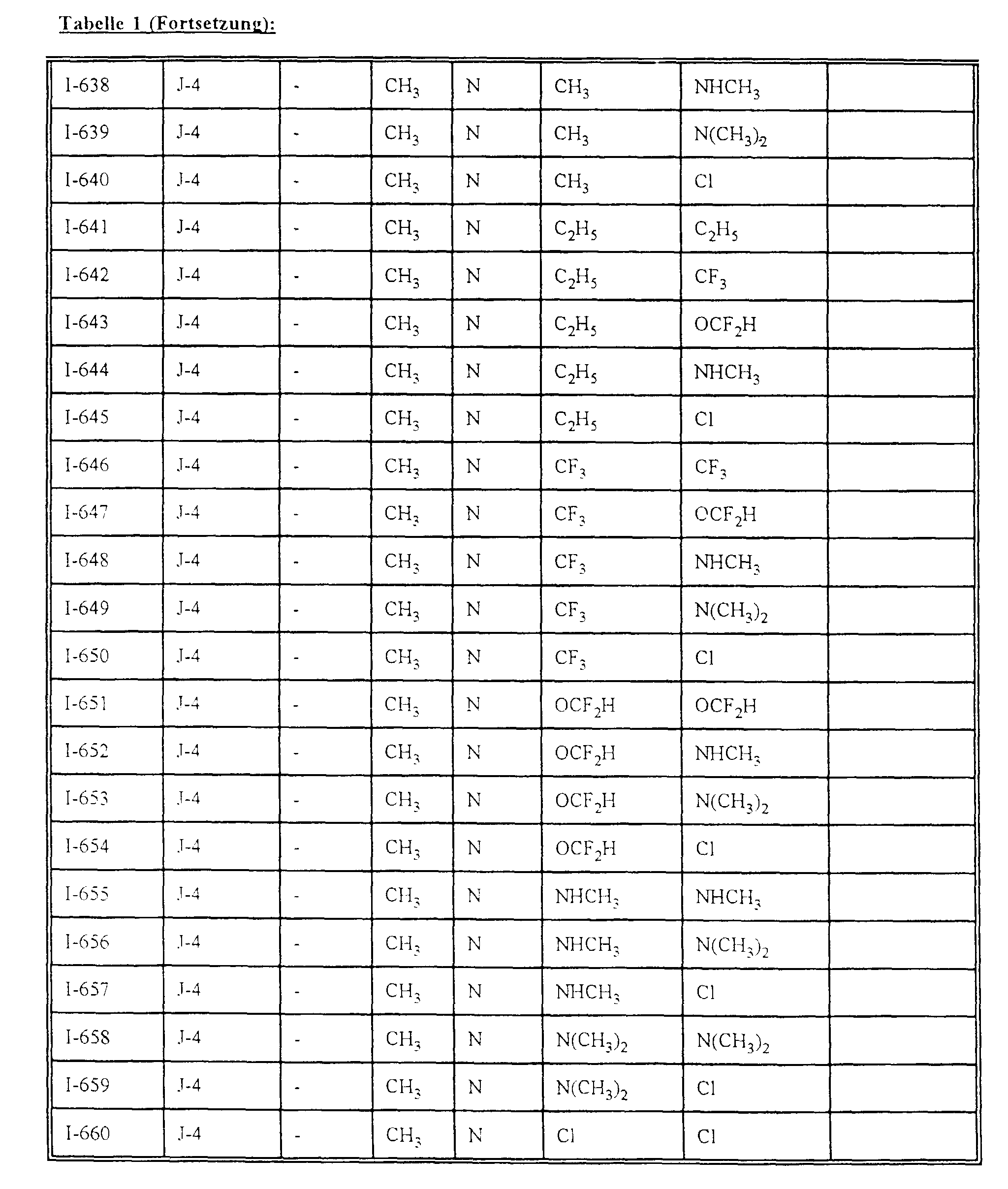

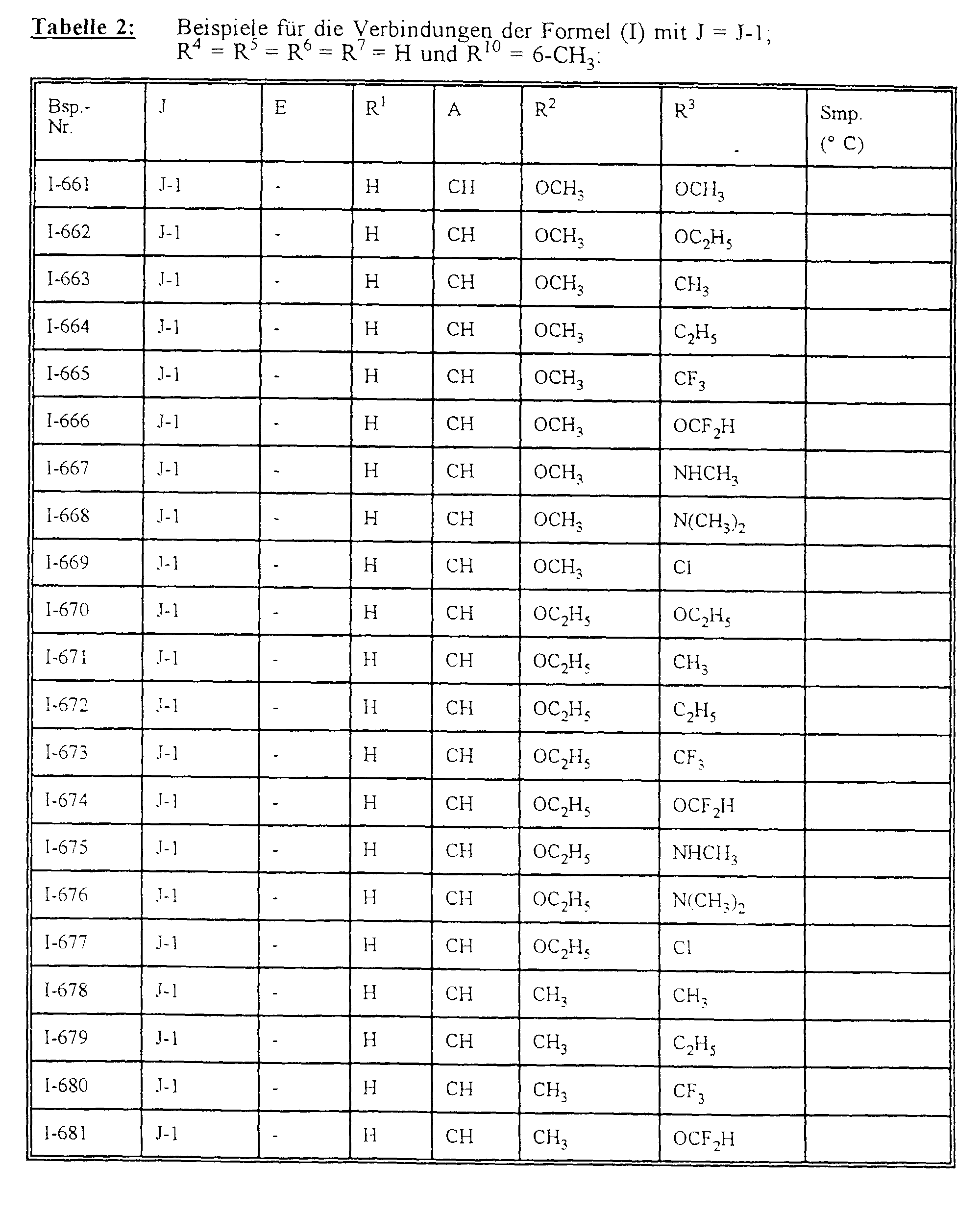

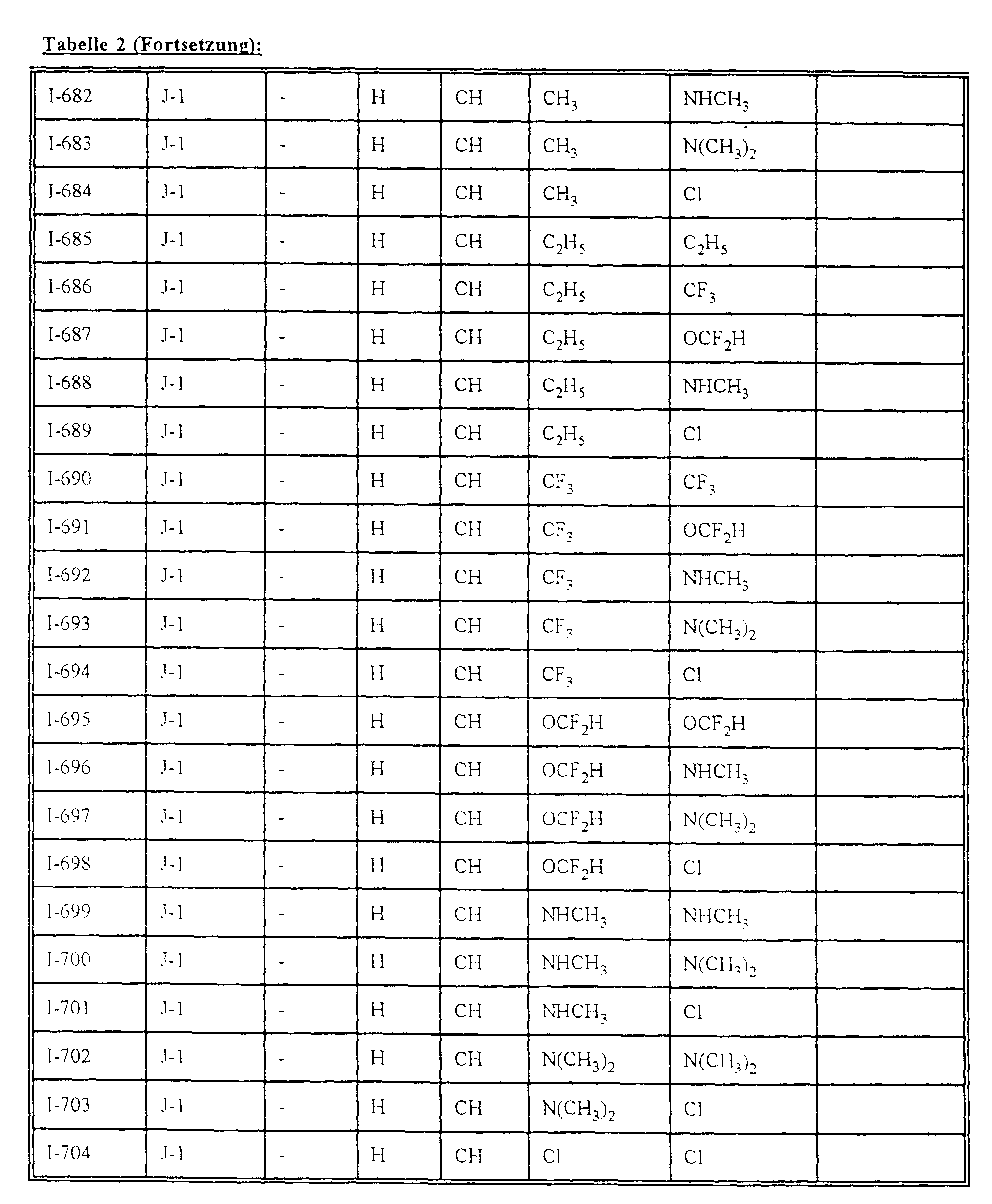

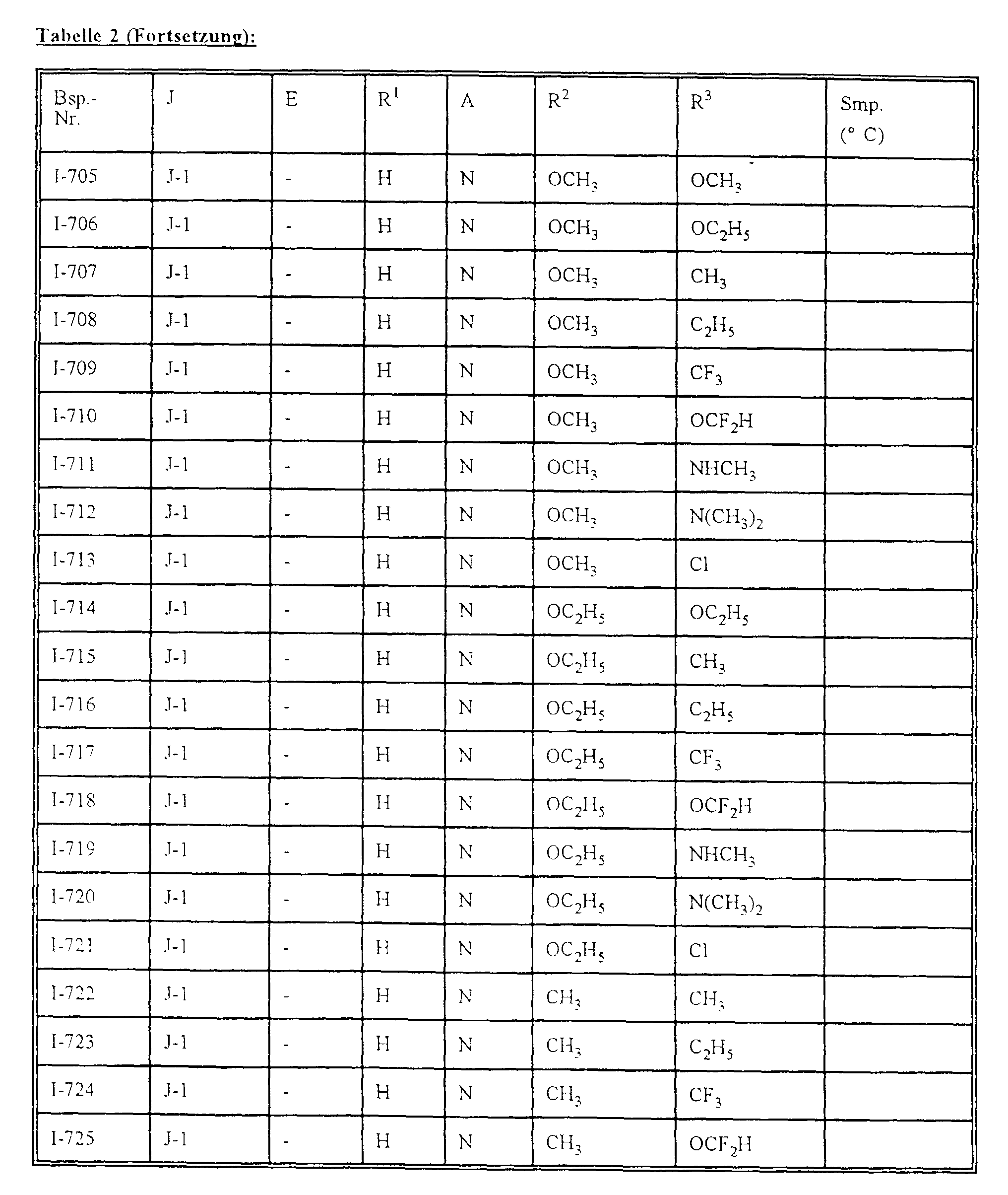

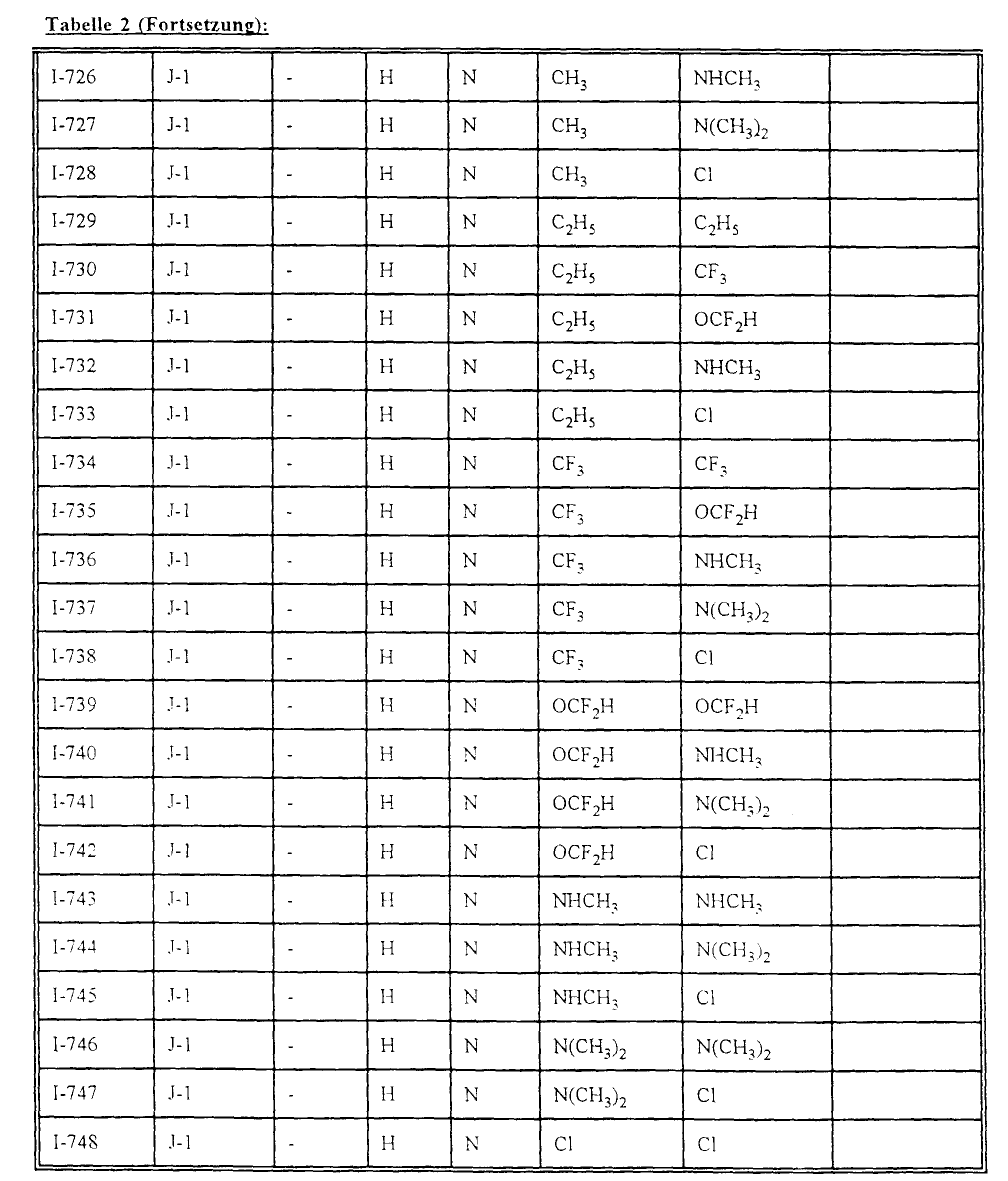

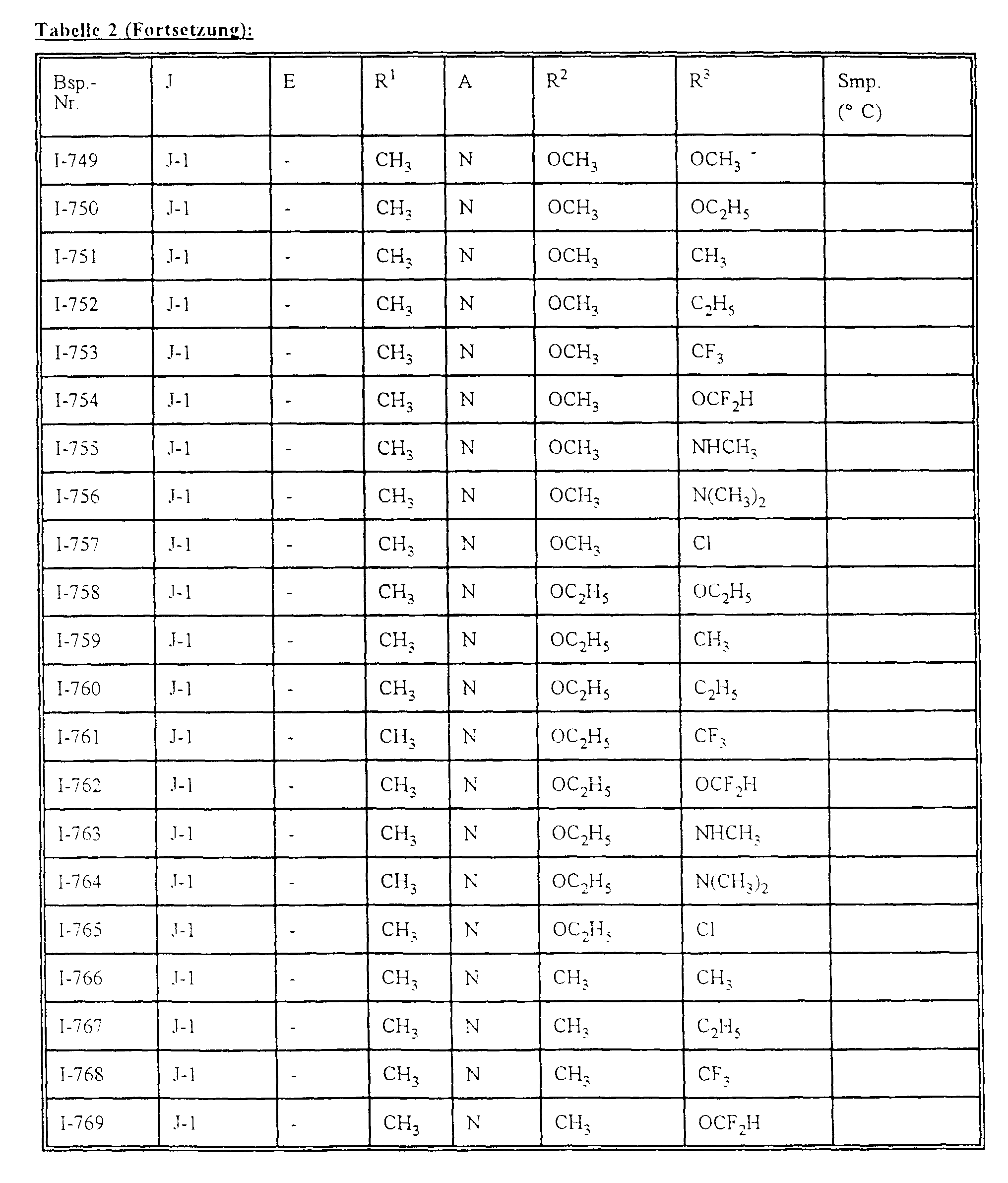

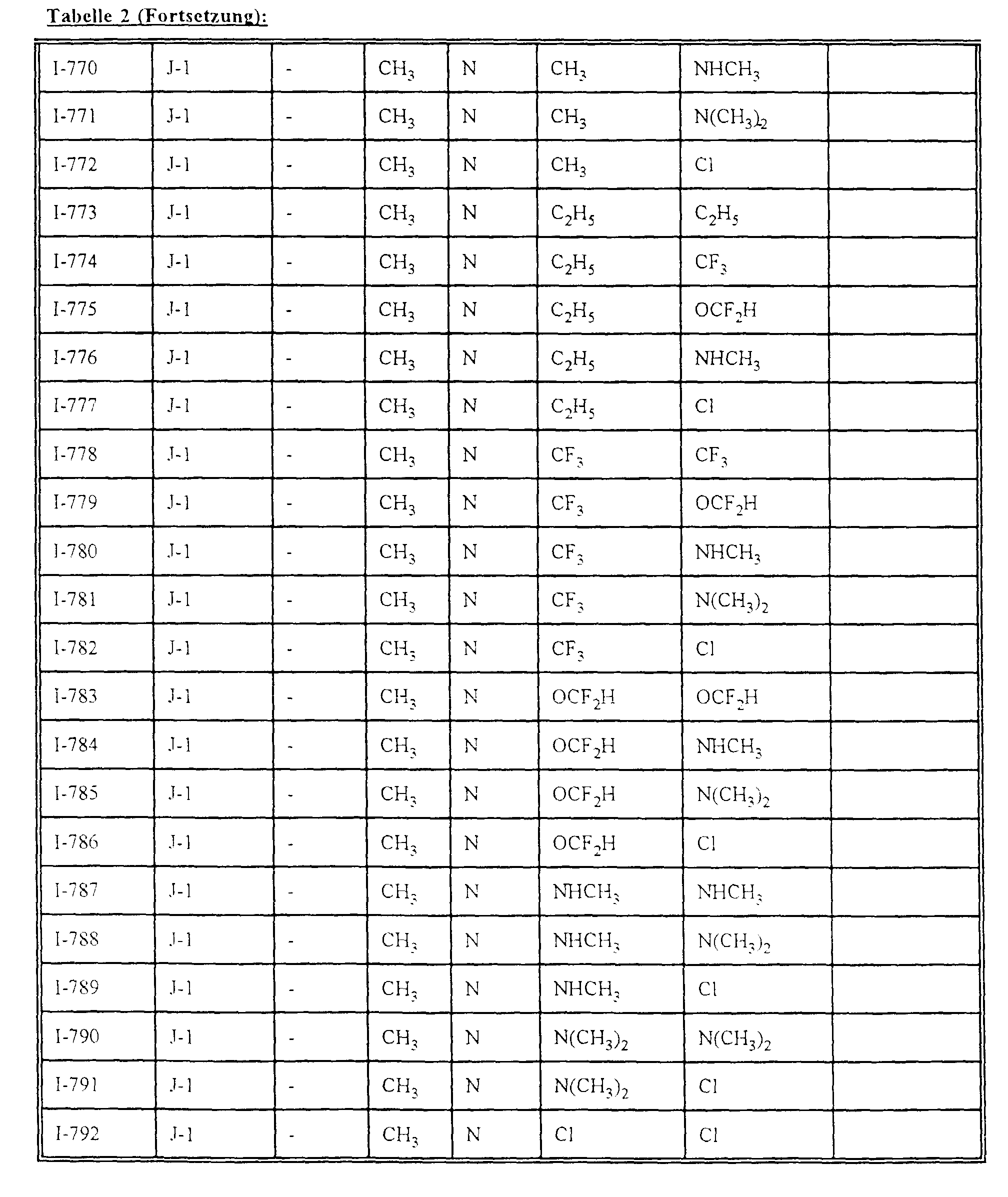

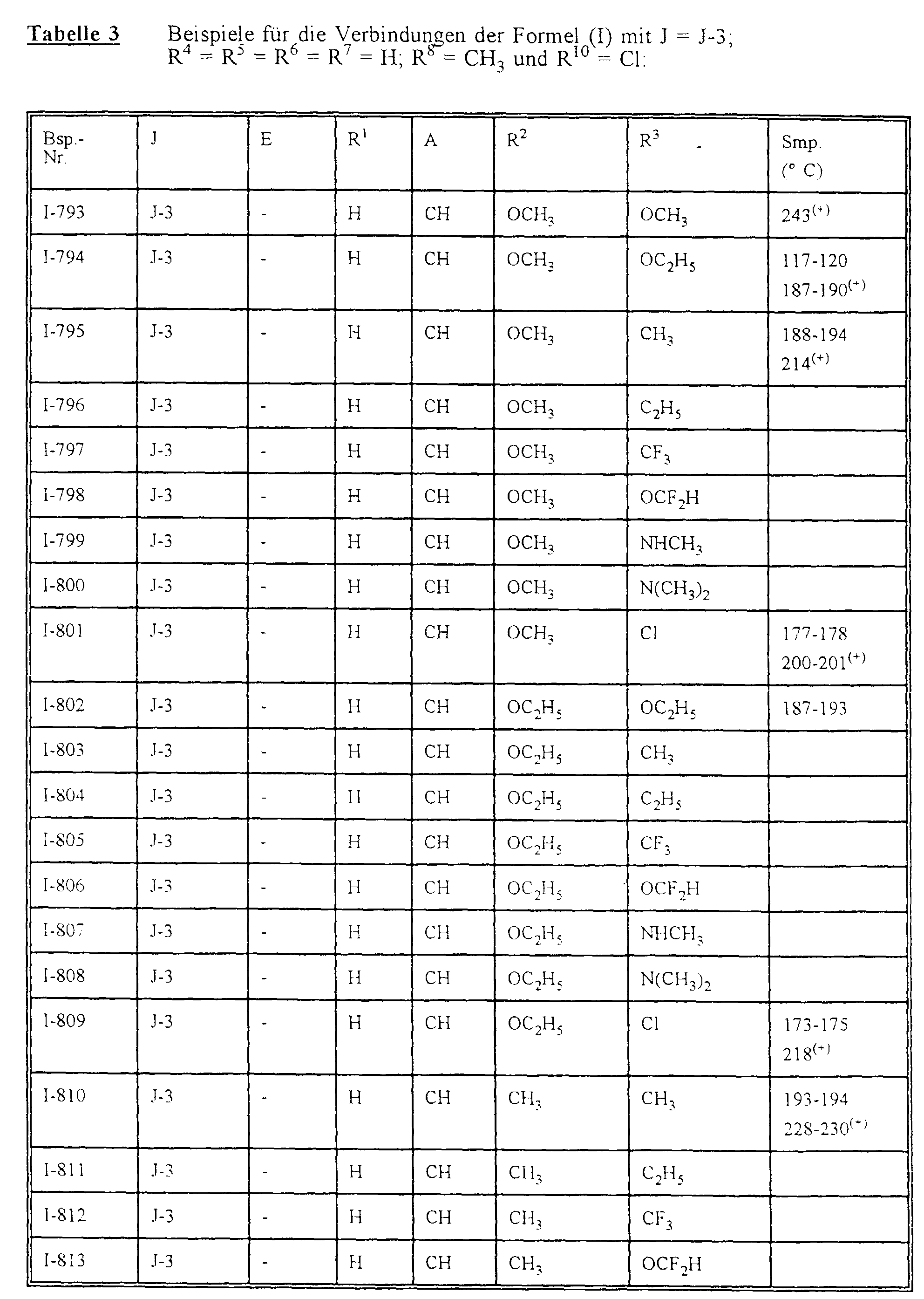

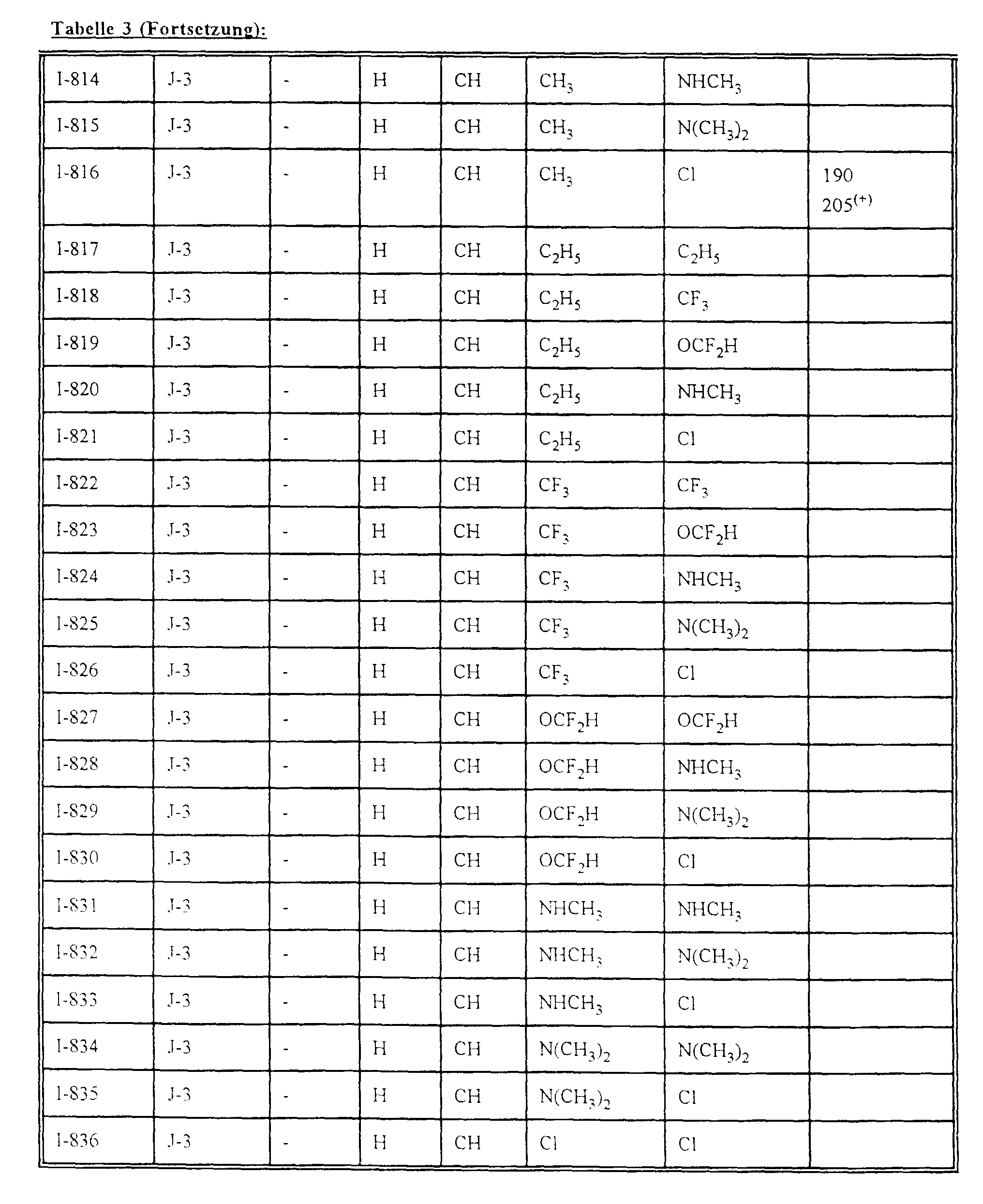

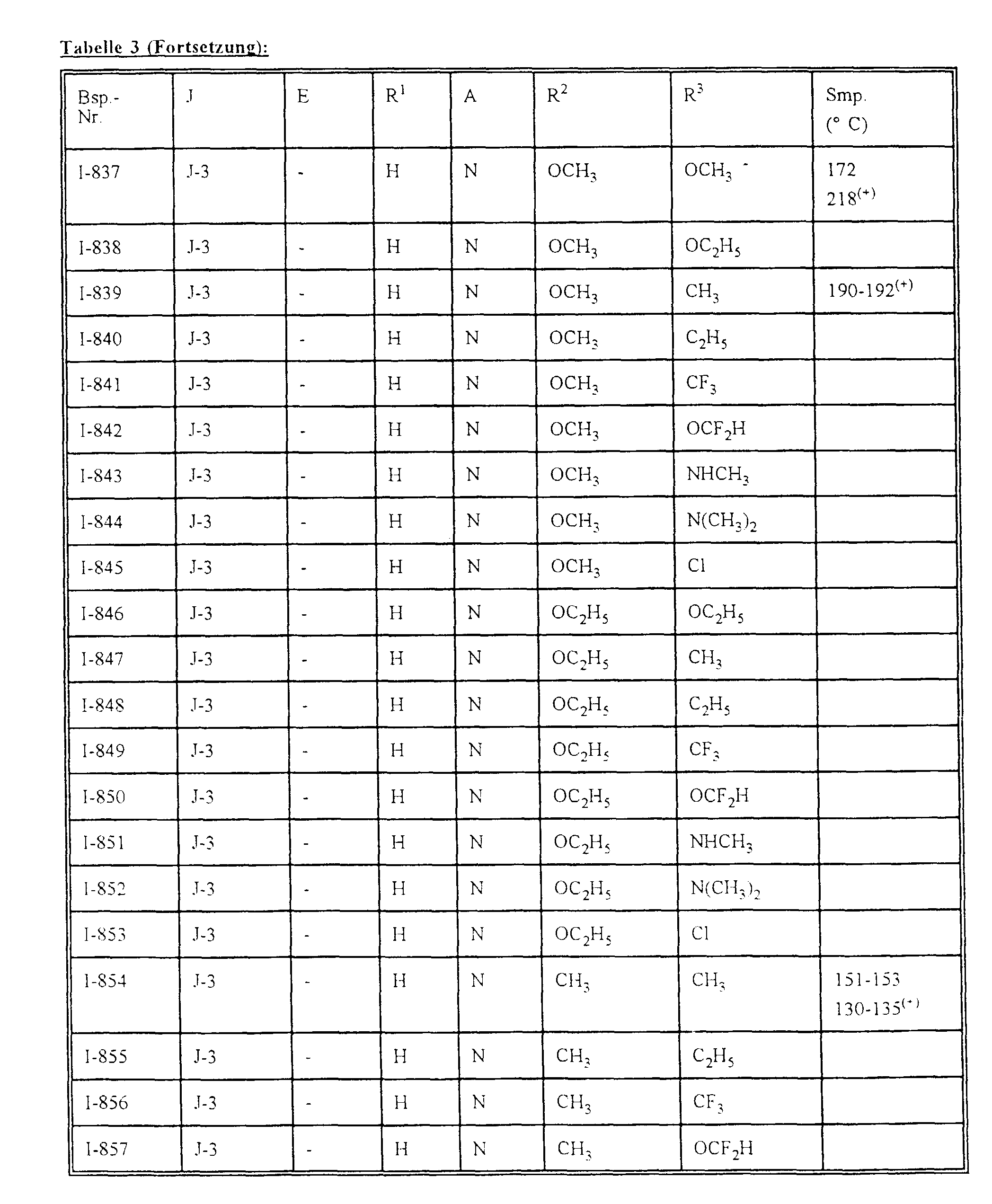

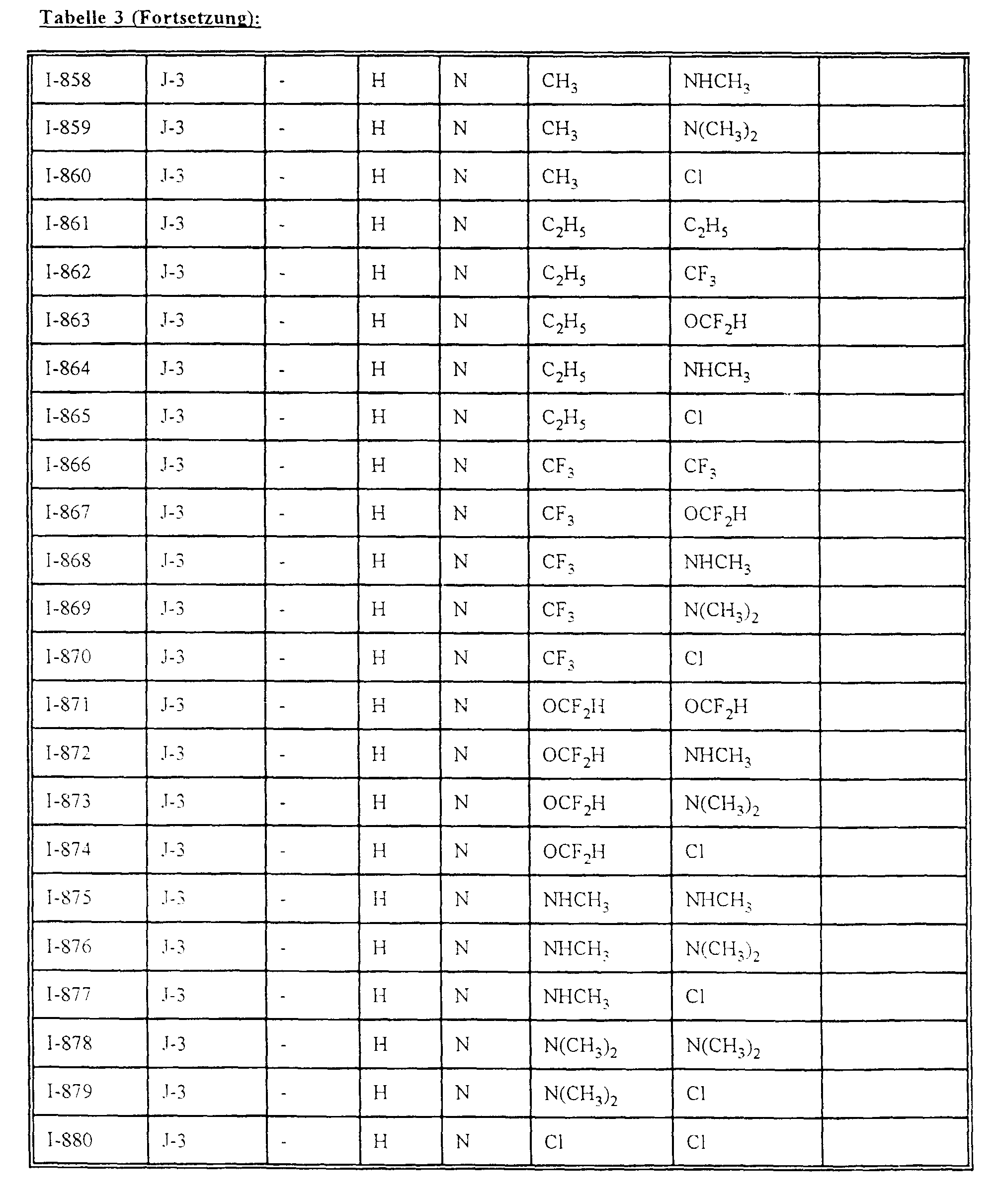

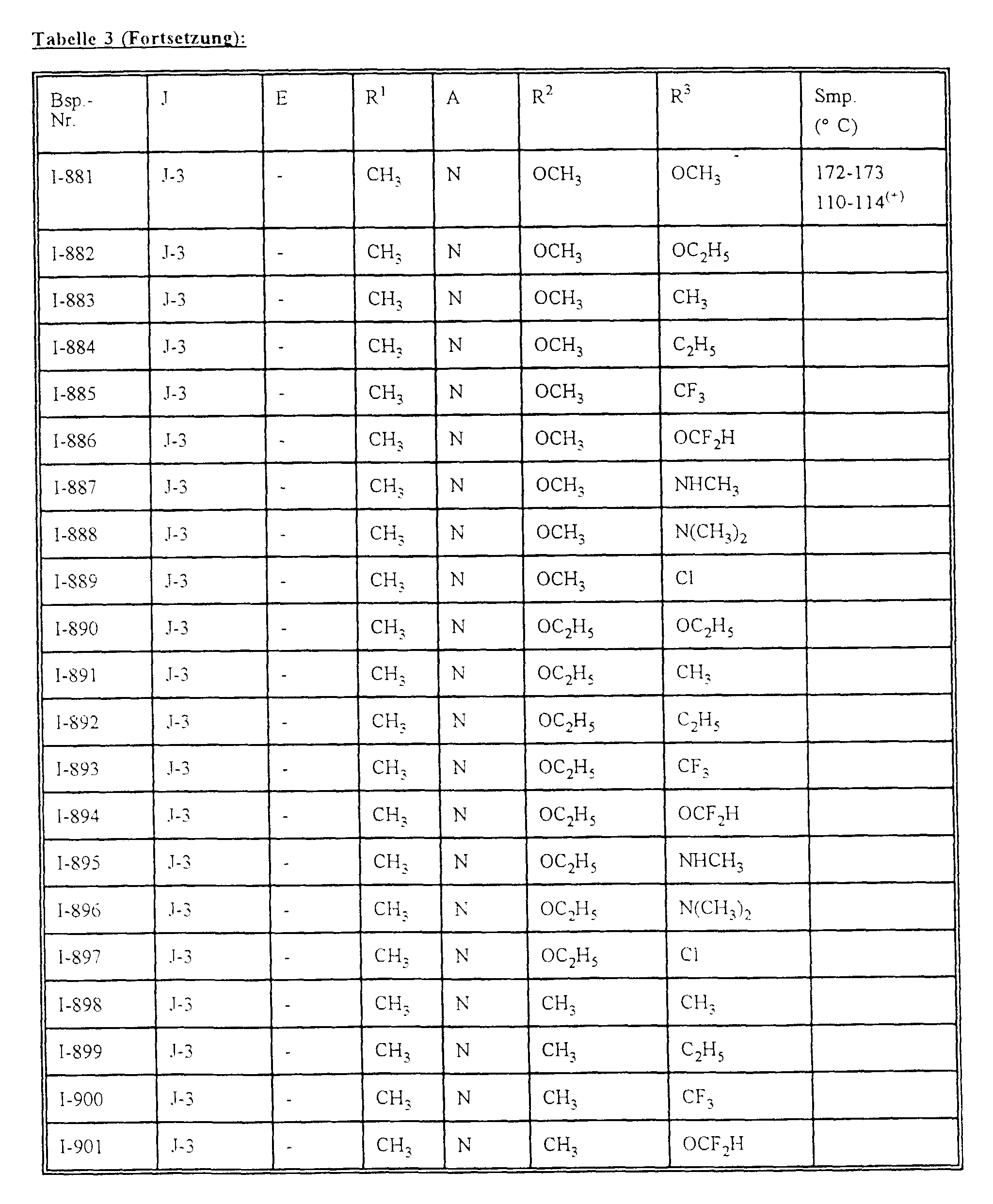

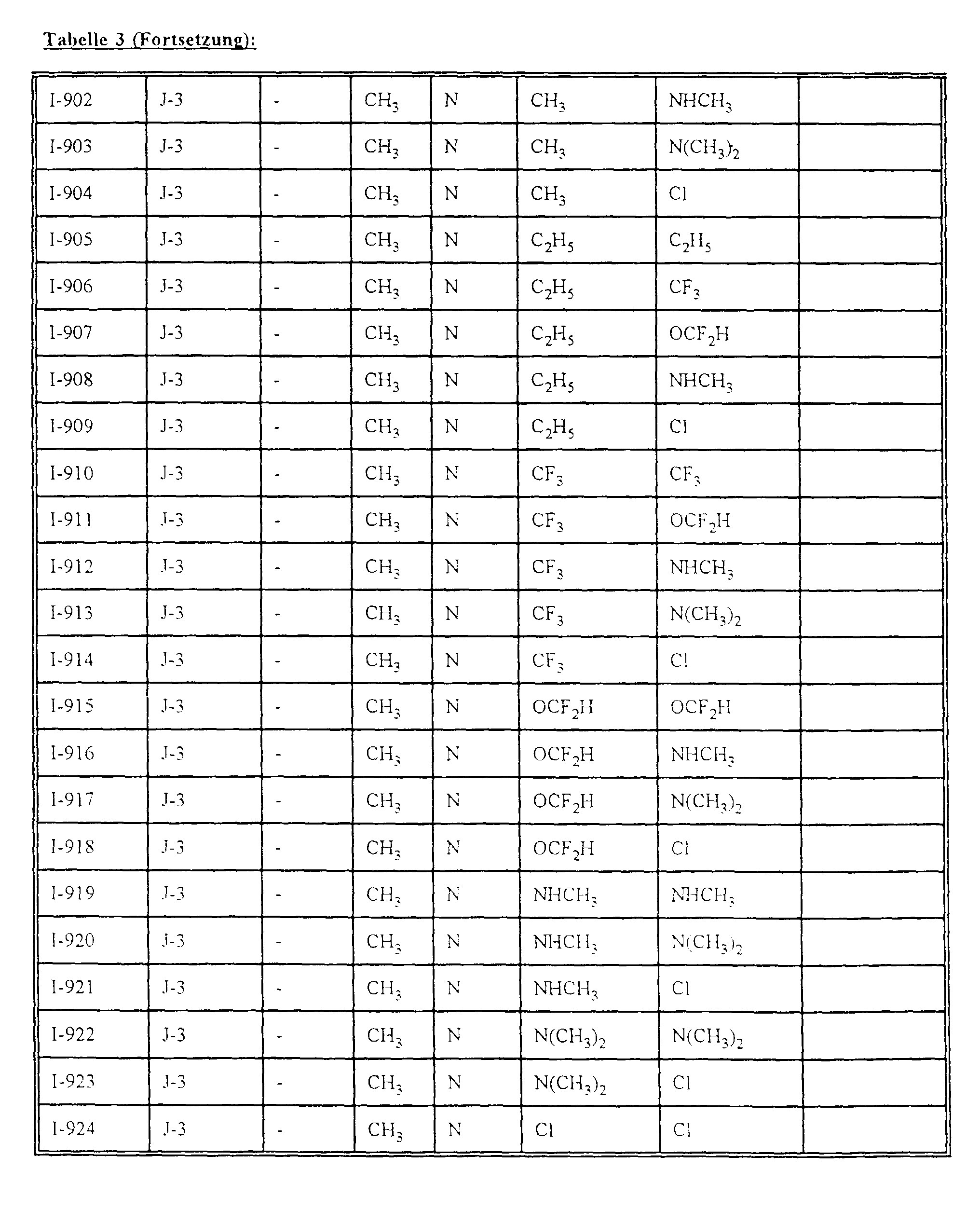

**Tabelle 4:**

| Beispiele für die Verbindungen der Formel (I) mit R¹=R⁴=R⁵=R⁶=R⁷=R¹⁰=H | | | | | | | |
|---|---|---|---|---|---|---|---|
| Bsp. Nr. | J | E | A | R² | R³ | R⁸ | Smp. (°C) |
| I-925 | J-1 | - | CH | OC₆H₅ | CH₃ | - | 105-109 |
| | | | | | | | 186-190⁽⁺⁾ |
| I-926 | J-1 | - | CH | Cl | OCH₂CF₃ | - | 111 |
| | | | | | | | 120-124⁽⁺⁾ |
| I-927 | J-2 | - | N | N(CH₃)₂ | OCH₂CF₃ | - | 158 |
| I-928 | J-2 | - | CH | Cl | OCH₂CF₃ | - | 204-205 |
| | | | | | | | 207⁽⁺⁾ |
| I-929 | J-3 | - | CH | Cl | OCH₂CF₃ | CH₃ | 110 |
| | | | | | | | 214-218⁽⁺⁾ |
| I-930 | J-3 | - | N | N(CH₃)₂ | OCH₂CF₃ | CH₃ | 183-184 |
| | | | | | | | 214-216⁽⁺⁾ |
| I-931 | J-3 | - | CH | CH₃ | OC₆H₅ | CH₃ | 195-198 |
| | | | | | | | 248-249⁽⁺⁾ |
| I-932 | J-4 | - | CH | Cl | OCH₂CF₃ | - | 115-118 |
| | | | | | | | 166⁽⁺⁾ |
| I-933 | J-4 | - | N | N(CH₃)₂ | OCH₂CF₃ | - | 173-174 |
| I-934 | J-4 | - | CH | CH₃ | OC₆H₅ | - | 104-108 |
| | | | | | | | 184⁽⁺⁾ |

### Salze von Verbindungen der Formel (I):

### Beispiel I-397-a:

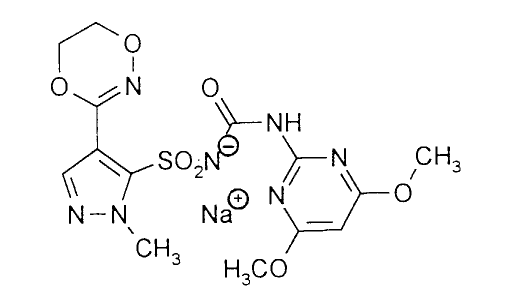

0.5 g (0.01 Mol) 80%iges Natriumhydroxid-Pulver werden unter Rühren zu einer Mischung aus 4.3 g (0.01 Mol) N-(4,6-Dimethoxypyrimidin-2-yl)-N'-(1-methyl-4-(5,6-dihydro-[1,4,2]-dioxazin-3-yl)pyrazol-5-sulfonyl)-harnstoff und 100 ml Toluol gegeben. Das Gemisch wird 15 Stunden bei 20°C gerührt; anschließend wird das kristalline Produkt durch Absaugen isoliert.

Man erhält 4.5 g (99% der Theorie) N-(4,6-Dimethoxypyrimidin-2-yl)-N'-(1-methyl-4-(5,6-dihydro-[1,4,2]-dioxazin-3-yl)-pyrazol-5-sulfonyl)-harnstoff-Na-Salz vom Schmelzpunkt 250°C (unter Zersetzung).

### Analog erhält man:

### Beispiel I-89-a

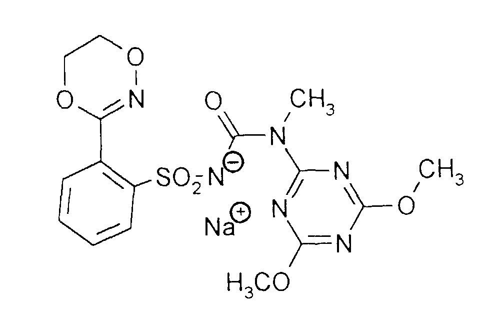

N-(4,6-Dimethoxy-1,3,5-triazin-2-yl)-N-methyl-N'-(2-(5,6-dihydro-[1,4,2]-dioxazin-3-yl)-benzolsulfonyl)-harnstoff-Na-Salz.
Schmelzpunkt 146 - 149°C.

### Beispiel I-617-a

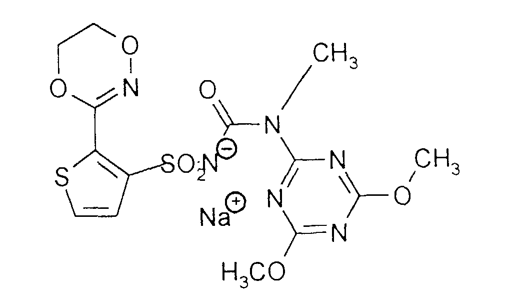

N-(4,6-Dimethoxy-1,3,5-triazin-2-yl)-N-methyl-N'-(2-(5,6-dihydro-[1,4,2]-dioxazin-3-yl)-thiophen-3-sulfonyl)-harnstoff-Na-Salz.
Schmelzpunkt 97 - 100°C.

### Ausgangsstoffe der Formel (II)

### Beispiel (II-1)

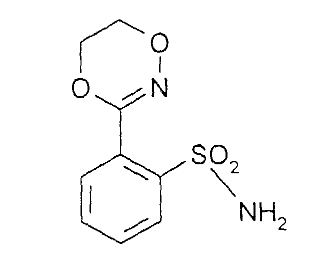

### (Verfahren (d))

In eine Mischung aus 22.7 g (0.0795 Mol) 1-Benzylthio-2-(5,6-dihydro-[1,4,2]-dioxazin-3-yl)-benzol in 150 ml Dichlormethan und 70 g Natriumdihydrogenphosphat-Hydrat in 150 ml Wasser wird bei 0°C bis zur Sättigung Chlor eingeleitet. Anschließend trennt man die Phasen, trocknet die organische Phase und zieht das Lösungsmittel bei vermindertem Druck ab. Das hierbei gebildete Sulfonylchlorid wird in 50 ml absolutem Tetrahydrofuran aufgenommen und bei -40°C zu einer Mischung aus 500 ml Tetrahydrofuran und 50 ml Ammoniak getropft. Man rührt bei Raumtemperatur 2 Stunden weiter und saugt dann die ausgefallenen Salze ab. Das Filtrat wird bei vermindertem Druck vom Lösungsmittel befreit und der Rückstand chromatographiert (Eluenten: 1. Dichlormethan, 2. Cyclohexan/Essigsäureethylester 1:1). Man erhält 11.5 g (60% der Theorie) 2-(5,6-Dihydro-[1,4,2]-dioxazin-3-yl)-benzolsulfonamid als blaßgelben Feststoff.
Schmelzpunkt: 131-133°C.

### Analog erhält man:

### Beispiel II-2

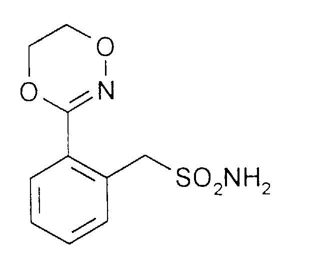

2-(5,6-Dihydro-[1,4,2]-dioxazin-3-yl)phenylmethansulfonamid. Schmelzpunkt:......° C

### Beispiel II-3

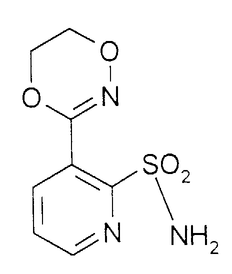

3-(5,6-Dihydro-[1,4,2]-dioxazin-3-yl)-pyridin-2-sulfonamid. Schmelzpunkt: 170°C.

### Beispiel II-4

### (Verfahren (e))

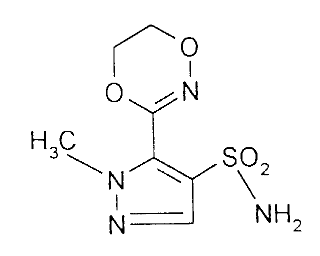

Zu einer Mischung aus 7.78 g (0.112 Mol) Hydroxylamin-Hydrochlorid und 150 ml Methanol tropft man bei Raumtemperatur 6.28 g (0.112 Mol) Kaliumhydroxid in 100 ml Methanol. Anschließend gibt man bei Raumtemperatur 13.1 g (0.056 Mol) 1-Methyl-5-ethoxycarbonylpyrazol-4-sulfonamid portionsweise zu Man rührt über Nacht bei Raumtemperatur und anschließend 2 Stunden bei 40°C und weitere 2 Stunden bei 60° C. Man läßt 3.14 g (0.056 Mol) Kaliumhydroxid in 50 ml Methanol zutropfen und rührt weitere 2 Stunden bei 60°C. Dann gibt man 7.74 g (0.056 Mol) Kaliumcarbonat zu, tropft 50.5 g (0.25 Mol) 1,2-Dibromethan (BrCH2CH2Br) zu und laßt über Nacht bei 60°C nachrühren. Anschließend wird das Lösungsmittel bei vermindertem Druck abdestilliert. Der Rückstand wird zwischen Methylenchlorid und Wasser verteilt. Die organische Phase wird getrocknet und das Lösungsmittel bei vermindertem Druck abdestilliert. Der Rückstand wird chromatographiert (Eluent: Cyclohexan/Essigsäureethylester 2:3).

Man erhält 5.91 g (43% der Theorie) 1-Methyl-5-(5,6-dihydro-[1,4,2]-dioxazin-3-yl)pyrazol-4-sulfonamid als blaßgelben Feststoff.
Schmelzpunkt: 126 - 130°C.

### Analog erhält man:

### Beispiel II-5

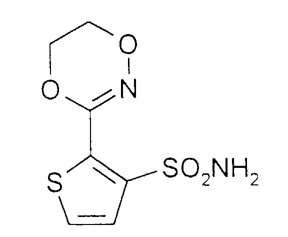

2-(5,6-Dihydro-[1,4,2]-dioxazin-3-yl)-thiophen-3-sulfonamid. Schmelzpunkt: 180-184 °C.

### Ausgangsstoffe der Formel (VII)

### Beispiel VII-1

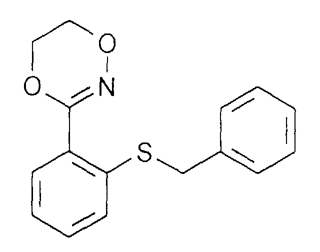

Zu einer Mischung aus 98.7 g (1.42 Mol) Hydroxylamin-Hydrochlorid und 700 ml Methanol tropft man bei Raumtemperatur 160 g (2.86 Mol) Kaliumhydroxid in 700 ml Methanol. Anschließend gibt man bei Raumtemperatur 184 g (0.712 Mol) 2-(Benzylthio)benzoesäuremethylester portionsweise zu. Man rührt über Nacht bei 40°C. Dann gibt man 98.1 g (0.712 Mol) Kaliumcarbonat zu, tropft 601 g (3.20 Mol) 1,2-Dibromethan zu und läßt über Nacht bei 60°C nachrühren. Anschließend wird das Lösungsmittel bei vermindertem Druck abdestilliert. Der Rückstand wird zwischen Methylenchlorid und Wasser verteilt. Die organische Phase wird getrocknet und das Lösungsmittel bei vermindertem Druck abdestilliert. Der Rückstand wird mit absolutem Ethylalkohol verrührt. Der Feststoff wird abgesaugt und im Hochvakuum getrocknet.

Man erhält 43.1 g (21% der Theorie) 1-Benzylthio-2-(5,6-dihydro-[1,4,2]-dioxazin-3-yl)-benzol als blaßgelben Feststoff.
Schmelzpunkt: 73 - 77°C.

### Analog erhält man:

### Beispiel VII-2

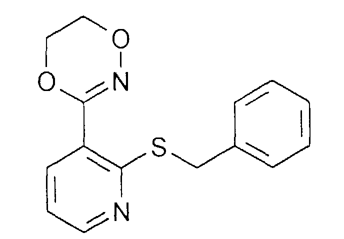

2-Benzylthio-3-(5,6-dihydro-[1,4,2]-dioxazin-3-yl)-pyridin.
Schmelzpunkt: 65.5-67.5°C.

### Anwendungsbeispiele:

In den folgenden Anwendungsbeispielen werden die nachstehend aufgeführten Verbindungen als Vergleichsverbindungen herangezogen: 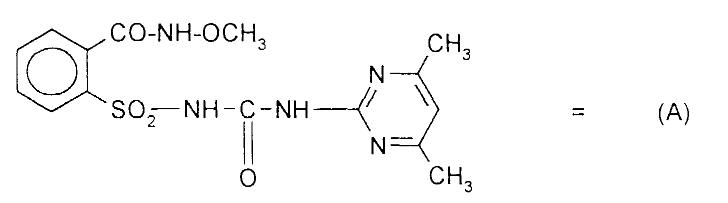 N-(4,6-Dimethylpyrimidin-2-yl)-N'-(2-methoxyaminocarbonyl-phenylsulfonyl)-harnstoff, und 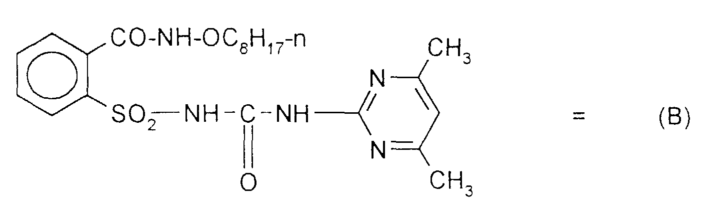 N-(4,6-Dimethylpyrimidin-2-yl)-N'-(2-n-octyloxyaminocarbonyl-phenylsulfonyl)-harnstoff,
(beide bekannt von DE-A-3 516 435, Beispiele 1 bzw. 2).

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
- 0 % =: keine Wirkung (wie unbehandelte Kontrolle)
- 100 % =: totale Vernichtung
Eine deutliche Überlegenheit in der Wirksamkeit gegenüber dem Stand der Technik zeigen in diesem Test z.B. die Verbindungen gemäß folgender Herstellungsbeispiele: (I-1), (I-9), (I-18), (I-47).

### Beispiel B

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

### Es bedeuten:

- 0 % =: keine Wirkung (wie unbehandelte Kontrolle)
- 100 % =: totale Vernichtung
Eine deutliche Überlegenheit in der Wirksamkeit gegenüber dem Stand der Technik zeigen in diesem Test z.B. die Verbindungen gemäß folgender Herstellungsbeispiele: (I-1), (I-18), (I-47), (I-89a), (I-397), (I-414), (I-485).

## Patentansprüche

1. N-Azinyl-N'-(het)arylsulfonyl-harnstoffe der Formel (I), 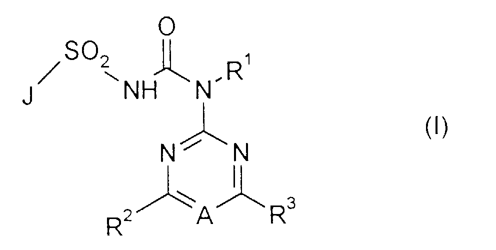 in welcher
A für Stickstoff oder eine CR¹¹-Gruppierung steht,
wobei
R¹¹ für Wasserstoff, Alkyl, Halogen und Haloalkyl steht,
R¹ für Wasserstoff oder einen gegebenenfalls substituierten Rest aus der Reihe Alkyl, Alkoxy, Alkoxyalkyl, Alkenyl, Alkinyl, Cycloalkyl, Cycloalkylalkyl, Aralkyl und Aryl steht,
R² für Wasserstoff, Halogen oder für jeweils gegebenenfalls durch Halogen substituiertes Alkyl, Alkoxy, Alkylthio, Alkylamino oder Dialkylamino mit jeweils 1 bis 6 Kohlenstoffatomen steht,
R³ für Wasserstoff, Halogen oder für jeweils gegebenenfalls durch Halogen substituiertes Alkyl, Alkoxy, Alkylthio, Alkylamino oder Dialkylamino mit jeweils 1 bis 6 Kohlenstoffatomen steht,
J für J-1 bis J-4 steht, wobei J-1 bis J-4 folgende Bedeutungen haben: 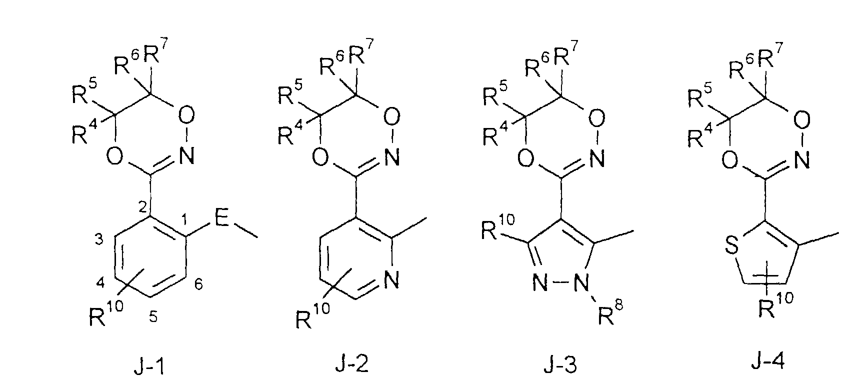 worin
E für eine direkte Bindung, Alkylen, Sauerstoff, Alkylamino oder Schwefel steht,
R⁴-R⁷ unabhängig voneinander für Wasserstoff, Halogen, Cyano, Thiocyanato oder für jeweils gegebenenfalls durch Halogen substituiertes Alkyl, Alkoxy, Alkylthio, Alkylsulfinyl, Alkylsulfonyl, Alkylamino, Alkylcarbonyl, Alkoxycarbonyl, Alkylaminocarbonyl mit jeweils 1 bis 3 Kohlenstoffatomen stehen,
R⁸ für Wasserstoff oder einen gegebenenfalls substituierten Rest aus der Reihe Alkyl, Alkenyl, Alkinyl, Cycloalkyl, Cycloalkylalkyl, Aralkyl und Aryl oder für eine Gruppe C(=O)R⁹ steht,
wobei
R⁹ für Wasserstoff, gegebenenfalls substituiertes Alkyl, gegebenenfalls substituiertes Aryl, Alkoxy, Alkylamino oder Dialkylamino steht,
R¹⁰ für Wasserstoff, Halogen, Cyano, Thiocyanato oder für jeweils gegebenenfalls durch Halogen substituiertes Alkyl, Alkoxy, Alkylthio, Alkylsulfinyl, Alkylsulfonyl, Alkylamino, Alkylcarbonyl, Alkoxycarbonyl, Alkylaminocarbonyl mit jeweils 1 bis 3 Kohlenstoffatomen steht, wobei in den vorgenannten Resten die Alkyl- und Alkylengruppen jeweils 1 bis 6 C-Atome, die Alkenyl- und Alkinylgruppen jeweils 2 bis 6 C-Atome, die Cycloalkylgruppen jeweils 3 bis 6 C-Atome und die Arylgruppen jeweils 6 bzw. 10 C-Atome enthalten können,
sowie Salze von Verbindungen der Formel (I).

2. Sulfonylharnstoffe der Formel (I) gemäß Anspruch 1, dadurch gekennzeichnet, daß darin
A für Stickstoff oder eine CH-Gruppierung steht,
R¹ für Wasserstoff oder einen gegebenenfalls durch Halogen substituierten Rest aus der Reihe Alkyl, Alkoxy, Alkoxyalkyl, Alkenyl und Alkinyl mit jeweils bis zu 3 Kohlenstoffatomen steht,
R² für Wasserstoff, Halogen oder für jeweils gegebenenfalls durch Halogen substituiertes Alkyl, Alkoxy, Alkylthio, Alkylamino oder Dialkylamino mit jeweils 1 bis 3 Kohlenstoffatomen in den Alkylresten steht,
R³ für Wasserstoff, Halogen oder für jeweils gegebenenfalls durch Halogen substituiertes Alkyl, Alkoxy, Alkylthio, Alkylamino oder Dialkylamino mit jeweils 1 bis 3 Kohlenstoffatomen in den Alkylresten steht,
J für J-1 bis J-4 steht,
E für eine direkte Bindung, Methylen, Sauerstoff, Alkylamino mit 1 bis 3 Kohlenstoffatomen oder Schwefel steht,
R⁴-R⁷ unabhängig voneinander für Wasserstoff, Halogen, Cyano, Thiocyanato oder für jeweils gegebenenfalls durch Halogen substituiertes Alkyl, Alkoxy, Alkylthio, Alkylsulfinyl, Alkylsulfonyl, Alkylamino, Alkylcarbonyl, Alkoxycarbonyl oder Alkylaminocarbonyl mit jeweils 1 bis 3 Kohlenstoffatomen in den Alkylresten steht,
R⁸ für Wasserstoff oder einen gegebenenfalls substituierten Rest aus der Reihe C₁-C₄-Alkyl, C₃-C₆-Cycloalkyl, C₇-C₁₁-Aralkyl und C₆-C₁₀-Aryl steht,
R¹⁰ für Wasserstoff, Halogen, Cyano, Thiocyanato oder für jeweils gegebenenfalls durch Halogen substituiertes Alkyl, Alkoxy, Alkylthio, Alkylsulfinyl, Alkylsulfonyl, Alkylamino, Alkylcarbonyl, Alkoxycarbonyl oder Alkylaminocarbonyl mit jeweils 1 bis 3 Kohlenstoffatomen in den Alkylresten steht.

3. Sulfonylharnstoffe der Formel (I) gemäß Anspruch 1, dadurch gekennzeichnet, daß darin
A für Stickstoff oder eine CH-Gruppierung steht,
R¹ für Wasserstoff, Methyl, Ethyl, Methoxy, Methoxymethyl oder Ethoxy steht,
R² für Wasserstoff, Chlor, Methyl, Ethyl, Trifluoromethyl, Methoxy, Ethoxy, Difluoromethoxy, Methylthio, Methylamino oder Dimethylamino steht,
R³ für Wasserstoff, Chlor, Methyl, Ethyl, Trifluoromethyl, Methoxy, Ethoxy, Difluoromethoxy, Methylthio, Methylamino oder Dimethylamino steht,
J für J-1 bis J-4 steht,
E für eine direkte Bindung, Methylen, Sauerstoff, C₁-C₂-Alkylamino oder Schwefel steht,
R⁴ - R⁷ unabhängig voneinander für Wasserstoff, Fluor, Chlor, Cyano, oder für jeweils gegebenenfalls durch Chlor oder Fluor substituiertes Methyl, Methylthio, Methylsulfinyl, Methylsulfonyl, Methoxycarbonyl und Ethoxycarbonyl steht,
R⁸ für Wasserstoff, Methyl, Ethyl, Phenyl oder Benzyl steht,
R¹⁰ für Wasserstoff, Fluor, Chlor, Brom, Cyano oder für jeweils gegebenenfalls durch Chlor oder Fluor substituiertes Methyl, Methoxy, Ethoxy, Methylthio, Ethylthio, Methylsulfinyl, Ethylsulfinyl, Methylsulfonyl, Ethylsulfonyl, Methyl- oder Dimethylamino steht.

4. Verfahren zur Herstellung von N-Azinyl-N'-(het)arylsulfonyl-harnstoffen der Formel (I) gemäß Anspruch 1, dadurch gekennzeichnet, daß man
(a) (Het)arylsulfonamide der allgemeinen Formel (II),
J-SO₂-NH₂ (II)
in welcher
J die in Anspruch 1 angegebenen Bedeutungen hat,
mit N-Azinylcarbamaten der Formel (In), 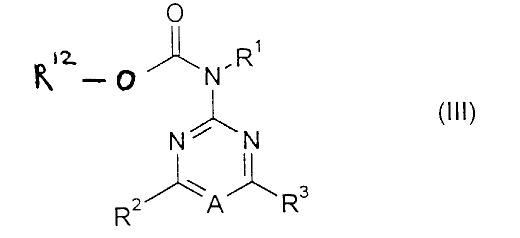 in welcher
A und R¹ - R³ die in Anspruch 1 angegebenen Bedeutungen haben, und
R¹² für Alkyl oder Aryl steht,
gegebenenfalls in Gegenwart eines Verdünnungsmittels und gegebenenfalls in Gegenwart eines Reaktionshilfsmittels umsetzt, oder daß man
(b) (Het)arylsulfonylisocyanate der allgemeinen Formel (IV),
J-SO₂-NCO (IV)
in welcher
J die oben angegebenen Bedeutungen hat,
mit Aminoazinen der Formel (V), 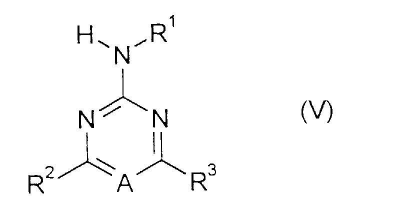 in welcher
A und R¹ - R³ die oben angegebenen Bedeutungen haben,
gegebenenfalls in Gegenwart eines Verdünnungsmittels und gegebenenfalls in Gegenwart eines Reaktionshilfsmittels umsetzt, oder daß man
(c) N-(Het)arylsulfonylcarbamate der allgemeinen Formel (VI) 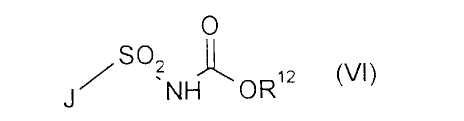 in welcher
J und R¹² die oben angegebenen Bedeutungen haben,
mit Aminoazinen der Formel (V), 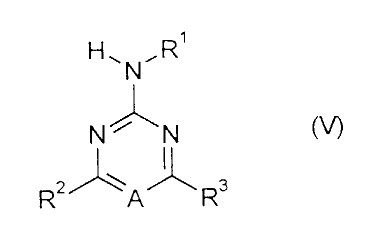 in welcher
A und R¹ - R³ die oben angegebenen Bedeutungen haben,
gegebenenfalls in Gegenwart eines Verdünnungsmittels und gegebenenfalls in Gegenwart eines Reaktionshilfsmittels umsetzt,
und gegebenenfalls die nach Verfahren (a), (b) oder (c) erhaltenen Produkte nach üblichen Methoden in Salze überführt.

5. Herbizide Mittel, gekennzeichnet durch einen Gehalt an mindestens einer Verbindung der Formel (I) gemäß Anspruch 1.

6. Verwendung von Verbindungen der allgemeinen Formel (I) gemäß Anspruch 1 zur Bekämpfung von unerwünschtem Pflanzenwachstum.

7. Verfahren zur Bekämpfung von Unkräutern, dadurch gekennzeichnet, daß man Verbindungen der allgemeinen Formel (I) gemäß Anspruch 1 auf die Unkräuter oder ihren Lebensraum einwirken läßt.

8. Verfahren zur Herstellung von herbiziden Mitteln, dadurch gekennzeichnet, daß man Verbindungen der allgemeinen Formel (I) gemäß Anspruch 1 mit Streckmitteln und/oder oberflächenaktiven Mitteln vermischt.

9. (Het)arylsulfonylverbindungen der Formel (XI),
J-SO₂-G (XI)
in welcher
J die in Anspruch 1 genannten Bedeutungen hat und
G für -NH₂, -N=C=O oder -NH-COOR¹² steht,
wobei
R¹² für Alkyl oder Aryl steht.

10. (Het)arylthiobenzylether der Formel (VII) 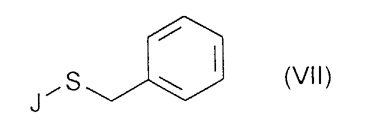 in welcher
J die in Anspruch 1 genannten Bedeutungen hat.

## Claims

1. N-Azinyl-N'-(het)arylsulphonyl-ureas of the formula (I), 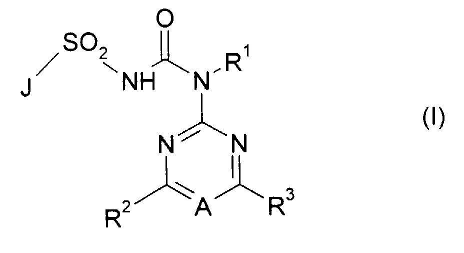 in which
A represents nitrogen or a CR¹¹ group,
where
R¹¹ represents hydrogen, alkyl, halogen and haloalkyl,
R¹ represents hydrogen or an optionally substituted radical from the series alkyl, alkoxy, alkoxyalkyl, alkenyl, alkinyl, cycloalkyl, cycloalkylalkyl, aralkyl and aryl,
R² represents hydrogen or halogen, or represents alkyl, alkoxy, alkylthio, alkylamino or dialkylamino having in each case 1 to 6 carbon atoms which are in each case optionally substituted by halogen,
R³ represents hydrogen or halogen, or represents alkyl, alkoxy, alkylthio, alkylamino or dialkylamino having in each case 1 to 6 carbon atoms which are in each case optionally substituted by halogen,
J represents J-1 to J-4, where J-1 to J-4 have the following meanings: 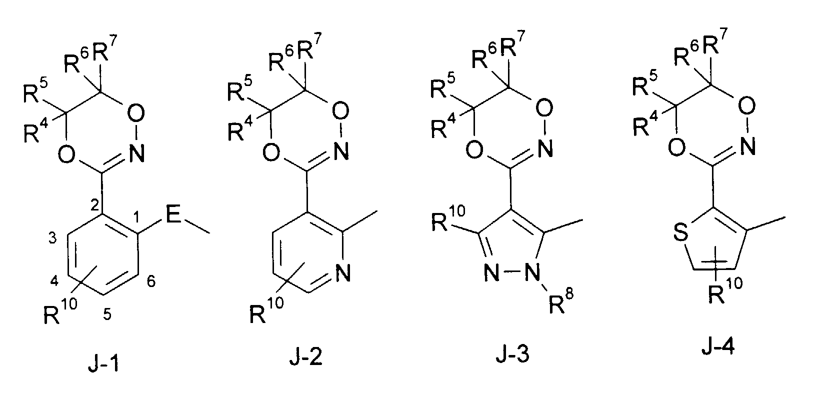 in which
E represents a direct linkage, alkylene, oxygen, alkylamino or sulphur,
R⁴-R⁷, independently of each other, represent hydrogen, halogen, cyano or thiocyanato, or represent alkyl, alkoxy, alkylthio, alkylsulphinyl, alkylsulphonyl, alkylamino, alkylcarbonyl, alkoxycarbonyl or alkylaminocarbonyl having in each case 1 to 3 carbon atoms which are in each case optionally substituted by halogen,
R⁸ represents hydrogen or an optionally substituted radical from the series alkyl, alkenyl, alkinyl, cycloalkyl, cycloalkylalkyl, aralkyl and aryl, or represents a group C(=O)R⁹,
where
R⁹ represents hydrogen, optionally substituted alkyl, optionally substituted aryl, alkoxy, alkylamino or dialkylamino,
R¹⁰ represents hydrogen, halogen, cyano or thio-cyanato, or represents alkyl, alkoxy, alkyl-thio, alkylsulphinyl, alkylsulphonyl, alkylamino, alkylcarbonyl, alkoxycarbonyl or alkylaminocarbonyl having in each case 1 to 3 carbon atoms which are in each case optionally substituted by halogen,
where, in the abovementioned radicals, the alkyl and alkylene groups can in each case contain 1 to 6 C atoms, the alkenyl and alkinyl groups in each case 2 to 6 C atoms, the cycloalkyl groups in each case 3 to 6 C atoms and the aryl groups in each case 6 or 10 C atoms,
as well as salts of compounds of the formula (I).

2. Sulphonylureas of the formula (I) according to Claim 1, characterized in that
A represents nitrogen or a CH group,
R¹ represents hydrogen or a radical from the series alkyl, alkoxy, alkoxyalkyl, alkenyl and alkinyl having in each case up to 3 carbon atoms which is optionally substituted by halogen,
R² represents hydrogen or halogen, or represents alkyl, alkoxy, alkylthio, alkylamino or dialkylamino having in each case 1 to 3 carbon atoms in the alkyl radicals and which are in each case optionally substituted by halogen,
R³ represents hydrogen or halogen, or represents alkyl, alkoxy, alkylthio, alkylamino or dialkylamino having in each case 1 to 3 carbon atoms in the alkyl radicals and which are in each case optionally substituted by halogen,
J represents J-1 to J-4,
E represents a direct linkage, methylene, oxygen, alkylamino having 1 to 3 carbon atoms, or sulphur,
R⁴-R⁷, independently of each other, represent hydrogen, halogen, cyano or thiocyanato, or represent alkyl, alkoxy, alkylthio, alkylsulphinyl, alkylsulphonyl, alkylamino, alkylcarbonyl, alkoxycarbonyl or alkylaminocarbonyl having in each case 1 to 3 carbon atoms in the alkyl radicals and which are in each case optionally substituted by halogen,
R⁸ represents hydrogen or an optionally substituted radical from the series C₁-C₄-alkyl, C₃-C₆-cycloalkyl, C₇-C₁₁-aralkyl and C₆-C₁₀-aryl,
R¹⁰ represents hydrogen, halogen, cyano or thio-cyanato, or represents alkyl, alkoxy, alkyl-thio, alkylsulphinyl, alkylsulphonyl, alkylamino, alkylcarbonyl, alkoxycarbonyl or alkylaminocarbonyl having in each case 1 to 3 carbon atoms in the alkyl radicals and which are in each case optionally substituted by halogen.

3. Sulphonylureas of the formula (I) according to Claim 1, characterized in that
A represents nitrogen or a CH group,
R¹ represents hydrogen, methyl, ethyl, methoxy, methoxymethyl or ethoxy,
R² represents hydrogen, chlorine, methyl, ethyl, trifluoromethyl, methoxy, ethoxy, difluoromethoxy, methylthio, methylamino or dimethylamino,
R³ represents hydrogen, chlorine, methyl, ethyl, trifluoromethyl, methoxy, ethoxy, difluoromethoxy, methylthio, methylamino or dimethylamino,
J represents J-1 to J-4,
E represents a direct linkage, methylene, oxygen, C₁-C₂-alkylamino or sulphur,
R⁴-R⁷, independently of each other, represent hydrogen, fluorine, chlorine or cyano, or represent methyl, methylthio, methylsulphinyl, methylsulphonyl, methoxycarbonyl and ethoxycarbonyl which are in each case optionally substituted by chlorine or fluorine,
R⁸ represents hydrogen, methyl, ethyl, phenyl or benzyl,
R¹⁰ represents hydrogen, fluorine, chlorine, bromine or cyano, or represents methyl, methoxy, ethoxy, methylthio, ethylthio, methylsulphinyl, ethylsulphinyl, methylsulphonyl, ethylsulphonyl, methylamino or dimethylamino which are in each case optionally substituted by chlorine or fluorine.

4. Process for preparing N-azinyl-N'-(het)arylsulphonyl-ureas of the formula (I) according to Claim 1, characterized in that
(a) (het)arylsulphonamides of the general formula (II),
J-SO₂-NH₂ (II),
in which
J has the meanings given in Claim 1,
are reacted with N-azinyl carbamates of the formula (III), 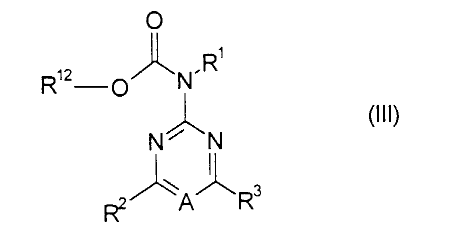 in which
A and R¹-R³ have the meanings given in Claim 1, and
R¹² represents alkyl or aryl,
optionally in the presence of a diluent and optionally in the presence of a reaction auxiliary, or in that
(b) (het)arylsulphonyl isocyanates of the general formula (IV),
J-SO₂-NCO (IV)
in which
J has the abovementioned meanings,
are reacted with aminoazines of the formula (V), 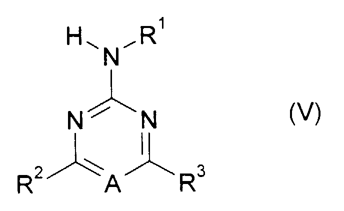 in which
A and R¹-R³ have the abovementioned meanings,
optionally in the presence of a diluent and optionally in the presence of a reaction auxiliary, or in that
(c) N-(het)arylsulphonyl carbamates of the general formula (VI), 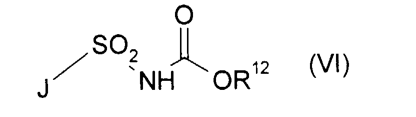 in which
J and R¹² have the abovementioned meanings,
are reacted with aminoazines of the formula (V), 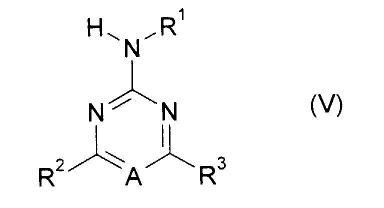 in which
A and R¹-R³ have the abovementioned meanings,
optionally in the presence of a diluent and optionally in the presence of a reaction auxiliary,
and the products obtained by processes (a), (b) or (c) are optionally converted into salts in accordance with customary methods.

5. Herbicidal agent, characterized by a content of at least one compound of the formula (I) according to Claim 1.

6. Use of compounds of the general formula (I) according to Claim 1 for controlling unwanted plant growth.

7. Process for controlling weeds, characterized in that compounds of the general formula (1) according to Claim 1 are permitted to act on the weeds or their habitat.

8. Process for preparing herbicidal agents, characterized in that compounds of the general formula (I) according to Claim 1 are mixed with extenders and/or surface-active agents.

9. (Het)arylsulphonyl compounds of the formula (XI),
J-SO₂-G (XI),
in which
J has the meanings given in Claim 1, and
G represents -NH₂, -N=C=O or -NH-COOR¹²,
where
R¹² represents alkyl or aryl.

10. (Het)arylthiobenzyl ethers of the formula (VII) 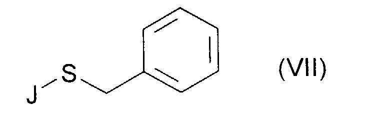 in which
J has the meanings given in Claim 1.

## Revendications

1. N-azinyl-N'-(hétéro)arylsulfonylurées de formule (I) 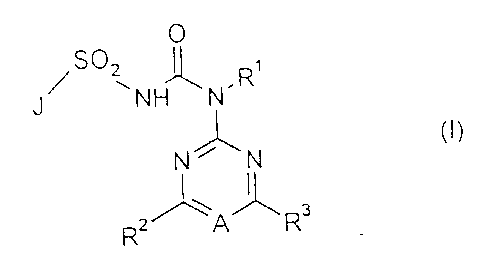 dans laquelle
A représente de l'azote ou un groupement CR¹¹, dans lequel
R¹¹ est de l'hydrogène, un groupe alkyle, un halogène ou un groupe halogénalkyle,
R¹ représente de l'hydrogène ou un reste éventuellement substitué de la série alkyle, alkoxy, alkoxyalkyle, alcényle, alcynyle, cycloalkyle, cycloalkylalkyle, aralkyle ou aryle,
R² représente de l'hydrogène, un halogène ou un groupe alkyle, alkoxy, alkylthio, alkylamino ou dialkylamino dont chacun est éventuellement substitué par un halogène et ayant chacun 1 à 6 atomes de carbone,
R³ représente de l'hydrogène, un halogène ou un groupe alkyle, alkoxy, alkylthio, alkylamino ou dialkylamino dont chacun est éventuellement substitué par un halogène et ayant chacun 1 à 6 atomes de carbone,
J représente un groupement J-1 à J-4, les groupements J-1 à J-4 ayant les définitions suivantes : 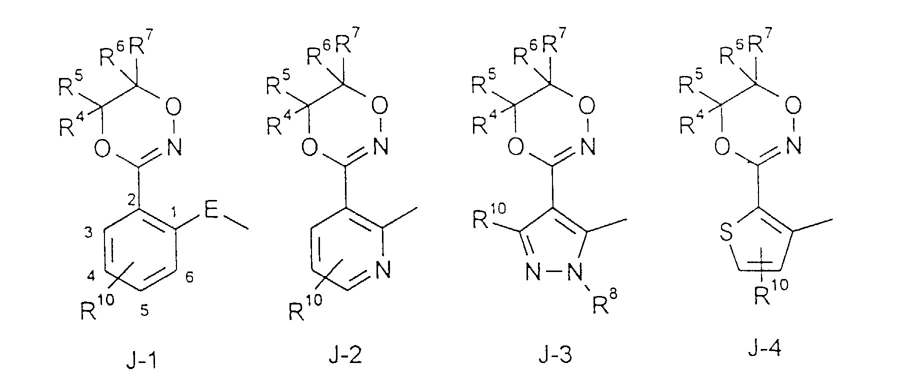 où
E représente une liaison directe, un groupe alkylène, de l'oxygène, un groupe alkylamino ou du soufre,
R⁴ à R⁷ représentent, indépendamment l'un de l'autre, de l'hydrogène, un halogène, un groupe cyano, thiocyanato, ou bien un groupe alkyle, alkoxy, alkylthio, alkylsulfinyle, alkylsulfonyle, alkylamino, alkylcarbonyle, alkoxycarbonyle, alkylaminocarbonyle dont chacun est éventuellement substitué par un halogène et ayant chacun 1 à 3 atomes de carbone,
R⁸ représente de l'hydrogène ou un reste éventuellement substitué de la série alkyle, alcényle, alcynyle, cycloalkyle, cycloalkylalkyle, aralkyle et aryle ou un groupe C(=O)R⁹,
où
R⁹ est de l'hydrogène, un groupe alkyle éventuellement substitué, un groupe aryle éventuellement substitué, alkoxy, alkylamino ou dialkylamino,
R¹⁰ représente de l'hydrogène, un halogène, un groupe cyano, thiocyanato, ou bien un groupe alkyle, alkoxy, alkylthio, alkylsulfinyle, alkylsulfonyle, alkylamino, alkylcarbonyle, alkoxycarbonyle, alkylaminocarbonyle dont chacun est éventuellement substitué par un halogène et ayant chacun 1 à 3 atomes de carbone,
les groupes alkyl et alkylène pouvant contenir chacun 1 à 6 atomes de carbone, les groupes alcényle et alcynyle chacun 2 à 6 atomes de carbone, les groupes cycloalkyle chacun 3 à 6 atomes de carbone et des groupes aryle chacun 6 ou 10 atomes de carbone dans les restes mentionnés ci-dessus,
ainsi que des sels de composés de formule (I).

2. Sulfonylurées de formule (I) suivant la revendication 1, caractérisées en ce que dans leur formule,
A représente de l'azote ou un groupement CH,
R¹ est de l'hydrogène ou un reste, éventuellement substitué par un halogène, de la série alkyle, alkoxy, alkoxyalkyle, alcényle et alcynyle avec dans chaque cas jusqu'à 3 atomes de carbone,
R² représente de l'hydrogène, un halogène ou bien un groupe alkyle, alkoxy, alkylthio, alkylamino ou dialkylamino dont chacun est éventuellement substitué par un halogène et ayant chacun 1 à 3 atomes de carbone dans les restes alkyle,
R³ représente de l'hydrogène, un halogène ou bien un groupe alkyle, alkoxy, alkylthio, alkylamino ou dialkylamino dont chacun est éventuellement substitué par un halogène et ayant chacun 1 à 3 atomes de carbone dans les restes alkyle,
J est un groupement J-1 à J-4,
E est une liaison directe, un groupe méthylène, de l'oxygène, un groupe alkylamino ayant 1 à 3 atomes de carbone, ou du soufre,
R⁴ à R⁷ représentent indépendamment l'un de l'autre de l'hydrogène, un halogène, un groupe cyano, thiocyanato, ou bien un groupe alkyle, alkoxy, alkylthio, alkylsulfinyle, alkylsulfonyle, alkylamino, alkylcarbonyle, alkoxycarbonyle ou alkylaminocarbonyle dont chacun est éventuellement substitué par un halogène et ayant chacun 1 à 3 atomes de carbone dans les restes alkyle,
R⁸ représente de l'hydrogène ou un reste, éventuellement substitué, de la série alkyle en C₁ à C₄, cycloalkyle en C₃ à C₆, aralkyle en C₇ à C₁₁ et aryle en ^{C}6 ^{à C}10,
R¹⁰ représente de l'hydrogène, un halogène, un groupe cyano, thiocyanato ou bien un groupe alkyle, alkoxy, alkylthio, alkylsulfinyle, alkylsulfonyle, alkylamino, alkylcarbonyle, alkoxycarbonyle ou alkylaminocarbonyle dont chacun est éventuellement substitué par un halogène et ayant chacun 1 à 3 atomes de carbone dans les restes alkyle.

3. Sulfonylurées de formule (I) suivant la revendication 1, caractérisées en ce que dans leur formule
A représente de l'azote ou un groupement CH,
R¹ est de l'hydrogène, un groupe méthyle, éthyle, méthoxy, méthoxyméthyle ou éthoxy,
R² est de l'hydrogène, du chlore, un groupe méthyle, éthyle, trifluorométhyle, méthoxy, éthoxy, difluorométhoxy, méthylthio, méthylamino ou diméthylamino,
R³ est de l'hydrogène, du chlore, un groupe méthyle, éthyle, trifluorométhyle, méthoxy, éthoxy, difluorométhoxy, méthylthio, méthylamino ou diméthylamino,
J est un groupement J-1 à J-4,
E est une liaison directe, un groupe méthylène, de l'oxygène, un groupe alkylamino en C₁ ou C₂ ou du soufre,
R⁴ à R⁷ représentent, indépendamment l'un de l'autre, de l'hydrogène, du fluor, du chlore, un groupe cyano, ou bien un groupe méthyle, méthylthio, méthylsulfinyle, méthylsulfonyle, méthoxycarbonyle et éthoxycarbonyle dont chacun est éventuellement substitué par du chlore ou du fluor,
R⁸ est de l'hydrogène, un groupe méthyle, éthyle, phényle ou benzyle,
R¹⁰ est de l'hydrogène, du fluor, du chlore, du brome, un groupe cyano ou bien un groupe méthyle, méthoxy, éthoxy, méthylthio, éthylthio, méthylsulfinyle, éthylsulfinyle, méthylsulfonyle, éthylsulfonyle, méthylamino ou diméthylamino dont chacun est éventuellement substitué par du chlore ou du fluor.

4. Procédé de production de N-azinyl-N'-(hétéro)arylsulfonylurées de formule (I) suivant la revendication 1, caractérisé en ce que :
(a) on fait réagir des (hétéro)arylsulfamides de formule générale (II)
J-SO₂-NH₂ (II)
dans laquelle
J a les définitions indiquées dans la revendication 1,
avec des N-azinylcarbamates de formule (III), 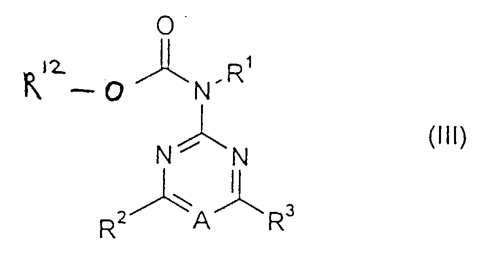 dans laquelle
A et R¹ à R³ ont les définitions indiquées dans la revendication 1,
et
R¹² est un groupe alkyle ou aryle,
le cas échéant en présence d'un diluant et en la présence éventuelle d'un auxiliaire de réaction, ou en ce que
(b) on fait réagir des (hétéro)arylsulfonylisocyanates de formule générale (IV)
J-SO₂-NCO (IV)
dans laquelle
J a les définitions indiquées ci-dessus, avec des amino-azines de formule (V) 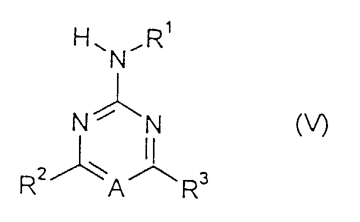 dans laquelle
A et R¹ à R³ ont les définitions indiquées ci-dessus, le cas échéant en présence d'un diluant et en la présence éventuelle d'un auxiliaire de réaction, ou en ce que
(c) on fait réagir des N-(hétéro)arylsulfonylcarbamates de formule générale (VI), 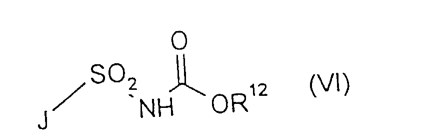 dans laquelle
J et R¹² ont les définitions indiquées ci-dessus, avec des aminoazines de formule (V) 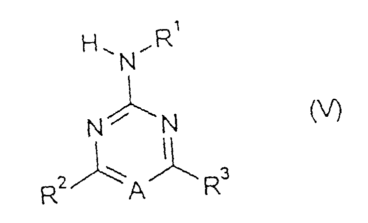 dans laquelle
A et R¹ à R³ ont les définitions indiquées ci-dessus, le cas échéant en présence d'un diluant et en la présence éventuelle d'un auxiliaire de réaction,
et on transforme éventuellement en sels par des opérations classiques les produits obtenus par le procédé (a), (b) ou (c).

5. Compositions herbicides, caractérisées par une teneur en au moins un composé de formule (I) suivant la revendication 1.

6. Utilisation de composés de formule générale (I) suivant la revendication 1 pour combattre la croissance de plantes indésirables.

7. Procédé pour combattre des mauvaises herbes, caractérisé en ce qu'on fait agir sur les mauvaises herbes ou sur leurs milieux des composés de formule générale (I) suivant la revendication 1.

8. Procédé de préparation de compositions herbicides, caractérisé en ce qu'on mélange des composés de formule générale (I) suivant la revendication 1 avec des diluants et/ou des agents tensio-actifs.

9. Composés (hétéro)arylsulfonyliques de formule (XI)
J-SO₂-G (XI)
dans laquelle
J a les définitions indiquées dans la revendication 1 et
G représente un groupe -NH₂, -N=C=O ou -NH-COOR¹², où
R¹² est un groupe alkyle ou aryle.

10. Ethers (hétéro) arylthiobenzyliques de formule (VII) 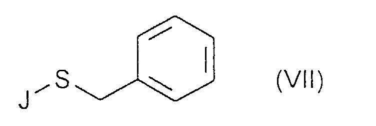 dans laquelle
J a les définitions indiquées dans la revendication 1.
